# EUROPEAN PATENT APPLICATION

(11) **EP 4 509 140 A1**
(43) Date of publication of application: **19.02.2025**
(21) Application number: 23787846.7
(22) Date of filing: 14.04.2023
(51) Int. Cl.: A61K 47/54, C12N 15/115, G01N 33/533

(54) **CONJUGATE, PHARMACEUTICAL COMPOSITION COMPRISING SAME, METHOD FOR PREPARING SAME, AND USE THEREOF**

(30) Priority: 14.04.2022 CN 202210386578; 14.04.2022 CN 202210377179; 14.04.2022 CN 202210401035
(71) Applicant: Suzhou Ribo Life Science Co., Ltd., Suzhou, Jiangsu 215300 (CN)
(72) Inventor: LIANG, Zicai, Suzhou, Jiangsu 215300 (CN); ZHANG, Hongyan, Suzhou, Jiangsu 215300 (CN); GAO, Shan, Suzhou, Jiangsu 215300 (CN)
(74) Representative: SSM Sandmair
(86) International application number: PCT/CN2023/088473
(87) International publication number: WO 2023/198204

(57) **Abstract**

The present disclosure provides a conjugate comprising one or more delivery groups and one or more diagnostic groups. Each of the delivery groups is independently linked to the diagnostic groups via a covalent bond or a linker group. The delivery group is formed by removing one or more hydrogen atoms or one or more functional groups from an aptamer comprising a continuous nucleotide sequence having a sequence set forth in formula (1). The conjugate provided by the present disclosure can efficiently target a tumor tissue and deliver the diagnostic groups to the tumor tissue, thereby helping effectively diagnose a tumor and a tumor-related disease.

## Description

### TECHNICAL FIELD

The present disclosure relates to a conjugate capable of specifically targeting tumor tissues and cells *in vivo,* and a pharmaceutical composition and a kit comprising the conjugate. The present disclosure also relates to the preparation methods and uses of these conjugates and pharmaceutical compositions.

### BACKGROUND ART

A tumor refers to a neoplasm formed by cell proliferation in local tissues under the action of various tumorigenic factors in the body. Among them, tumor cells that metastasize and invade surrounding tissues are called malignant tumors. According to the classification of the source tissue cells of tumors, the tumors could be generally classified into malignant tumors derived from epithelial cells (cancer), malignant tumors derived from mesenchymal tissue cells (sarcoma), malignant tumors derived from blood stem cells (leukemia, etc.), and malignant tumors derived from glial cells (gliomas). Among them, glioma is the most common primary intracranial malignant tumor, accounting for approximately 40% to 50% of brain tumors, with an annual incidence of 3 to 8 cases per 100,000 persons worldwide. According to the WHO pathological classification standards, gliomas belong to neuroepithelial tumors, which include many pathological types, including but not limited to, pilocytic astrocytoma, diffuse astrocytoma, anaplastic astrocytoma, glioblastoma, oligodendroglioma, anaplastic oligodendroglioma, etc.

Currently, one of the important issues in the art of tumor diagnosis and treatment, especially glioma, is how to specifically deliver diagnostic agents to tumor tissues and cells and enable these diagnostic agents to produce corresponding diagnostic effects at the appropriate time and in the appropriate manner.

Aptamers, also known as nucleic acid aptamers, are oligonucleotide molecules that can bind to various molecules of interest, such as small molecule compounds, proteins, nucleic acids, and even cells, tissues and organs. The aptamers can provide the important property of "recognizing specific molecules", and thus, similar to antibodies, they are commonly used in biotechnology and therapy. The aptamers can be designed in test tubes and quickly synthesized by chemical methods. They also have the excellent properties of being easy to store and having low or no immunogenicity. Therefore, they have gradually gained attention from researchers in the art in recent years. However, the aptamers suitable for tumor-targeted delivery still need to be further developed and applied in the art.

### SUMMARY OF THE INVENTION

The inventors of the present application have unexpectedly discovered a conjugate that could specifically target tumor cells, especially glioma cells, comprising an aptamer-based delivery group and a diagnostic agent group. The conjugates provided by the preset disclosure can effectively deliver diagnostic agents suitable for various types of tumors to tumor cells, especially glioma tissues and/or cells, showing excellent diagnostic effects. Thus, the inventors have made the following invention:
In one aspect, the present disclosure provides a conjugate comprising one or more delivery groups and one or more diagnostic agent groups, wherein each of the delivery groups is independently linked to the diagnostic agent group via a covalent bond or via a linking group;
wherein the delivery group is formed by removing one or more hydrogen atoms or one or more functional radicals from an aptamer, the aptamer comprises a segment of consecutive nucleotide sequence, wherein the group connecting two adjacent nucleotides is independently a phosphate ester group or a phosphate ester group with modification group(s), each nucleotide is selected from one of modified or unmodified A, U, C or G, and the consecutive nucleotide sequence has a sequence shown in Formula (1) as follows:

   5'- T₁-S₁-Nₐ-S₂-N_{b}-S₃-N_{c}-S₄-T₂ -3' Formula (1)
wherein, T₁ is a motif consisting of 1-3 nucleotides, T₂ is a motif consisting of 0-15 nucleotides, and T₂ does not comprise a motif that is completely reverse complementary to T₁;
S₁ and S₄ are each motifs consisting of 3-7 nucleotides, S₁ and S₄ are of the same length and are completely reverse complementary;
Nₐ and N_{c} are each motifs consisting of 1-4 nucleotides, each nucleotide in Nₐ is not complementary to each nucleotide in Nc, and the total number of U in Nₐ and N_{c} accounts for more than 50% of the total number of all nucleotides in Nₐ and N_{c};
S₂ and S₃ are each motifs consisting of 1-4 nucleotides, S₂ and S₃ are of the same length and are completely reverse complementary;
N_{b} is a motif consisting of 3-6 nucleotides, and the nucleotides at both ends of N_{b} do not form AU or GC complementarity.

In another aspect, the present disclosure also provides a pharmaceutical composition comprising the conjugate of the present disclosure and a pharmaceutically acceptable carri er.

In yet another aspect, the present disclosure also provides use of the conjugate and/or the pharmaceutical composition of the present disclosure in the manufacture of a medicament for diagnosing tumors and tumor-related diseases.

In yet another aspect, the present disclosure also provides a method for diagnosing tumors and tumor-related diseases, comprising administering an effective amount of the conjugate and/or the pharmaceutical composition of the present disclosure to a subject in need thereof.

In yet another aspect, the present disclosure further provides a kit comprising the conjugate and/or the pharmaceutical composition of the present disclosure.

### INCORPORATION BY REFERENCE

All publications, patents, and patent applications mentioned in this description are incorporated by reference into the present disclosure to the same extent as if each individual publication, patent, or patent application is specifically and individually incorporated by reference into the present disclosure.

### BENEFICIAL EFFECTS

The conjugates and pharmaceutical compositions provided by the present disclosure have excellent ability to target tumors, especially glioma tissues and cells, and the conjugates provide by the present disclosure can significantly improve diagnostic accuracy of tumors and tumor related diseases, thereby providing effective help for treatment of related diseases.

In one aspect, the conjugates provided by the present disclosure can efficiently and specifically enter into tumor cells *in vitro.* For example, compared with the comparative conjugates of random sequences, the conjugates provided by the present disclosure all show significantly higher mean fluorescence intensity in U118MG glioma cells, with a R value basically above 2, indicating that the conjugates provided by the present disclosure have excellent ability to enter into U118MG glioma cells. For another example, the conjugates provided by the present disclosure have very strong ability to enter into various cancer cells, such as, U118MG glioma cells, U251 human glioma cells, A549 human non-small cell lung cancer cells, and MCF-7 human breast cancer cells, and basically do not enter into normal cells, such as, SVGp12 normal astrocytes and 293T human renal epithelial cells. For another example, compared with comparative AP10, the conjugates provided by the present disclosure have very strong ability to enter into the interior of U118MG and A549 tumor spheres. For another example, the conjugates with different lengths and sequences all can effectively target U118MG glioma tissue.

In a further aspect, the conjugates provided by the present disclosure can deliver various diagnostic agent groups conjugated thereon, such as, different fluorescent groups, to tumor tissue. For another example, the different conjugates provided by the present disclosure all can exhibit strong fluorescence signals at the site of tumor inoculation in mice, indicating that the conjugates provided by the present disclosure can specifically target U118MG glioma. Furthermore, the conjugates provided by the present disclosure still exhibit strong fluorescence signals from 24h to 48h after administration, indicating that they can stably target glioma tissue for a long period of time. For another example, the conjugates provided by the present disclosure can still reach and target brain gliomas upon tail vein administration, implying that the conjugates of the present disclosure also have excellent ability to penetrate the Blood Brain Barrier (BBB) and deliver diagnostic agent groups across the blood brain barrier to brain glioma. For another example, the conjugates with various nucleotide sequence modifications all can stably target U118MG glioma, A549 human non-small cell lung cancer tumor and PAN02 pancreatic cancer tumor cells, demonstrating broad ability to specifically target tumors. For another example, the conjugates provided by the present disclosure can efficiently and specifically deliver different diagnostic agent groups (such as different fluorescent groups Cy3 or Cy5) to U118MG glioma, and can remain stable for a long time. For another example, by observing with a laser confocal microscope, the results show that the conjugates provided by the present disclosure can efficiently and specifically deliver diagnostic agent groups into the tumor cells of U118MG glioma, demonstrating excellent targeting effect and potential diagnostic ability.

Furthermore, the inventors of the present disclosure have unexpectedly discovered that the conjugates and/or pharmaceutical compositions provided by the present disclosure can efficiently pass through the blood brain barrier and target gliomas in the brain upon systemic administration, thereby further improving the delivery efficiency of the diagnostic agent groups, saving costs, and reducing undesired side effects.

This indicates that the conjugates provided by the present disclosure have excellent ability of targeting tumors, especially glioma tissues and cells, can significantly improve the success rate of tumor diagnosis, and have good application prospects.

### BRIEF DESCRIPTION OF THE DRAWINGS

Figures 1A and 1B are respectively the high content imaging images showing AP1 and comparative AP10 entering into U118MG glioma cells and SVGp12 normal astrocytes.
Figure 1C-1G are respectively the high content imaging images showing AP1 and comparative AP10 entering into U251 human glioma cells, A549 human non-small cell lung cancer cells, MCF-7 human breast cancer cells, and 293T human renal epithelial cells.
Figures 2A and 2B are respectively the high content imaging images showing AP1 and comparative AP10 entering into U118MG glioma tumor spheres and A549 human non-small cell lung cancer tumor spheres.
Figures 3A-3D are respectively images showing the *in vivo* imaging and tumor tissue imaging of U118MG subcutaneous tumor model mice treated with different conjugates at 1h, 4h, 24h, and 48h after administration.
Figures 4A and 4B are respectively images showing the fluorescence imaging of brain tissue in U118MG tumor in situ model mice established after administration of different conjugates at 24h and 48h after administration.
Figures 5A-5E are respectively images showing the *in vivo* fluorescence imaging of mice at different time points after administering different conjugates provided by the present disclosure and comparative conjugates. Figures 5F-5H are respectively images showing imaging of tumor tissues at different time points.
Figure 6 is an image showing the fluorescence imaging of the organs of one mouse in each group at 1h after administration of the conjugates.
Figure 7 is an image showing the fluorescence imaging of various organ tissues in PAN02 subcutaneous tumor model mice established after administration of the conjugate provided by the present disclosure.
Figures 8A-8E are respectively images showing the fluorescence imaging of the organs in mice administered with the conjugates provided by the present disclosure at different times after administration.
Figure 9 is an image showing the fluorescence imaging of tumors in each group of mice on D9 after administration of the conjugates.
Figures 10A-10C are respectively images showing the fluorescence imaging results in mice at 1h, 24h, and 48h after administration of different conjugates. Figure 10D is an image showing the fluorescence signal imaging of tumor tissues and kidneys in each group of mice after the mice were euthanized at D5.
Figures 11A-11C are respectively images showing the fluorescence imaging results in mice at 1h, 24h, and 48h after administration of different conjugates. Figure 11D is an image showing the fluorescence signal imaging of tumor tissues and kidneys in each group of mice after the mice were euthanized at D6.
Figure 12 is an image showing the laser confocal imaging results of U118MG glioma cells at 24h after administration of different conjugates to the mice subcutaneously inoculated with U118MG glioma.

### DETAILED DESCRIPTION OF THE INVENTION

The specific embodiments of the present disclosure are described in detail as below. It should be understood that the specific embodiments described herein are only for the purpose of illustration and explanation of the present disclosure and are not intended to limit the present disclosure.

### Definition

In the present disclosure, unless otherwise specified, A, U, C, G and T respectively refer to adenine nucleotide, uracil nucleotide, cytosine nucleotide, guanine nucleotide, and thymine nucleotide, and 2-methylcytosine nucleotide refers to a nucleotide obtained by substituting the 2' hydrogen on the cytosine base of the cytosine nucleotide with a methyl group. The structures of these nucleotides are well known to those skilled in the art. As used herein, a "nucleic acid motif" or a "motif" refers to a nucleic acid sequence fragment in an aptamer, consisting of one or more nucleotides. In some embodiments, a motif is a nucleic acid sequence fragment having a biological function.

As used herein, "alkyl" refers to straight-chain and branched chain saturated hydrocarbyl having the specified number of carbon atoms, typically from 1 to 20 carbon atoms, for example from 1 to 10 carbon atoms, such as from 1 to 8 or from 1 to 6 carbon atoms. For example, C₁-C₆ alkyl refers to both straight-chain and branched chain alkyl groups encompassing from 1 to 6 carbon atoms. When referring to an alkyl residue with a specific number of carbon atoms, it is intended to encompass all branched and straight-chain forms with that number of carbon atoms; thus, for example, "butyl" is meant to encompass n-butyl, sec-butyl, isobutyl and tert-butyl; "propyl" includes n-propyl and isopropyl. Alkylene is a subset of alkyl, referring to the same residues as alkyl, but having two attachment points.

As used herein, "alkenyl" refers to an unsaturated branched or straight-chain alkyl group having one or more carbon-carbon double bonds obtained by resp3+ectively removing one hydrogen molecule from two adjacent carbon atoms of the parent alkyl. The group can be in either cis or trans configuration of the double bond(s). Typical alkenyl groups include, but are not limited to: ethenyl; propenyl, such as prop-1-en-1-yl, prop-1-en-2-yl, prop-2-en-1-yl (allyl), prop-2-en-2-yl; butenyl, such as, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl; and the like. In certain embodiments, an alkenyl group has from 2 to 20 carbon atoms, while in other embodiments, from 2 to 10, from 2 to 8, or from 2 to 6 carbon atoms. Alkenylene is a subset of alkenyl, referring to the same residues as alkenyl, but having two attachment points.

As used herein, "alkynyl" refers to an unsaturated branched or straight-chain alkyl group having one or more carbon-carbon triple bonds obtained by respectively removing two molecules of hydrogen from adjacent carbon atoms of the parent alkyl. Typical alkynyl groups include, but are not limited to, ethynyl; propynyl, such as, prop-1-yn-1-yl, prop-2-yn-1-yl; butynyl, such as, but-1-yn-1-yl, but-1-yn-3-yl, but-3-yn-1-yl; and the like. In certain embodiments, an alkynyl group has from 2 to 20 carbon atoms, and in other embodiments, from 2 to 10, 2 to 8, or 2 to 6 carbon atoms. Alkynylene is a subset of alkynyl, referring to the same residues as alkynyl, but having two attachment points.

As used herein, "heterocyclyl" refers to a stable 3 to 18 membered non-aromatic ring radical that comprises 2 - 12 carbon atoms and 1 - 6 heteroatoms selected from nitrogen, oxygen or sulfur. Unless otherwise stated in the description, the heteroaryl is a monocyclic, bicyclic, tricyclic, or tetracyclic ring system, which may include fused or bridged ring system(s). The heteroatom(s) in the heteroaryl can be oxidized heteroatom(s). One or more nitrogen atoms, if present, can be quaternized nitrogen atoms. The heterocyclyl is partially or fully saturated. The heteroaryl can be linked to the rest of the molecule through any ring atom. Examples of such heterocyclic groups include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. heterocyclylene is a subset of heterocyclyl, referring to the same residues as heterocyclyl , but having two attachment points.

As used herein, "aryl" refers to a radical derived from an aromatic monocyclic or multicyclic hydrocarbyl ring system by removing hydrogen atom(s) from a ring carbon atom. The aromatic monocyclic or multicyclic hydrocarbyl ring system comprises only hydrogen and carbon, including from 6 to 18 carbon atoms, wherein one or more rings in the ring system is fully unsaturated, i.e., it comprises a cyclic, delocalized (4n+2)π-electron system in accordance with the Hückel theory. Aryl groups include, but are not limited to, groups such as phenyl, fluorenyl, and naphthyl. Arylene is a subset of aryl, referring to the same residues as aryl, but having two attachment points.

"Heteroaryl" refers to a radical derived from a 3 to 18 membered aromatic ring free radical that comprises from 2 to 17 carbon atoms and 1 to 6 heteroatoms selected from nitrogen, oxygen or sulfur. As used herein, the heteroaryl can be a monocyclic, bicyclic, tricyclic, or tetracyclic ring system, wherein one or more rings in the ring system is fully unsaturated, i.e., it comprises a cyclic, delocalized (4n+2)π-electron system in accordance with the Hückel theory. Heteroaryl includes fused or bridged ring system(s). The heteroatom(s) in the heteroaryl can be oxidized heteroatom(s). One or more nitrogen atoms, if present, can be quaternized nitrogen atoms. The heteroaryl is linked to the rest of the molecule through any ring atom. Examples of heteroaryl include, but are not limited to, azepinyl, acridinyl, benzimidazolyl, benzindolyl, 1,3-benzodioxazolyl, benzofuranyl, benzoxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzooxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10 hexahydrocycloocta[d]pyrimidinyl, 5,6,7,8,9,10-hexahydrocyclohepta[d]pyridazinyl, 5,6,7,8,9,10- hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinonyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1H-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta [4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl and thiophenyl/thienyl. Heteroarylene is a subset of heteroaryl, referring to the same residues as heteroaryl, but having two attachment points.

### Conjugates provided by the present disclosure

In one aspect, the present disclosure provides a conjugate comprising one or more delivery groups and one or more diagnostic agent groups, wherein each of the delivery groups is independently linked to the diagnostic agent group via a covalent bond or via a linking group. By comprising delivery groups and diagnostic agent groups, the conjugates provided by the present disclosure could efficiently and specifically target and deliver the diagnostic agent groups to tumors, thereby accurately and efficiently diagnose tumors or tumor-related diseases.

In the conjugate provided by the present disclosure, the delivery group is formed by removing one or more hydrogen atoms or one or more functional radicals from an aptamer, wherein the aptamer comprises a consecutive nucleotide sequence, wherein the group linking two adjacent nucleotides is independently a phosphate ester group or a phosphate ester group with modification group(s), and each nucleotide is selected from one of modified or unmodified A, U, C or G. The consecutive nucleotide sequence has the sequence shown by Formula (1):

5'-T₁-S₁-Nₐ-S₂-N_{b}-S₃-N_{c}-S₄-T₂-3' Formula (1)

wherein T₁ is a motif consisting of 1-3 nucleotides. The inventors have found that the presence of T₁ could enable the conjugate provided by the present disclosure to exhibit high tumor-targeting effect. In some embodiments, T₁ consists of 2 nucleotides. In this case, the conjugate provided by the present disclosure has more excellent tumor-targeting ability. In some embodiments, T₁ consists of 2 nucleotides and comprises at least one C. In some embodiments, T₁ is CU, UC or AC in the 5'-3' direction.

T₂ is a motif consisting of 0-15 nucleotides. The inventors have found that T₂ with these numbers of nucleotides and with various nucleotide sequences does not significantly affect the tumor-targeting ability of the conjugate provided by the present disclosure. In some embodiments, T₂ is a motif consisting of 0-10 nucleotides. In some embodiments, in the 5'-3' direction, T₂ consists of 1-9 nucleotides starting with U. In this case, the conjugate provided by the present disclosure may have more excellent stability.

T₂ does not comprise the motif that is complete reverse complementary to T₁. In the context of the present disclosure, "reverse complementary" means that hydrogen bond linkages are formed between two nucleotide sequences or motifs according to the rules of nucleic acid base pairing, and each nucleotide of one nucleotide sequence or motif in the 5'-3' direction can subsequentially form base pairs with each nucleotide of the other end nucleotide sequence or motif in the 3'-5' direction. In some embodiments, "reverse complementary" includes one or more of AU, GC, and UG complementarity.

S₁ and S₄ are each motifs consisting of 3-7 nucleotides, while S₁ and S₄ are of the same length and are completely reverse complementary. The conjugate provided by the present disclosure with the motifs S₁ and S₄ above has better stability and can target tumor tissues and cells over a long period of time. In some embodiments, S₁ and S₄ each consist of 3-5 nucleotides and are of the same length. In some embodiments, in the reverse complement formed by S₁ and S₄, GC complementarity accounts for more than 40% of the total number of complementarity. In this case, the conjugate provided by the present disclosure has further more excellent stability and tumor-targeting ability. In some embodiments, in the 5'-3' direction, S₁ is GCU and S₄ is AGC, or S₁ is GAGU and S₄ is GCUC, or S₁ is GGAGU and S₄ is GCUCU, or S₁ is UAUGG and S₄ is CCAUG.

Nₐ and N_{c} are each motifs consisting of 1-4 nucleotides. Each nucleotide in Nₐ is not complementary to each nucleotide in N_{c}, and the total number of U in Nₐ and N_{c} accounts for more than 50% of the total number of all nucleotides in Nₐ and N_{c}. The aptamer with the motifs Nₐ and N_{c} above shows excellent tumor tissue-targeting ability. In some embodiments, the sum of the number of nucleotides in Nₐ and N_{c} is an integer of 2-4. In some embodiments, the sum of the number of nucleotides in Nₐ and N_{c} is 3 or 4, and the sum of the number of U in Nₐ and N_{c} is 2 or 3. In some embodiments, in the 5'-3' direction, Nₐ and/or N_{c} are U, UU, UC or CU.

S₂ and S₃ are each motifs consisting of 1-4 nucleotides, while S₂ and S₃ are of the same length and are completely reverse complementary. By comprising the motifs S₂ and S₃, the conjugate provided by the present disclosure shows good stability and excellent tumor-targeting ability. In some embodiments, S₂ and S₃ each consist of 2-3 nucleotides and are of the same length. In some embodiments, the reverse complement formed by S₂ and S₃ comprises at least one GC complementarity, and in this case, the reverse complementarity has better stability. In some embodiments, in the 5'-3' direction, S₂ is CA and S₃ is UG, or S₂ is AC and S₃ is GU, or S₂ is GCC and S₃ is GGU.

N_{b} is a motif consisting of 3-6 nucleotides, and the nucleotides at both ends of N_{b} do not form AU or GC complementarity. Without being limited by theory, the aptamer with the motif N_{b} above can maintain a specific configuration in terms of space, thereby enabling the aptamer provided by the present disclosure to stably and efficiently target tumor tissues and cells. In some embodiments, N_{b} consists of 4-5 nucleotides. In some embodiments, in the 5'-3' direction, N_{b} is GACG, GACGU, GACCG, UACU, GUUG or GAUCU.

The inventors of the present disclosure have unexpectedly found that the aptamer comprising the sequence shown in Formula (1) above can effectively target tumors, especially glioma tissues, thereby enabling the delivery group in the conjugate provided by the present disclosure to deliver the diagnostic agent group with diagnostic effect on tumors to the tumors, thereby deliver diagnostic agent groups more effectively.

In some embodiments, in the conjugate provided by the present disclosure, the consecutive nucleotide sequence has a length of 18-50 nucleotides, or 20-40 nucleotides, or 21-36 nucleotides, or 24-32 nucleotides. The delivery groups formed by the aptamers having these lengths of the consecutive nucleotide sequences can more easily target tumors and achieve a good balance between synthesis cost and targeting effect.

In some embodiments, in the conjugate provided by the present disclosure, the consecutive nucleotide sequences have the following sequences as shown in SEQ ID NO: 1, SEQ ID NO: 2, or SEQ ID NO: 3:
5'-CUGCUUCAGACGUGUUAGCUU-3' (SEQ ID NO: 1)
   wherein, in the 5'-3' direction, T₁ is CU, S₁ is GCU, Nₐ is U, S₂ is CA, N_{b} is GACG, S₃ is UG, N_{c} is UU, S₄ is AGC, T₂ is UU;
5'-CUGAGUUCAGACGUGUUGCUCU-3' (SEQ ID NO: 2)
   wherein, in the 5'-3' direction, T₁ is CU, S₁ is GAGU, Nₐ is U, S₂ is CA, N_{b} is GACG, S₃ is UG, N_{c} is UU, S₄ is GCUC, T₂ is U;
5'-UCUAUGGCUGCCGAUCUGGUCUCCAUGUACGU-3' (SEQ ID NO: 3),
   wherein, in the 5'-3' direction, T₁ is CU, S₁ is GAGU, Nₐ is U, S₂ is CA, N_{b} is GACG, S₃ is UG, N_{c} is UU, S₄ is GCUC, T₂ is U.

In some embodiments, the consecutive nucleotide sequence has the nucleotide sequence as shown in SEQ ID NO: 4:
5'- N₆GGAGUUCAN₁N₂N₃N₄UGN₅GCUCN₇ -3' (SEQ ID NO: 4),
wherein, N₁, N₂ and N₃ independently of one another are one of A, U, C and G, N₄ is U, C or G or a motif consisting of two of U, C or G; N₅ is U, CU or UU; N₆ is CU, UC or AC; N₇ is U, UU or UUN₈, and N₈ is a motif consisting of 1-15 nucleotides.

In the nucleotide sequence above, in the 5'-3' direction, T₁ is the motif as shown by N₆, S₁ is the motif represented by GGAGU, Nₐ is U, S₂ is CA, N_{b} is the motif N₁N₂N₃N₄ consisting of N₁, N₂, N₃ and N₄, S₃ is UG, N_{c} is the motif represented by N₅, S₄ is the motif consisting of GCUC and the first nucleotide in N₇, and T₂ is the motif consisting of the remaining nucleotides in N₇.

The aptamer comprising the nucleotide sequences as shown in SEQ ID NO: 4 above can more effectively target tumors, especially gliomas, and can be enriched in tumor tissues.

Experimental verification shows that, in the nucleotide sequence as shown in SEQ ID NO: 4, the above selection of N₁, N₂, N₃ and N₄ does not significantly affect the tumor-targeting ability of the conjugate provided by the present disclosure. In some embodiments, the motif N₁N₂N₃N₄ consisting of N₁, N₂, N₃ and N₄ is one of GACG, GACGU, GACCG, UACU, GUUG, or GAUCU, and the conjuagets comprising these motifs provided by the present disclosure have higher tumor-specific targeting effects.

In some embodiments, in the nucleotide sequence as shown in SEQ ID NO: 4, N₅ is U or absent. In this case, the conjugates provided by the present disclosure all have excellent tumor-targeting effects.

In some embodiments, the consecutive nucleotide sequence has any one of the nucleotide sequences as shown in SEQ ID NOs: 5-11:
5'- CUGGAGUUCAGACGUGUUGCUCUU -3' (SEQ ID NO: 5)
5'- CUGGAGUUCAGACGUUGUGCUCUU -3' (SEQ ID NO: 6)
5'- CUGGAGUUCAGACCGUGUGCUCUU -3' (SEQ ID NO: 7)
5'- CUGGAGUUCAGACGUGUUGCUCU -3' (SEQ ID NO: 8)
5'- ACGGAGUUCAGACGUGUUGCUCUU -3' (SEQ ID NO: 9)
5'- CUGGAGUUCACUACUGUUGCUCUU -3' (SEQ ID NO: 10)
5'- CUGGAGUUCAGUUGUGUUGCUCUU -3' (SEQ ID NO: 11).

The conjugates having the nucleotide sequences above provided by the present disclosure show highly tumor-targeting effects.

In some embodiments, the motif N₈ consists of 1-15 nucleotides. In some embodiments, N₈ consists of 1-8 nucleotides.

In some embodiments, the presence of the motif N₈ makes the conjugate provided by the present disclosure more stable against an *in vivo* exonuclease, thereby enabling it to exert the tumor-targeting effect *in vivo* for a long period of time. In some embodiments, N₈ can increase or maintain the tumor-targeting effect of the conjugate provided by the present disclosure. In some embodiments, the motif N₈ consists of 8 nucleotides, considering the balance among stability, targeting, and synthesis efficiency. In some embodiments, in the 5'-3' direction, the motif N₈ is CCGAUCUC. In some embodiments, the consecutive nucleotide sequence has any one of the nucleotide sequences as shown in SEQ ID NOs: 12-14:
5'- CUGGAGUUCAGACGUGUUGCUCUUCCGAUCUC -3' (SEQ ID NO: 12)
5'- CUGGAGUUCAGACGUUGUGCUCUUCCGAUCUC -3' (SEQ ID NO: 13)
5'- CUGGAGUUCAGACCGUGUGCUCUUCCGAUCUC -3' (SEQ ID NO: 14)

In the consecutive nucleotide sequence, the terminal groups at the 5' end of the ribose of the 5' terminal nucleotide and at the 3' end of the ribose of the 3' terminal nucleotide are independently hydroxyl or phosphate groups, and the selection of these terminal groups does not change the targeting ability of the conjugate provided by the present disclosure. In some embodiments, the terminal groups at the 5' end of the ribose of the 5' terminal nucleotide and at the 3' end of the ribose of the 3' terminal nucleotide are hydroxyl groups in the consecutive nucleotide sequence.

In the conjugate provided by the present disclosure, each nucleotide may be a modified or unmodified nucleotide. In general, modifications of nucleotides may alter the stability and/or the tumor-targeting ability of the conjugate provided by the present disclosure. In some embodiments, at least one nucleotide in the conjugate provided by the present disclosure is a modified nucleotide. In some embodiments, at least one phosphate ester group in the conjugate provided by the present disclosure is a phosphate ester group with modification group(s).

The modification of nucleotide includes, but are not limited to, modification of sugar, modification of base, and/or substitution of a nucleotide with a nucleotide analog. In some embodiments, in the conjugate provided by the present disclosure, each of the modified nucleotides independently is one of 2'-halogen modified nucleotide, 2'-alkoxy modified nucleotide, 2'-alkyl modified nucleotide, 2'-substituted alkyl modified nucleotide, 2'-amino modified nucleotide, 2'-substituted amino modified nucleotide, 2'-deoxy nucleotide, a nucleotide with a modified base, and a nucleotide analog.

In the context of the present disclosure, a "fluoro modified nucleotide" refers to a nucleotide formed by substituting the 2'-hydroxy of the ribose group of the nucleotide with a fluoro group, which has a structure as shown by the following Formula (7). A "non-fluoro modified nucleotide" refers to a nucleotide formed by substituting the 2'-hydroxy of the ribose group of the nucleotide with a non-fluoro group, or a nucleotide analogue. In some embodiments, each non-fluoro modified nucleotide is independently selected from the group consisting of a nucleotide formed by substituting the 2'-hydroxy of the ribose group of the nucleotide with a non-fluoro group, or a nucleotide analogue.

These nucleotides formed by substituting the 2'-hydroxy of the ribose group with a non-fluoro group are well-known to those skilled in the art, and can be selected from one of 2'-alkoxy modified nucleotide, 2'-alkyl modified nucleotide, 2'-substituted alkyl modified nucleotide, 2'-amino modified nucleotide, 2'-substituted amino modified nucleotide, and 2'-deoxy nucleotide.

In some embodiments, the 2'-alkoxy modified nucleotide is a methoxy modified nucleotide (2'-OMe), as shown by Formula (8). In some embodiments, the 2'-amino modified nucleotide (2'-NH₂) is as shown by Formula (9). In some embodiments, the 2'-deoxy nucleotide (DNA) is as shown by Formula (10):

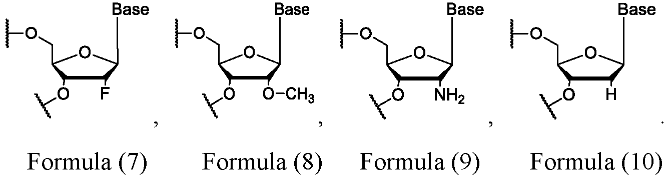

Those skilled in the art know various ways of modifying the bases of nucleotides. In some embodiments, base modification includes, but is not limited to, adding one or more methyl groups to a base. In some embodiments, thymine (T) is considered as one uracil (U) with a modified base. In some embodiments, 2-methylcytosine is considered as one cytosine (C) with a modified base.

A "nucleotide analogue" refers to a group that can replace a nucleotide in the nucleic acid, while structurally differs from an adenine ribonucleotide, a guanine ribonucleotide, a cytosine ribonucleotide, a uracil ribonucleotide or thymine deoxyribonucleotide. In some embodiments, a nucleotide analogue may be an isonucleotide, a bridged nucleic acid (bridged nucleic acid, abbreviated as BNA) nucleotide or an acyclic nucleotide.

A BNA refers to a nucleotide that is constrained or is not accessible. BNA can comprise a 5-, 6- membered or even a 7-membered ring bridged structure with a "fixed" C3'-endo sugar puckering. The bridge is typically incorporated at the 2'- and 4'-position of the ribose to afford a 2', 4'-BNA nucleotide. In some embodiments, BNA may be LNA, ENA and cET BNA, wherein LNA is as shown by Formula (12), ENA is as shown by Formula (13) and cET BNA is as shown by Formula (14).

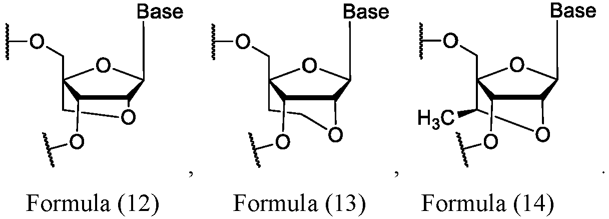

An acyclic nucleotide is a nucleotide in which the ribose ring is opened. In some embodiments, an acyclic nucleotide may be an unlocking nucleic acid (UNA), a glycerol nucleic acid (GNA) or a peptide nucleic acids (PNA), wherein UNA is as shown by Formula (15) and GNA is as shown by Formula (16):

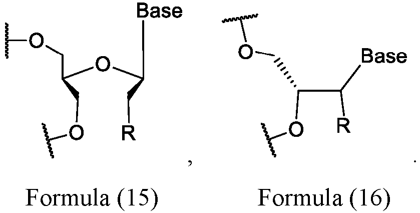

In the above Formula (15) and Formula (16), R is selected from H, OH, or alkoxy (O-alkyl).

A peptide nucleic acid is a nucleotide analog formed by replacing the glycoside-phosphate backbone with a polypeptide backbone. In some embodiments, the peptide nucleic acid may be, for example, a nucleotide analog formed by replacing the glycoside-phosphate unit with a 2-aminoethyl glycine bond.

An isonucleotide is a compound in which the position of the base on the ribose ring in the nucleotide is changed. In some embodiments, an isonucleotide may be a compound in which the base is transposed from position-1' to position-2' or -3' on the ribose ring, as shown by Formula (17) or (18), respectively.

In the above compounds of Formulae (17)-(18), "Base" represents a nucleic acid base, such as, A, U, G, C, or T; R is selected from H, OH, F, or a non-fluoro group above.

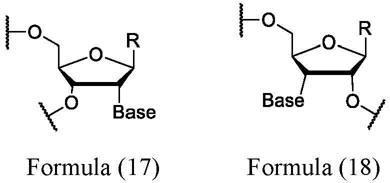

In some embodiments, a nucleotide analogue is selected from the group consisting of an isonucleotide, LNA, ENA, cET, UNA, and GNA.

In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group.

In the context of the present disclosure, a "fluoro modified nucleotide", a "2'-fluoro modified nucleotide", a "nucleotide in which 2'-hydroxy of the ribose group is substituted with a fluoro group" and a "nucleotide comprising 2'-fluororibosyl" have the same meaning, referring to the nucleotide formed by substituting the 2'-hydroxy of the ribose group with a fluoro group, having the structure as shown by Formula (7). A "methoxy modified nucleotide", a "2'-methoxy modified nucleotide", a "nucleotide in which 2'-hydroxy of a ribose group is substituted with a methoxy" and a "nucleotide comprising 2'-methoxyribosyl" have the same meaning, referring to a compound in which 2'-hydroxy of the ribose group in the nucleotide is substituted with a methoxy, and has a structure as shown by Formula (8).

In some embodiments, each cytosine nucleotide of the consecutive nucleotide sequence in the aptamer provided by the present disclosure is a fluoro modified cytosine nucleotide, and/or each uracil nucleotide of the consecutive nucleotide sequence in the aptamer provided by the present disclosure is a fluoro modified uracil nucleotide. In some embodiments, each nucleotide of the consecutive nucleotide sequence in the aptamer provided by the present disclosure is a 2'-methoxy modified nucleotide. In some embodiments, one or more uracil nucleotides in the aptamer provided by the present disclosure have modified bases.

The group connecting two adjacent nucleotides may be a phosphate ester group or a modified phosphate ester group. The modification of a phosphate ester group may be, for example, substituting at least one non-bridging oxygen atom in a phosphate ester group with a sulfur atom to form a phosphorothioate group or a phosphorodithioate group. In some embodiments, at least one group connecting two adjacent nucleotides in the aptamer provided by the present disclosure is a phosphorothioate group. In some embodiments, at least one of the three groups connecting two adjacent nucleotides in the first four nucleotides at the 5' terminal of the aptamer provided by the present disclosure is a phosphorothioate group. In some embodiments, at least two of the three groups connecting two adjacent nucleotides in the first four nucleotides at the 5' terminal of the aptamer provided by the present disclosure are phosphorothioate groups. In some embodiments, at least one group connecting two adjacent nucleotides in the first four nucleotides at the 3' terminal of the aptamer provided by the present disclosure is a phosphorothioate groups. In some embodiments, at least two of the three groups connecting two adjacent nucleotides in the first four nucleotides at the 5' terminal of the aptamer provided by the present disclosure are phosphorothioate groups. In some embodiments, each group connecting two adjacent nucleotides in the aptamer provided by the present disclosure is a phosphorothioate group.

The conjugate with the modification above provided by the present disclosure not only has low cost, but also can make the delivery group less susceptible to cleavage by *in vivo* ribonuclease, thereby increasing the stability of the conjugate provided by the present disclosure and make it more resistant to nuclease hydrolysis. Meanwhile, the conjugate with the modification above provided by the present disclosure has higher activity of targeting tumor tissues and/or cells.

In some embodiments, the conjugate provided by the present disclosure has one of the nucleotide sequences as shown in SEQ ID NOs: 15-39:
5'- CfUfGGAGUfUfCfAGACfGUfGUfUfGCfUfCfUfUfCfCfGAUfCfUfCf -3' (SEQ ID NO: 15)
5'- CfUfGGAGUfUfCfAGACfGUfGUfUfGCfUfCfUfUf -3' (SEQ ID NO: 16)
5'- CfUfGGAGUfUfCfAGACfGUfUfGUfGCfUfCfUfUfCfCfGAUfCfUfCf -3' (SEQ ID NO: 17)
5'- CfUfGGAGUfUfCfAGACfGUfUfGUfGCfUfCfUfUf -3' (SEQ ID NO: 18)
5'- CfUfGGAGUfUfCfAGACfCfGUfGUfGCfUfCfUfUfCfCfGAUfCfUfCf -3' (SEQ ID NO: 19)
5'- CfUfGGAGUfUfCfAGACfCfGUfGUfGCfUfCfUfUf -3' (SEQ ID NO: 20) 
5'- CmUmGmGmAmGmUmUmCmAmGmAmCmGmUmGmUmUmGmCmUmCm
UmUmCmCmGmAmUmCmUmCm -3' (SEQ ID NO: 21)
 
5'- CmUmGmGmAmGmUmUmCmAmGmAmCmGmUmGmUmUmGmCmUmCm
UmUm -3' (SEQ ID NO: 22)
 
5'- CmUmGmGmAmGmUmUmCmAmGmAmCmGmUmUmGmUmGmCmUmCm
UmUmCmCmGmAmUmCmUmCm -3' (SEQ ID NO: 23)
 
5'- CmUmGmGmAmGmUmUmCmAmGmAmCmGmUmUmGmUmGmCmUmCm
UmUm -3' (SEQ ID NO: 24)
 
5'- CmUmGmGmAmGmUmUmCmAmGmAmCmCmGmUmGmUmGmCmUmCm
UmUmCmCmGmAmUmCmUmCm -3' (SEQ ID NO: 25)
 
5'- CmUmGmGmAmGmUmUmCmAmGmAmCmCmGmUmGmUmGmCmUmCm
UmUm -3' (SEQ ID NO: 26)
 
5'- CmUmGmGmAmGmUmUmCmAmGmAmCmGmUmGmUmUmGmCmUmCm
Um -3' (SEQ ID NO: 27)
 
5'- CmUmGmAmGmUmUmCmAmGmAmCmGmUmGmUmUmGmCmUmCmUm
-3' (SEQ ID NO: 28)
5'- CmUmGmCmUmUmCmAmGmAmCmGmUmGmUmUmAmGmCmUmUm -3' (SEQ ID NO: 29) 
5'- AmCmGmGmAmGmUmUmCmAmGmAmCmGmUmGmUmUmGmCmUmCm
UmUm -3' (SEQ ID NO: 30)
 
5'- CmUmGmGmAmGmUmUmCmAmCmUmAmCmUmGmUmUmGmCmUmCm
UmUm -3' (SEQ ID NO: 31)
 
5'- CmUmGmGmAmGmUmUmCmAmGmUmUmGmUmGmUmUmGmCmUmCm
UmUm -3' (SEQ ID NO: 32)
 
5'- UmCmUmAmUmGmGmCmUmGmCmCmGmAmUmCmUmGmGmUmCmUm
CmCmAmUmGmUmAmCmGmUm -3' (SEQ ID NO: 33)
 
5'- CmsUmsGmGmAmGmUmUmCmAmGmAmCmGmUmGmUmUmGmCmUmC
mUmUmCmCmGmAmUmCmsUmsCm -3' (SEQ ID NO: 34)
 
5'- CmsUmsGmGmAmGmUmUmCmAmGmAmCmGmUmGmUmUmGmCmUmC
msUmsUm -3' (SEQ ID NO: 35)
 
5'- CmsUmsGmGmAmGmUmUmCmAmGmAmCmGmUmUmGmUmGmCmUmC
mUmUmCmCmGmAmUmCmsUmsCm -3' (SEQ ID NO: 36)
 
5'- CmsUmsGmGmAmGmUmUmCmAmGmAmCmGmUmUmGmUmGmCmUmC
msUmsUm -3' (SEQ ID NO: 37)
 
5'- CmsUmsGmGmAmGmUmUmCmAmGmAmCmCmGmUmGmUmGmCmUmC
mUmUmCmCmGmAmUmCmsUmsCm -3' (SEQ ID NO: 38)
 
5'- CmsUmsGmGmAmGmUmUmCmAmGmAmCmCmGmUmGmUmGmCmUmC
msUmsUm -3' (SEQ ID NO: 39)
wherein C, G, U, and A represent the base composition of the nucleotide; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by a phosphorothioate linkage.

In some embodiments, the conjugate provided by the present disclosure has a structure as shown by Formula (2): 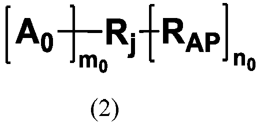 wherein each R_{AP} group is independently a group having a structure as shown by Formula (3): 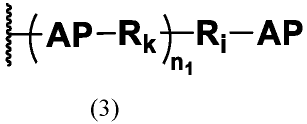 wherein each AP group is identical or different, and independently represents one of the delivery groups; each A₀ group is identical or different, and independently represents one of the diagnostic agent functional groups; Rⱼ, each Rₖ or each Rᵢ is identical or different, and independently represents a covalent bond or a linking group respectively and, and Rᵢ and Rₖ are not covalent bonds at the same time; m₀ is an integer of 1 to 6; n₀ is an integer of 1 to 6; each n₁ independently of one another represents an integer of 0 to 4; 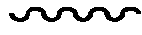 represents the site where the group is covalently linked.

In some embodiments, m₀ is an integer of 1 to 6, that is, the conjugate represented by Formula (2) comprises 1 to 6 of the diagnostic agent groups A₀. Considering delivery efficiency and synthesis cost, in some embodiments, m₀ is an integer of 1 to 4, that is, the conjugate represented by Formula (2) comprises 1 to 4 of the diagnostic agent groups A₀. In some embodiments, m₀ is 1, that is, the conjugate represented by Formula (2) comprises one of the diagnostic agent group A₀.

In some embodiments, n₀ is an integer of 1 to 6, that is, the conjugate represented by Formula (2) comprises 1 to 6 R_{AP} groups. Considering delivery efficiency and synthesis cost, in some embodiments, n₀ is an integer of 1 to 3, that is, the conjugate represented by Formula (2) comprises 1 to 3 R_{AP} groups. In some embodiments, n₀ is 1, that is, the conjugate represented by Formula (2) comprises one R_{AP} group.

In some embodiments, each n₁ independently of each other represents an integer of 0 to 4, and Rᵢ and Rₖ are not covalent bonds at the same time, so that each R_{AP} group comprises 1 to 5 delivery groups AP obtained from the aptamer of the present disclosure. In some embodiments, each n₁ independently of each other represents an integer of 0 to 1, so that each R_{AP} group comprises 1-2 delivery groups AP. In some embodiments, n₀ is 1 and n₁ is 0; in thi case, the conjugate represented by Formula (2) comprises one functional group AP.

In the R_{AP} group, the function of Rₖ and Rᵢ is to covalently link the delivery group AP to the Rⱼ group, and link to the diagnostic agent group A₀ through the Rⱼ group. Therefore, as long as the above-mentioned linkage can be achieved, any Rₖ or Rᵢ that has no negative affects on the delivery group AP and the diagnostic agent group A₀ can be used in the present invention. In some embodiments, each of the Rₖ and/or each of the Rᵢ is independently a straight-chain alkylene having a length of 1-70 carbon atoms, or one or more carbon atoms in the straight-chain alkylene are replaced by one or more groups selected from the group consisting of: C(O), NH, O, S, CH=N, S(O)₂, OP(O)₂, OP(O)(S), C₅-C₈ glycosidylene, C₂-C₁₀ alkenylene, C₂-C₁₀ alkynylene, C₆-C₁₀ arylene, C₃-C₁₈ heterocyclylene, and C₅-C₁₀ heteroarylene; and wherein the straight-chain alkylene may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl), and -NHSO₂(C₁-C₁₀ haloalkyl).

Considering synthesis cost and synthesis difficulty and the tumor-targeting effect of the conjugates, in some embodiments, each n₁ is independently 0 or 1, and each Rᵢ is independently one following linking group, or one connection combination of more than one following linking groups: C₁-C₂₀ alkylene, phosphate ester bond, phosphorothioate bond, amide bond, ester bond, ether bond, thioether bond, disulfide bond, 1,2,3- triazolylene group, a polyethylene glycol subunit, pyrrolidinylene group, 2-oxopyrrolidinylene group, phenylene, cyclohexylene, 2-succinimidylene group, 2-thiosuccinimidylene group, an amino acid subunit and a nucleotide subunit. In some embodiments, each Rᵢ is independently one following linking group, or one connection combination of two following linking groups: a covalent bond, a disulfide bond, a dodecylene group or a GAU trinucleotide subunit.

The function of the group Rⱼ is to connect the R_{AP} group and the diagnostic agent group A₀, thereby the diagnostic agent group A₀ is specifically delivered to tumor tissues and/or cells via the tumor-targeting effect of the delivery group AP in the R_{AP} group. Therefore, any Rⱼ group that is capable of achieving the above-mentioned linkage without affecting the tumor-targeting function of the delivery group AP and the effect of the diagnostic agent group AP₀ could achieve the purpose of the present invention and solve the technical problem to be solved by the present invention. In some embodiments, after the conjugate represented by Formula (2) reaches tumor tissues and/or enters tumor cells, the cleavage of Rⱼ takes place, releasing the diagnostic agent molecule corresponding to the individual diagnostic agent group A₀. In some embodiments, the Rⱼ is not cleaved *in vivo,* while the presence of the Rⱼ group and the R_{AP} group in the conjugate would not affect the diagnostic effect of the diagnostic agent group A₀.

In some embodiments, Rⱼ is a covalent bond, m₀ is 1; in this case, the conjugate represented by formula (2) comprises one diagnostic agent group A₀ and n₀ R_{AP} groups, and each R_{AP} group is directly linked to the diagnostic agent group A₀. In some embodiments, each R_{AP} group is linked to the same atom of the diagnostic agent group A₀. In some embodiments, each R_{AP} group is linked to different atoms of the diagnostic agent group A₀.

In some embodiments, Rⱼ is a linking group, which comprises a main chain moiety, a side chain moiety, and a conjugation linking moiety.

The main chain moiety is respectively linked to the conjugation linking moiety and the side chain moiety. In some embodiments, the main chain moiety is a straight-chain alkylene group having a length of 1-70 carbon atoms, or one or more carbon atoms in the straight-chain alkylene group are replaced by one or more groups selected from the group consisting of: C(O), NH, O, S, CH=N, S(O)₂, OP(O)₂, C₅-C₈ glycosidylene, C₂-C₁₀ alkenylene, C₂-C₁₀ alkynylene, C₆-C₁₀ arylene, C₃-C₁₈ heterocyclylene, and C₅-C₁₀ heteroarylene; and wherein the straight-chain alkylene may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl) (C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl), and -NHSO₂(C₁-C₁₀ haloalkyl).

The side chain moiety is respectively linked to the main chain moiety and the R_{AP} group. In some embodiments, each side chain moiety is independently a covalent bond or a straight-chain alkylene group having a length of 1-70 carbon atoms, or one or more carbon atoms in the straight-chain alkylene group are replaced by one or more groups selected from the group consisting of: C(O), NH, O, S, CH=N, S(O)₂, OP(O)₂, C₅-C₈ glycosidylene, C₂-C₁₀ alkenylene, C₂-C₁₀ alkynylene, C₆-C₁₀ arylene, C₃-C₁₈ heterocyclylene, and C₅-C₁₀ heteroarylene; and wherein the straight-chain alkylene has any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl) (C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl), and -NHSO₂(C₁-C₁₀ haloalkyl).

The conjugation linking moiety is respectively linked to the main chain moiety and the diagnostic agent group A₀. In some embodiments, each conjugation linking moiety is independently a covalent bond, or one following linking structure, or one connection combination of more than one following linking structures: C₁-C₁₀ straight-chain alkylene, phosphate ester bond, phosphorothioate bond, amide bond, ester bond, ether bond, disulfide bond, 1,2,3-triazolylene group, a polyethylene glycol subunit, pyrrolidinylene group, 2-oxopyrrolidinylene group, phenylene, cyclohexylene, 2-succinimidylene group, 2-thiosuccinimidylene group, an amino acid subunit and a nucleotide subunit.

In some embodiments, the linking group Rⱼ comprises the linking groups that are well known to those skilled in the art to be useful for antibody-conjugated drugs. The linking group Rⱼ may be cleavable or non-cleavable. In some embodiments, the linking group Rⱼ may be cleavable. In the context, "cleavable" means that after the conjugate of the present disclosure targets a tumor, the covalent bond of the linking group Rⱼ undergoes cleavage in the tumor environment and/or within tumor cells, releasing an individual diagnostic agent group to produce a diagnostic effect. In some embodiments, the linking group Rⱼ comprises one or more of an active enzyme linking group, a sulfatase-cleavable linking group, a galactose-cleavable linking group, a lysosomal protease-sensitive linking group, a peptidyl linking group, a glucuronide linking group, an acid-sensitive cleavable linking group, or a glutathione-sensitive disulfide linking group. In some embodiments, the linking group Rⱼ comprises a peptidyl linking group. In some embodiments, the peptidyl linking group is selected from one or more of a valine-citrulline dipeptide linker (Val-Cit), an alanine-alanine dipeptide linker (Ala-Ala), a valine-alanine dipeptide linker (Val-Ala), a glycine-glycine-phenylalanine-glycine tetrapeptide linker (Gly-Gly-Phc-Gly). In some embodiments, the linking group Rⱼ is selected from one of N-succinimidyl 4-(2-pyridyldithio)butyrate (SPDB), N- succinimidyl-4-(2-thiopyridinylene) pentanoate (SPP), (S)-2-((S)-2-amino-3-methylbutanamido)-5-ureidopentanoic acid (Val-Cit-PAB-OH), N-succinimidyl-4-(N-maleimidomethyl)cyclohexane-1-carboxylate (SMCC) or 2-(phosphate-(CH₂)₆-S-)-maleimidohexanoyl-valine-citrulline-p-aminobenzylene. In some embodiments, the linking group Rⱼ comprises the linking groups listed in Mckertish CM, Kayser V. Advances and Limitations of Antibody Drug Conjugates for Cancer. Biomedicines. 2021 Jul 23; 9 (8): 872., which are incorporated herein by reference in their entirety. In some embodiments, the linking group Rⱼ is non-cleavable.

In some embodiments, the linking group Rⱼ comprises one or more of a valine-citrulline dipeptide linker (Val-Cit), a polyethylene glycol subunit, an iminohexylene, an N-succinimidyl group and a GAU trinucleotide linking group. In some embodiments, each Rᵢ is independently one following linking group, or one connection combination of two following linking groups: a covalent bond, a disulfide bond, a dodecylene group, a valine-citrulline dipeptide linker (Val-Cit), a polyethylene glycol subunit, an iminohexylene, an N-succinimidyl group or a GAU trinucleotide subunit.

In some embodiments, each of the conjugation linking moiety in the linking group Rⱼ is respectively linked to the main chain moiety and one of the diagnostic agent groups A₀; the number of the side chain moieties is n₀ and each side chain moiety is respectively linked to the main chain moiety and one of the R_{AP} groups. Thus, each diagnostic agent group A₀ and the R_{AP} group independently of one another are linked to a linking group Rⱼ. In some embodiments, all side chain moieties are linked to the same atom in the main chain moiety; alternatively, each side chain moiety is linked to different atoms in the main chain moiety.

In some embodiments, m₀ is 1, and the linking group Rⱼ comprises the structure as shown in Formula (301): 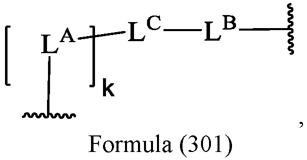 wherein k is an integer of 1 to 3; L^{C} is the main chain moiety, L^{A} is the side chain moiety, L^{B} is the conjugation linking moiety, and 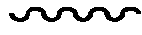 represents the site where the group is covalently linked.

The main chain moiety L^{C} is a covalent bond or a 2-4 valence, straight-chain or branched C₁-C₂₅ saturated hydrocarbyl, or one or more carbon atoms in the saturated hydrocarbyl are replaced by one or more groups selected from the group consisting of: C(O), NH, O, S, CH=N, S(O)₂, OP(O)₂, C₅-C₈ glycosidylene, C₂-C₅ alkenylene, C₂-C₅ alkynylene, C₆-C₁₀ arylene, C₃-C₈ heterocyclylene, and C₅-C₁₀ heteroarylene; and wherein the saturated hydrocarbyl may have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, -C₁-C₅ alkyl-OH, -SC₁-C₅ alkyl, nitro, -C(O)O(C₁-C₅ alkyl), -CON(C₁-C₅ alkyl)(C₁-C₅ alkyl), -CONH(C₁-C₅ alkyl), -CONH₂, -NHC(O)(C₁-C₅ alkyl), -NHC(O)(phenyl), -N(C₁-C₅ alkyl)C(O)(C₁-C₅ alkyl), -N(C₁-C₅ alkyl)C(O)(phenyl), -C(O)C₁-C₅ alkyl, -C(O)C₁-C₅ alkylphenyl, -OC(O)C₁-C₅ alkyl, -SO₂(C₁-C₅ alkyl), -SO₂(phenyl), -SO₂NH₂, -SO₂NH(C₁-C₅ alkyl), -SO₂NH(phenyl), -NHSO₂(C₁-C₅ alkyl), and -NHSO₂(phenyl). In some embodiments, L^{C} is a 2-4 valence, C₁-C₂₅ saturated hydrocarbyl, or one or more carbon atoms in the saturated hydrocarbyl are replaced by one or more groups selected from the group consisting of: C(O), NH, O, S, CH=N, S(O)₂, OP(O)₂, C₅-C₈ glycosidylene, C₂-C₅ alkenylene, C₂-C₅ alkynylene, C₆-C₁₀ arylene, C₃-C₈ heterocyclylene, and C₅-C₁₀ heteroarylene; and wherein the saturated hydrocarbyl may have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, -C₁-C₅ alkyl-OH, -SC₁-C₅ alkyl, nitro, and -CONH₂. In some embodiments, L^{C} is of 5-30 atoms in length, wherein the length of L^{C} refers to the number of the chain-forming atoms in the longest chain of atoms formed by the atom directly linked to L^{A} to the atom directly linked to L^{B} in L^{C}. To simplify the structure, in some embodiments, L^{C} is of 8-25 atoms in length.

The side chain moiety L^{A} is a covalent bond, or C₁-C₂₀ alkylene group, or one or more carbon atoms in the alkylene group are replaced by one or more groups selected from the group consisting of: C(O), NH, O, S, CH=N, S(O)₂, OP(O)₂, C₅-C₈ glycosidylene, C₂-C₅ alkenylene, C₂-C₅ alkynylene, C₆-C₁₀ arylene, C₃-C₈ heterocyclylene, and C₅-C₁₀ heteroarylene; and wherein the alkylene may have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, -C₁-C₅ alkyl-OH-SC₁-C₅ alkyl, -SC₁-C₅ alkylphenyl, -C₁-C₅ alkyl-SH, -OH, -SH, -NH₂, -C₁-C₅ alkyl-NH₂, -N(C₁-C₅ alkyl)(C₁-C₅ alkyl), -NH(C₁-C₅ alkyl), -N(C₁-C₅ alkyl) (C₁-C₅ alkylphenyl), -NH(C₁-C₅ alkylphenyl), nitro, -C(O)O(C₁-C₅ alkyl), -CON(C₁-C₅ alkyl)(C₁-C₅ alkyl), -CONH(C₁-C₅ alkyl), -CONH₂, -NHC(O)(C₁-C₅ alkyl), -NHC(O)(phenyl), -N(C₁-C₅ alkyl)C(O)(C₁-C₅ alkyl), -N(C₁-C₅ alkyl)C(O)(phenyl), -C(O)C₁-C₅ alkyl, -C(O)C₁-C₅ alkylphenyl, -OC(O)C₁-C₅ alkyl, -SO₂(C₁-C₅ alkyl), -SO₂(phenyl), -SO₂NH₂, -SO₂NH(C₁-C₅ alkyl), -SO₂NH(phenyl), -NHSO₂(C₁-C₅ alkyl), and -NHSO₂(phenyl).

The conjugation linking moiety L^{B} is one following linkage or one connection combination of 2-5 following linkages: phosphate ester bond, phosphorothioate bond, amide bond, ester bond, ether bond, disulfide bond.

In some embodiments, k is an integer of 1 to 3; L^{C} comprises any one of the groups represented by formula (L1) - (L3), and is linked to L^{A} moeity through an ether bond in the group represented by formula (L1) - (L3): 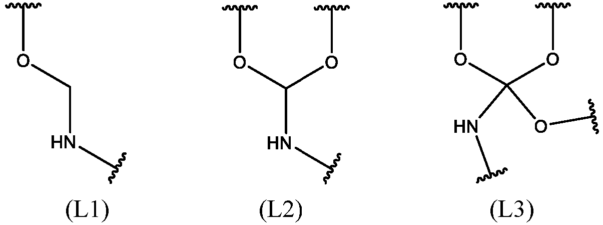 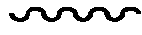 represents the site where a group is linked to the rest of the molecule.

In some embodiments, k = 1, L^{C} comprises a group represented by Formula (L1), and the oxygen atom in the group (L1) is directly linked to L^{A}. In some embodiments, k = 2, L^{C} comprises a group represented by Formula (L2), and each of two oxygen atoms in the group (L1) is directly linked to one L^{A}. In some embodiments, k = 4, L^{C} comprises a group represented by Formula (L3), and each of three oxygen atoms in the group (L1) is directly linked to one L^{A}.

L^{B} is a phosphate ester bond or a disulfide bond.

Each L^{A} is a covalent bond, or each L^{A} is selected from the group consisting of the groups (L4)-(L23) and the connection combinations thereof:

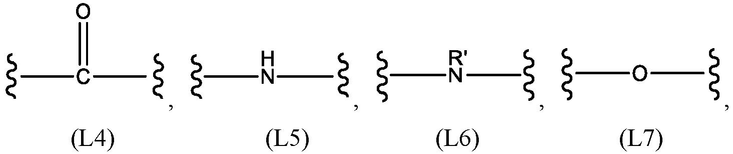

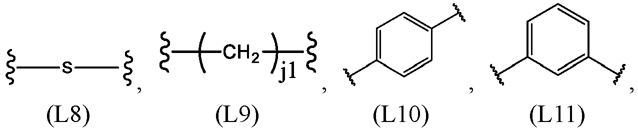

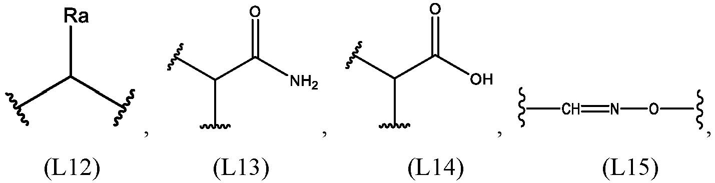

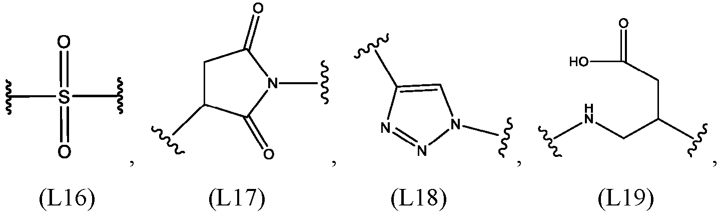

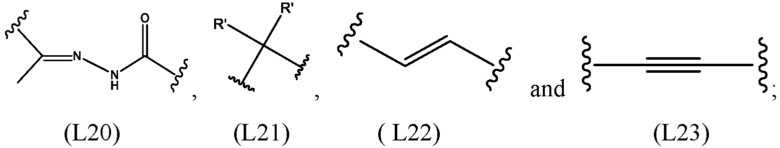

In the formula, each j 1 is an integer of 1 to 10;
each R' is a C₁-C₁₀ alkyl;
each Ra is a hydrogen atom, C₁-C₁₀ alkyl, or selected from the group consisting of the groups (L24)-(L37):

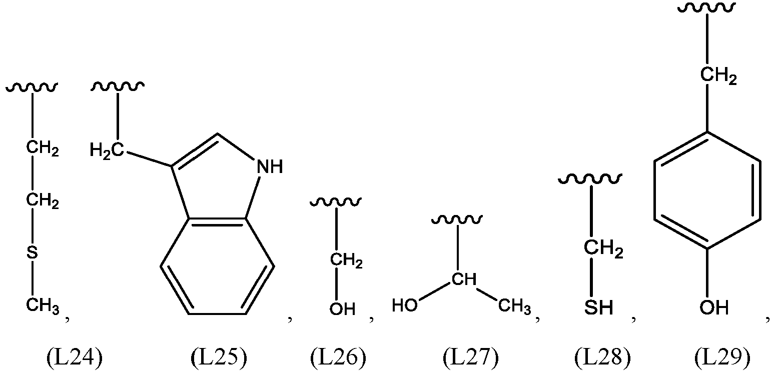

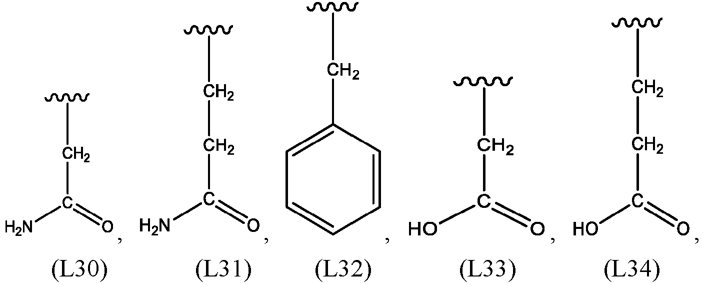

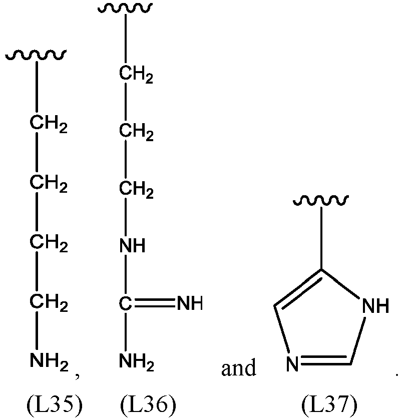

In some embodiments, L^{A} is of 3-35 atoms in length, wherein the length of the L^{A} refers to the number of the chain-forming atoms in the longest chain of atoms formed by the atom directly linked to L^{C} to the atom directly linked to R_{AP} in L^{A}. In some embodiments, each L^{A} is a connection combination of at least two of the groups (L4) to (L9), (L13), (L14), and (L18). In some embodiments, each L^{A} is a connection combination of at least two of the groups (L4), (L5), (L7), (L9), (L13), (L14) and (L18).

In some embodiments, L^{A} has an amide bond-containing structure as shown by Formula (302); L^{B} has a N-acylpyrrolidine-containing structure as shown by Formula (303), comprising carbonyl groups and oxygen atoms; L^{C} is a linking group based on (hydroxymethyl)aminomethane, bis(hydroxymethyl)aminomethane, or tris(hydroxymethyl)aminomethane: 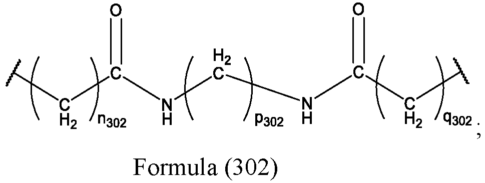 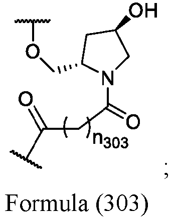 wherein n₃₀₂, q₃₀₂, and p₃₀₂ independently of one another are an integer of 2-6 and, optionally, n₃₀₂, q₃₀₂, and p₃₀₂ independently of one another are 2 or 3; n₃₀₃ is an integer of 4-16 and, optionally, n₃₀₃ is an integer of 8-12, and 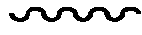 represents the site where a group is covalently linked.

In some embodiments, each of the side chain moieties L^{A} is linked to an R_{AP} group via a phosphate ester bond, an ether bond, or an ester bond, and is linked to said main chain moiety L^{C} by forming an ether bond with the oxygen atom of a hydroxyl group in the main chain moiety L^{C}; the conjugation linking moiety L^{B} is linked to the main chain moiety L^{C} by forming an amide bond between the nitrogen atom of the amino group in the main chain moiety L^{C} and the carbonyl group in Formula (303), and is linked to the functional group A₀ by forming a phosphate ester bond, an ether bond, or an ester bond between the oxygen atom in Formula (303) and the diagnostic agent group A₀. In some embodiments, the main chain moiety L^{C} is a linking group based on (hydroxymethyl)aminomethane, bis(hydroxymethyl)aminomethane, or tris(hydroxymethyl)aminomethane, and the main chain moiety L^{C} is linked to each of the side chain moieties L^{A} through an ether bond via the oxygen atom of the hydroxyl group, and is linked to the conjugation linking moiety L^{B} through an amide bond via the nitrogen atom of the amino group. Therefore, 1-3 side chains in the linking group Rⱼ are linked to the carbon atom of the same aminomethyl group, and are linked to delivery group-containing R_{AP} groups via the conjugation linking moiety L^{B}.

In some embodiments, the conjugate has a structure as shown by Formula (305):

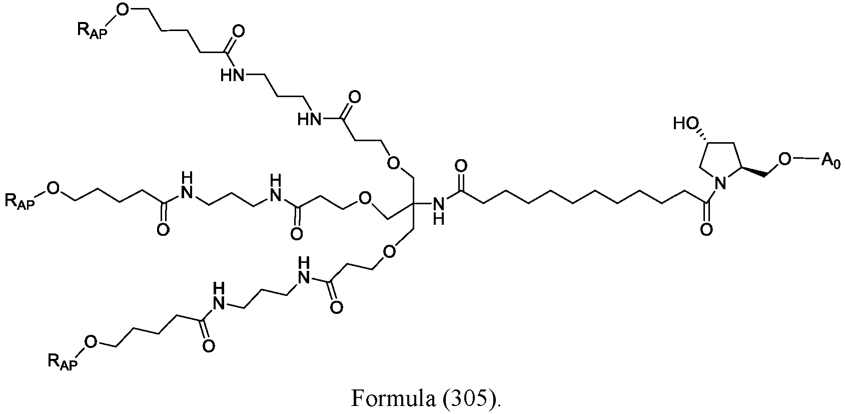

In some embodiments, the linking group Rⱼ comprises a structure as shown by Formula (306): 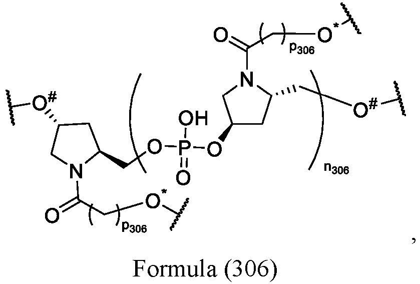 wherein n₃₀₆ is an integer of 0-3, each p₃₀₆ is independently an integer of 1-6, and 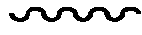 represents the site where a group is covalently linked; the structure formed by alternating pyrrolidinylene groups and phosphodiester group constitutes the main chain moiety, the atomic chain between the carbonyl group linked to the nitrogen atom on a pyrrolidinyl group and an oxygen atom marked with * constitutes each side chain moiety, and the side chain moiety is linked to an R_{AP} group by forming an ether bond between the oxygen atom marked with * and the R_{AP} group; at least one of the oxygen atoms marked with # is a conjugation linking moiety and is linked to the diagnostic agent group A₀ by forming an ether bond, an ester bond, or a phosphate ester bond with the diagnostic agent group A₀, and the other oxygen atom marked with # is linked to a hydrogen atom to form a hydroxyl group, or is linked to a C₁-C₃ alkyl group to form a C₁-C₃ alkoxy group. Therefore, 1-3 side chain moieties in the linking group Rⱼ are linked to different carbon atoms in the main chain moiety, and are linked to delivery-group-containing R_{AP} groups via an oxygen atom.

In some embodiments, the compound has a structure as shown by Formula (307a), (307b), or (307c):

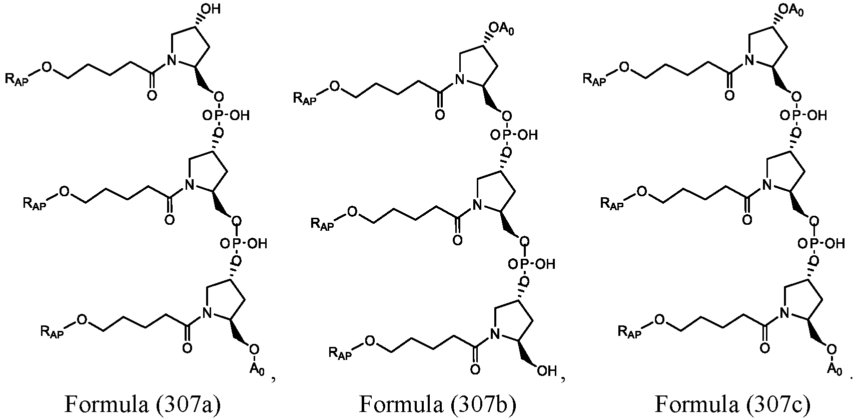

In some embodiments, the compound has a structure as shown by Formula (308): 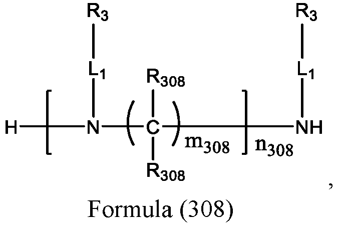 wherein n₃₀₈ may be an integer of 1-10; in some embodiments, in view of various factors such as synthesis convenience, structure/process costs, and tumor cell specificity, n₃₀₈ is an integer of 2-6. In some embodiments, n₃₀₈ is 3 or 4.

Each R₃ is independently a diagnostic agent group A₀, or a delivery group AP-containing R_{AP} group. In some embodiments, at least one R₃ is a diagnostic agent group A₀, and at least one R₃ is the R_{AP} group.

In some embodiments, when each m₃₀₈ is independently an integer of 2-10, it is considered that in the compound, the spatial position among multiple delivery group APs may be more suitable for interaction with corresponding receptors on the surface of tumor cells. In order to make the compound as shown by Formula (308) have simpler structure, easier synthesis, and/or reduced costs, according to some embodiments of the present disclosure, each m₃₀₈ is independently an integer of 2-5; in some embodiments, each m₃₀₈ is equal.

Those skilled in the art could understand that when each R₃₀₈ is independently selected from H, C₁-C₁₀ alkyl, C₁-C₁₀ haloalkyl, and C₁-C₁₀ alkoxy, they would not change the properties of the compound as shown by Formula (308) and could all realize the purpose of the present disclosure. In some embodiments, each R₃₀₈ is independently selected from H, methyl or ethyl. In some embodiments, each R₃₀₈ is H.

Each L₁ linked to the diagnostic agent group A₀ represents the conjugation linking moiety, and each L₁ linked to the R_{AP} represents the side chain moiety. In some embodiments, one R₃ is the diagnostic agent group A₀, and the other R₃ are the R_{AP} groups. In some embodiments, one or more L₁, as the side chain moieties, link the R_{AP} group to the N atom of the nitrogen-containing backbone; and the other one or more L₁, as the conjugation linking moieties, link the diagnostic agent group A₀ to the N atom on the nitrogen-containing backbone. The nitrogen-containing backbones together form the main chain moiety of the linking group Rⱼ. In the context of the present disclosure, a "nitrogen-containing backbone" refers to the chain structure in the structure as shown by Formula (308), wherein the N atom is coadjacently linked to the carbon atom where R₃₀₈ is linked.

Considering delivery efficiency and synthesis costs, in some embodiments, each L₁ independently has a length of 3-25 atoms. In some embodiments, each L₁ independently has a length of 4-15 atoms. Those skilled in the art would understand that, though L₁ is defined as a linear alkylene for convenience, it may not be a linear group or may have different names, such as an amine or alkenylene produced by the above replacement and/or substitution. For the purpose of the present disclosure, the length of L₁ is the number of the atoms in the chain linking the two attachment points. For this purpose, for the ring obtained by replacing a carbon atom in the straight-chain alkylene(e.g., a heterocyclylene or heteroarylene), the length of the portion in the chain corresponding to the ring is calculated according to the minimum number of atoms between attachment points on the ring.

In some embodiments, L₁ is selected from the group consisting of the groups as shown by Formulae L4-L23 above and any connection combination thereof. In some embodiments, each L₁ is independently selected from the group consisting of the connection combinations of at least two of the groups (L4)-(L9), (L13), (L14), and (L18). In some embodiments, each L₁ is independently a connection combination of at least two of the groups (L4), (L5), (L7), (L9), (L13), (L14), and (L18).

In some embodiments, in the conjugate as shown by Formula (308), each L₁ has both a site linking to the N atom on the nitrogen-containing backbone and a site linking to the diagnostic agent group A₀ or the R_{AP} group, and the site linking to the N atom on the nitrogen-containing backbone forms an amide bond with the N atom. In some embodiments, one or more L₁ are selected from B5, B6, B5', or B6': 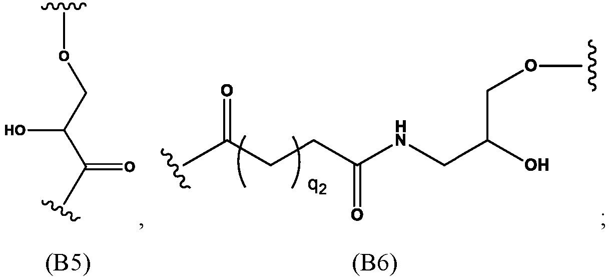 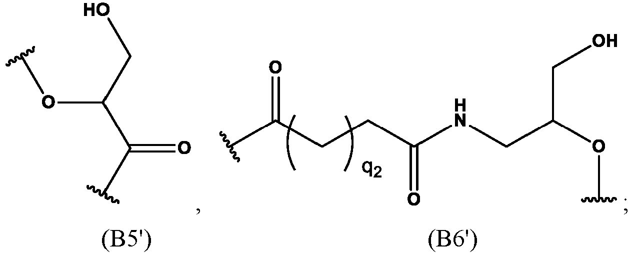 wherein 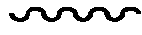 represents the site where a group is covalently linked, and q₂ is an integer of 1-10. In some embodiments, q₂ is an integer of 1-5.

In some embodiments, each R_{AP} group comprises one or more delivery groups. In some embodiments, the compound as shown by Formula (308) comprises multiple diagnostic agent groups. In some embodiments, each diagnostic agent group in the compound of Formula (308) is the same diagnostic agent group. In some embodiments, each diagnostic agent group in the compound of Formula (308) is a diagnostic agent group for the same purpose and function. In some embodiments, the compound of Formula (308) comprises different types of diagnostic agent groups for different purposes and functions.

In some embodiments, the compound as shown by Formula (308) has a structure as shown by Formula (403), (404), (405), (406), (407), (408), (409), (410), (411), (412), (413), (414), (415), (416), (417), (418), (419), (420), (421), (422), (423), (424), (425), (426), or (427):

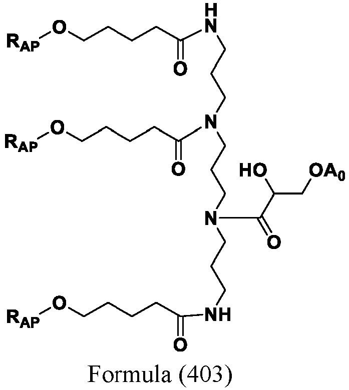

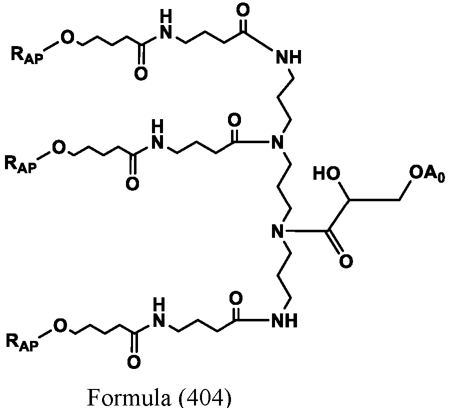

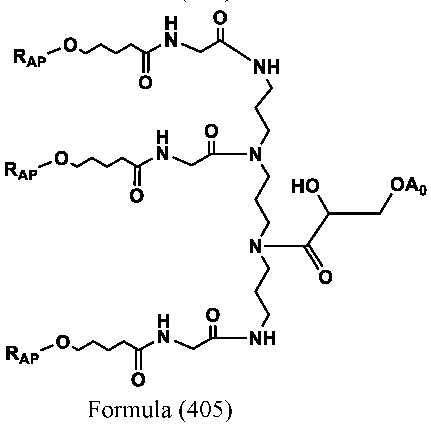

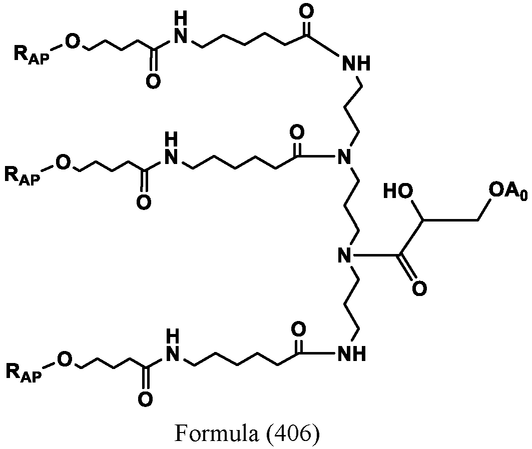

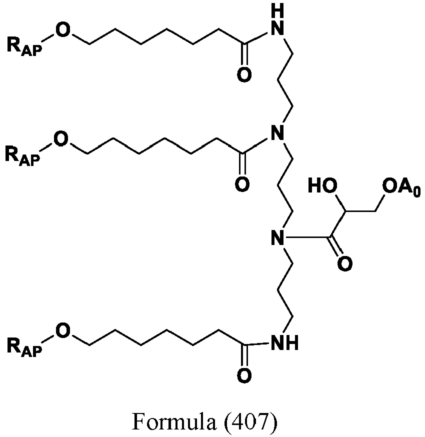

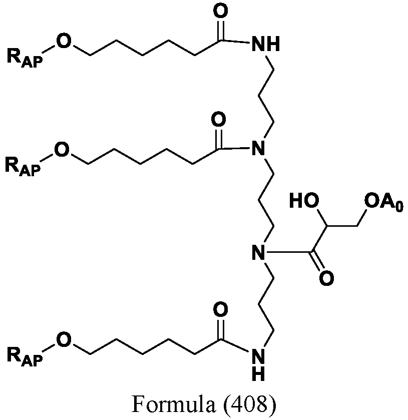

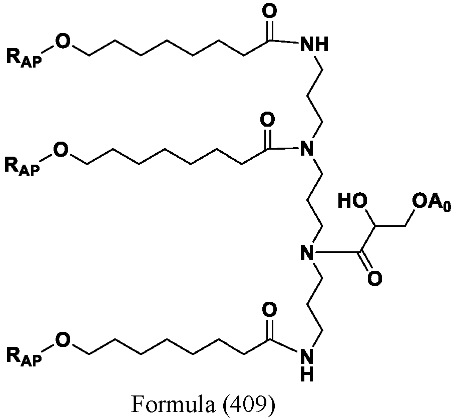

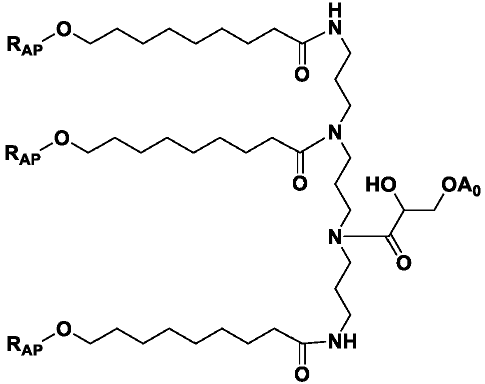

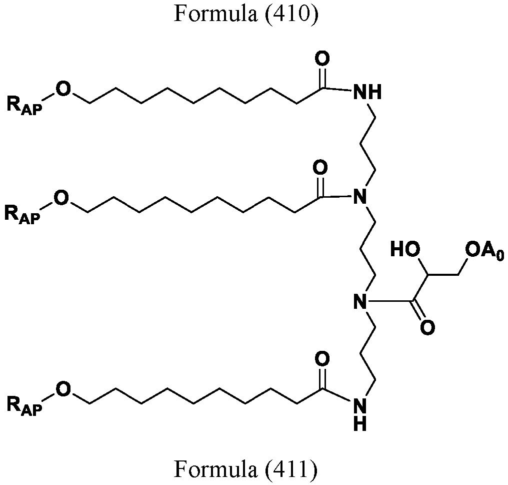

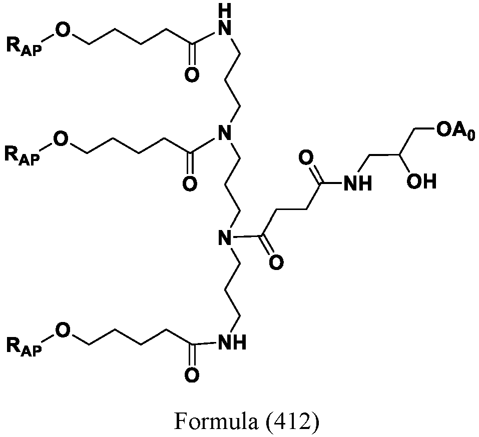

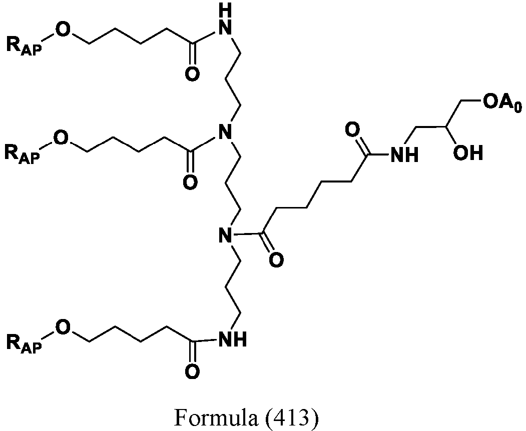

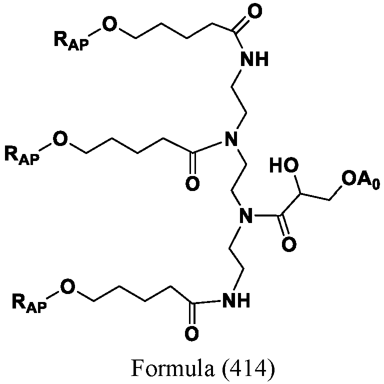

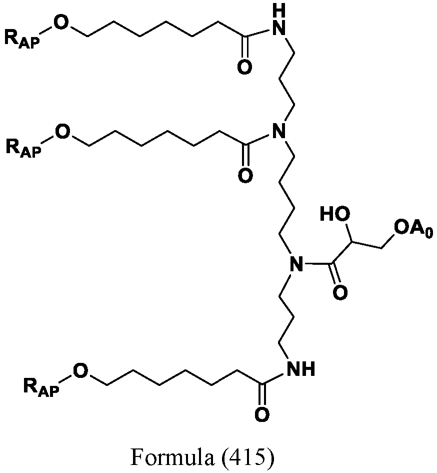

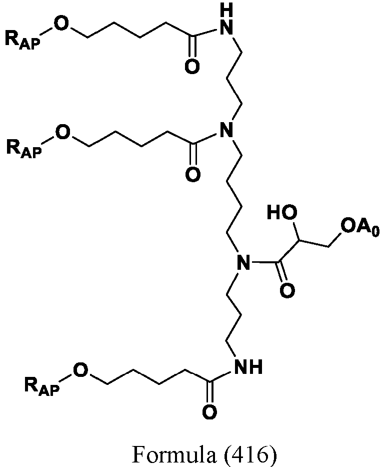

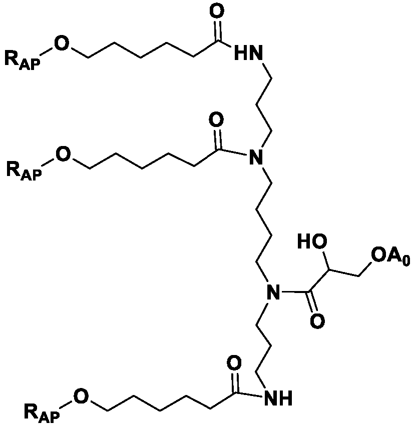

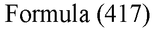

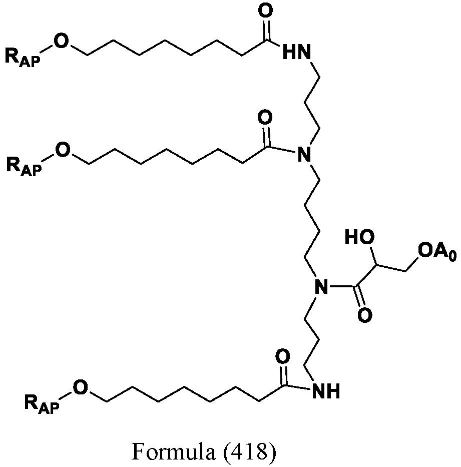

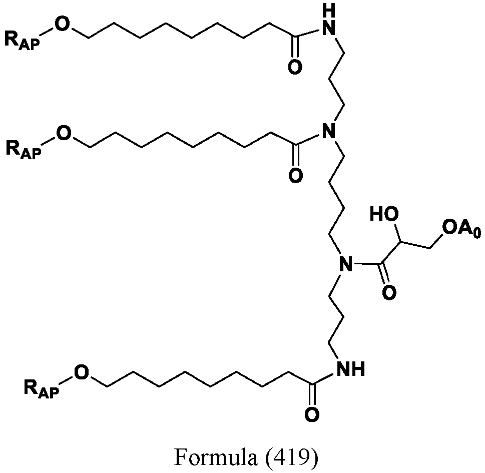

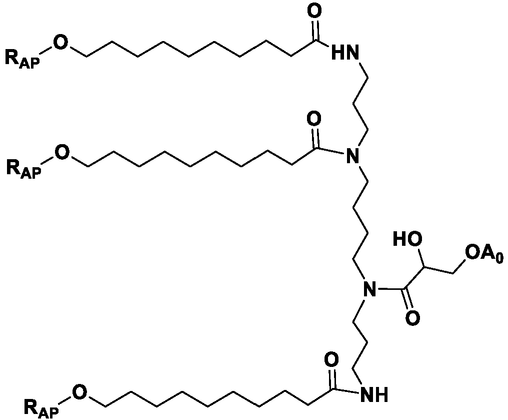

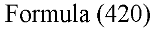

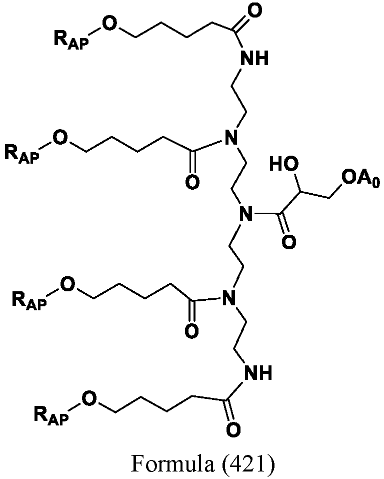

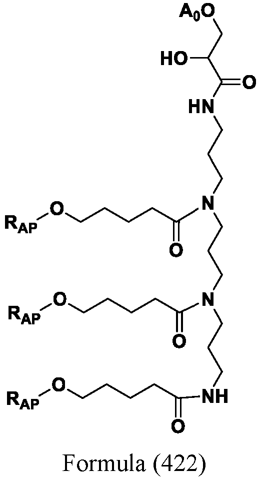

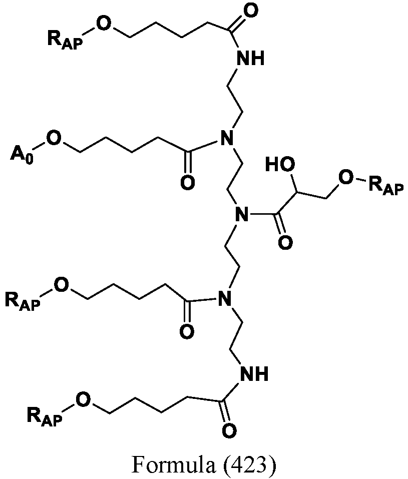

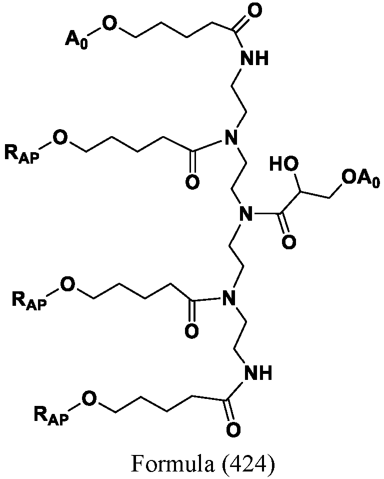

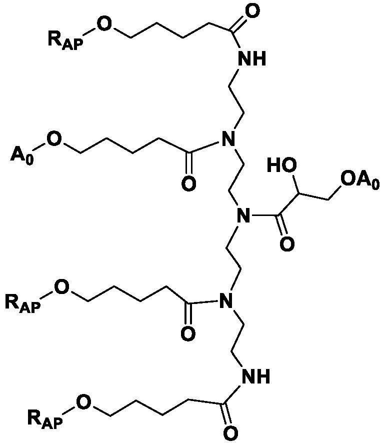

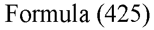

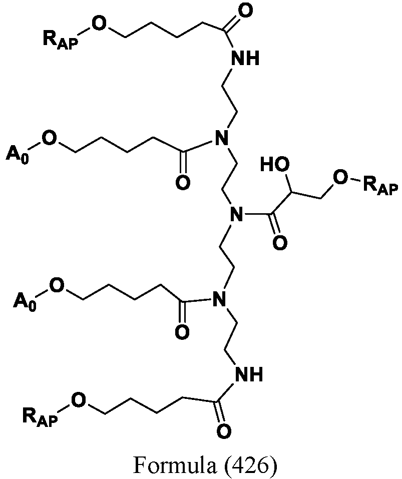

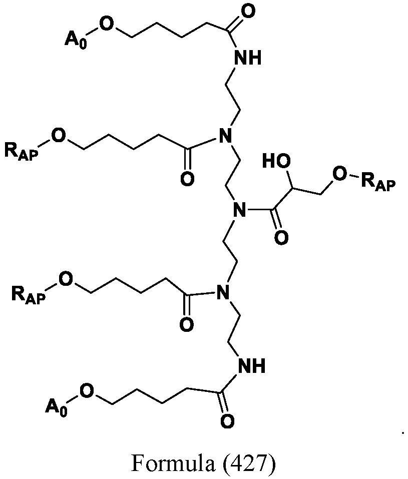

In some embodiments, the linking group Rⱼ comprises a nucleotide sequence I and a nucleotide sequence II, the nucleotide sequence I and the nucleotide sequence II each comprise 5-25 modified or unmodified nucleotides, and the nucleotide sequence I and the nucleotide sequence II are at least partly reverse complementary, the delivery group is linked to the nucleotide sequence I, the diagnostic agent group is linked to the nucleotide sequence II, and the nucleotide sequence I and the nucleotide sequence II do not elicit immune response or toxic reaction in a subject. In some embodiments, the nucleotide sequence I and the nucleotide sequence II are substantially reverse complementary or completely reverse complementary; or the nucleotide sequence I and the nucleotide sequence II have the same length and are both 10-20 modified or unmodified nucleotides; or the nucleotide sequence I and the nucleotide sequence II both consist of 17 nucleotides and are completely reverse complementary. In some embodiments, the 3' terminal of the delivery group is linked to the 5' position of the ribose in the 5'-terminal nucleotide of the nucleotide sequence I via a phosphate ester bond, and the diagnostic agent group is linked to the 5' position of the ribose in the 5'-terminal nucleotide of the nucleotide sequence II; or the diagnostic agent group comprises a segment of nucleotide sequence, and the 3' terminal is linked to the 5' position of the ribose in the 5'-terminal nucleotide of the nucleotide sequence II via a phosphate ester bond. In some embodiments, the nucleotide sequence I and the nucleotide sequence II respectively have the sequences as shown by SEQ ID NO: 40 and SEQ ID NO: 41:
5'- GUACAUUCUAGAUAGCC -3' (SEQ ID NO: 40)
5'- GGCUAUCUAGAAUGUAC -3' (SEQ ID NO: 41).

In some embodiments, the nucleotide sequence I and the nucleotide sequence II respectively have sequences as shown by SEQ ID NO: 42 and SEQ ID NO: 43:
5'- GmUfAmCfAmUfUfCfUfAmGmAmUfAmGmCfCf -3' (SEQ ID NO: 42)
5'- GmGmCfUfAmUfCfUfAmGmAmAmUfGmUfAmCf -3' (SEQ ID NO: 43)

The conjugate provided by the present disclosure may comprise one or more diagnostic agent groups. In the context of the present disclosure, a diagnostic agent group refers to a free radical-containing group formed by removing one or more atoms, functional groups, or some structural groups (e.g., removing one hydrogen atom) from the corresponding diagnostic agent compound), and the diagnostic agent group is covalently linked to the rest of the molecule of the conjugate provided by the present disclosure via the free radical.

In some embodiments, each of the diagnostic agent group is independently selected from contrast agent groups or fluorescence labelling tracer groups. By comprising a diagnostic agent group, the conjugate provided by the present disclosure can deliver the diagnostic agent group to tumors, and thus specifically, efficiently, and accurately diagnose relevant diseases and/or symptoms, such as, the presence and progression of tumors. In some embodiments, the diagnostic agent group may be used for diagnosing tumor cells/tissues in a sample to be tested in an *in vitro* experiment. In some embodiments, the diagnostic agent group may be used for diagnosing the presence and/or characteristic of tumor cells/tissues in a subject.

The contrast agent groups suitable for different photography technologies may be, for example, independently selected from the groups formed from one or more of the following contrast agents well-known to those skilled in the art: computed tomography (CT) contrast agents, nuclear magnetic resonance tomography (MRI) contrast agents, magnetic particle imaging contrast agents, and ultrasound (US) contrast agents. In some embodiments, at least one contrast agent group in the specific contrast agent compound of the present disclosure is a contrast agent group formed by an MRI contrast agent. In some embodiments, all contrast agent groups in the conjugates provided by the present disclosure are contrast agent groups formed from MRI contrast agents.

The contrast agent groups of different types of compounds may be, for example, iodine-containing groups, chelating-agent-containing groups and metal ions chelated by the chelatin agents, surface modification groups with surface-modified iron particles, or groups formed by perfluorinated compounds well-known to those skilled in the art.

In some embodiments, the computed tomography (CT) contrast agents can be selected from iothalamate, iohexol, meglumine diatrizoate, iopamidol, ethiodized oil preparations, and iopanoate.

In some embodiments, the nuclear magnetic resonance tomography (MRI) contrast agents or magnetic particle imaging contrast agents can be selected from gadolinium chelates, manganese chelates, chromium chelates, or iron particles.

In some embodiments, the ultrasound (US) contrast agents can be selected from perfluorinated compounds or perfluorinated compound analogues.

In some embodiments, the fluorescence labeling tracer groups (also referred to as fluorescence imaging groups or fluorescent groups in the art) may be groups formed from fluorescence labeling tracers, which can be selected from fluorescein, fluorescein derivatives, indocyanine green, Oregon green, Oregon green derivatives, rhodamine green, rhodamine green derivatives, eosin, erythrosin, Texas red, Texas red derivatives, malachite green, nanogold sulfosuccinimidyl ester, cascade blue, coumarin derivatives, naphthalene, pyridyloxazole derivatives, cascade yellow dye, and dapoxyl dye. In some embodiments, the fluorescence labeling tracer group is a Cy5 (Cyanine 5) fluorescent dye group or a Cy3 (Cyanine 3) fluorescent dye group.

The diagnostic agent group may be comprised in the conjugate provided by the present disclosure in any suitable manner. For example, the diagnostic agent group A₀ may be linked to the main chain moiety via the conjugation linking moiety above.

In some embodiments, the conjugate provided by the present disclosure further comprises at least one delivery aid group selected from one or more of C₁₀-C₃₀ hydrocarbyl, cholesteryl, and phospholipid groups. By comprising the delivery aid group, the conjugate provided by the present disclosure can be more compatible with the *in vivo* environment in the central nervous system, may have better bioavailability, and/or is delivered to tumors more effectively. In some embodiments, the delivery aid group is linked to the delivery group or the linking group via a covalent bond or a linking group. In some embodiments, the delivery aid group is linked to the diagnostic agent group.

Those skilled in the art may prepare the conjugate provided by the present disclosure using any appropriate synthetic route.

In some embodiments, the synthesis method of the conjugate provided by the present disclosure comprises contacting a protected conjugate with a deprotection reagent in a solvent under deprotection reaction condition, and isolating the conjugate provided by the present disclosure. The protected conjugate is a compound formed by protecting all active functional groups in the conjugate provided by the present disclosure with protecting groups. In some embodiments, the active functional groups include, but are not limited to, hydroxyl, amino, and/or phosphate group, and the protecting groups are correspondingly hydroxyl protecting groups, amino protecting groups, and/or phosphate hydroxyl protecting groups (e.g., cyanoethyl protecting groups). The solvent, deprotection reaction condition, and deprotection reagent to be used are selected and determined according to different protecting groups. In some embodiments, the deprotection reaction condition, solvent, and deprotection reagent are the deprotection reaction condition, solvent, and deprotection reagent used in solid phase synthesis of nucleic acids. In some embodiments, the method comprises adding the protected conjugate to a mixed solution of methylamine aqueous solution and aqueous ammonia, and the deprotection reaction condition comprises reacting for 1-5 h under normal temperature and pressure. In some embodiments, the methylamine aqueous solution and saturated concentrated aqueous ammonia are mixed in equal volumes to obtain the mixed solution, whose amount is 0.1-10 mL/µmol relative to the protected conjugate. In some embodiments, the isolation comprises performing purification through column chromatography separation, collecting product eluate, and removing solvent. Purification condition may be, for example, performing elution by using a preparative ion chromatography purification column with a gradient eluant of sodium chloride solution and sodium phosphate solution. In some embodiments, gradient elution is performed with: eluant A: 20 mM sodium phosphate (pH 8.1), solvent is water/acetonitrile in 9:1 (v/v); eluant B: 1.5 M sodium chloride, 20 mM sodium phosphate (pH 8.1), solvent is water/acetonitrile in 9:1 (v/v); elution gradient: the ratio of eluant A: eluant B = 100:0-50:50.

In some embodiments, the conjugate provided by the present disclosure has a structure as shown by Formula (101). The synthesis method of the protected conjugate comprises: contacting a compound comprising an active group Rₓ₁ and a delivery group with a compound comprising an active group Rₓ₂ and a diagnostic agent group in an organic solvent under coupling reaction condition, and reacting to obtain the protected conjugate, wherein the delivery group is formed by removing one or more hydrogen atoms or one or more functional groups from the aptamer provided by the present disclosure, wherein all active groups in the delivery group and diagnostic agent group are protected by protecting groups, and the active group Rₓ₁ and the active group Rₓ₂ are groups capable of reacting to form a covalent bond or linking group Rⱼ. In some embodiments, the molar ratio of the delivery group linked with an active group Rₓ₁ to the functional group linked with an active group Rₓ₂ is m₀:n₀. In some embodiments, active groups in the delivery group and diagnostic agent group include, but are not limited to, one or more of hydroxyl, amino, and phosphate group, and the protecting group is correspondingly one or more of hydroxyl protecting group, amino protecting group, and phosphate hydroxyl protecting group (e.g. cyanoethyl protecting group).

Those skilled in the art could obtain the compound comprising an active group Rₓ₁ and a delivery group by various methods. In some embodiments, the compound comprising an active group Rₓ₁ and a delivery group may be obtained by nucleic acid synthesis methods well-known to those skilled in the art, for example, phosphoramidite solid phase synthesis or liquid phase synthesis by phosphodiester method/phosphotriester method. In some embodiments, the compound comprising an active group Rₓ₁ and a delivery group is obtained using the phosphoramidite solid phase synthesis method. The method comprises sequentially linking nucleoside monomers according to the sequence of the nucleotides in an oligonucleotide single strand under condition for phosphoramidite solid phase synthesis , wherein at least one nucleoside monomer is a nucleoside monomer; or after linking all nucleoside monomers, linking a phosphoramidite monomer with an active group Rₓ₁ according to the phosphoramidite solid phase synthesis method, or linking a protected phosphoramidite monomer, and then removing the protecting group to form the active group Rₓ₁. The phosphoramidite solid phase synthesis method is well-known to those skilled in the art, whose process and conditions are disclosed in detail in Methods in Molecular Biology, vol. 288: Oligonucleotide Synthesis: Methods and Applications, P17-P31, which is incorporated herein by reference in its entirety.

In some embodiments, the coupling reaction condition is the condensation reaction condition or the condition for thiol-disulfide exchange reaction.

In some embodiments, the coupling reaction condition is the condensation reaction condition, which is acylation condensation reaction condition, dehydration condensation reaction condition, or click chemistry reaction condition, and the active group Rₓ₁ and active group Rₓ₂ are groups capable of undergoing the condensation reaction above. In some embodiments, the condensation reaction condition is acylation condensation reaction condition, and the active groups Rₓ₁ and Rₓ₂ are groups capable of undergoing acylation condensation reaction to form R_{I}. In some embodiments, the condensation reaction condition is dehydration condensation reaction condition, and one of the active groups Rₓ₁ and Rₓ₂ is a group comprising an acyl halide or carboxyl group, and the other is a group comprising an amino or hydroxyl group. In some embodiments, the condensation reaction condition is click chemistry reaction condition, and one of the active groups Rₓ₁ and Rₓ₂ is a group comprising an alkynyl group, and the other is a group comprising an azide group. In some embodiments, the condensation reaction condition is Michael addition reaction condition, and one of the active groups Rₓ₁ and Rₓ₂ is a group comprising a thiol group, and the other is a group comprising a succinimidyl group. In some embodiments, the condensation reaction condition is the condition for *N-*hydroxysuccinimide-carbodiimide (NHS-EDC) coupling reaction, and one of the active groups Rₓ₁ and Rₓ₂ is a group comprising *N*-hydroxysuccinimide (NHS) , and the other is a group comprising a carbodiimide (EDC).

In some embodiments, the compound comprising an active group Rₓ₁ and a delivery group is prepared by contacting the aptamer provided by the present disclosure having an active group Rₓ₀ with a cross-linking agent under coupling reaction condition, and the cross-linking agent comprises a click chemistry active group and an acylation group. The active group Rₓ₀ and the acylation group are covalently linked through coupling reaction, linking the click chemistry active group to the aptamer provided by the present disclosure.

In some embodiments, an active group Rₓ₁ is an active group comprising 1-3 click chemistry active groups at its end, wherein the click chemistry active group comprises a terminal alkynyl group. In some embodiments, the acylation group is an active ester group, for example, one of an NHS ester group, an imidate group, and a pentafluorophenyl ester group. Those skilled in the art could obtain the cross-linking agent by various methods. For example, when the acylation group is a pentafluorophenyl ester group and the click chemistry group comprises a terminal alkynyl group, the cross-linking agent may be prepared according to the method as described in Scheme 1a (A) in Østergaard, Michael E., et al. "Efficient synthesis and biological evaluation of 5'-GalNAc conjugated antisense oligonucleotides." Bioconjugate chemistry 26.8 (2015): 1451-1455, which is incorporated herein by reference in its entirety. In some embodiments, the active group Rₓ₀ is an amino group. In some embodiments, the coupling condition is basic condition. In some embodiments, the basic condition is the condition in which an weak base aqueous solution is present, for example the condition in which sodium bicarbonate aqueous solution is present.

Those skilled in the art could obtain the aptamer with an active group Rₓ₀ by various methods. In some embodiments, the aptamer with an active group Rₓ₀ is prepared by using a phosphoramidite monomer comprising an active group at the corresponding position during aptamer synthesis. Those skilled in the art could obtain a phosphoramidite monomer with an active group by various methods. In some embodiments, the active group Rₓ₀ is an amino group, and a phosphoramidite monomer with an Rₓ₀ can be commercially available or prepared by the methods well-known to those skilled in the art. For example, the phosphoramidite monomer with an Rₓ₀ can be readily commercially available 6-(trifluoroacetylamino)-hexyl-(2-cyanoethyl)-(N,N-diisopropyl)-phosphoramidite monomer, wherein the active group Rₓ₀ is an amino group. The active group Rₓ₀ may be obtained by linking the phosphoramidite monomer to an oligonucleotide single strand, and then removing the trifluoroacetyl protecting group through deprotection reaction readily realized by those skilled in the art (e.g., ammonolysis by concentrated aqueous ammonia) according to the phosphoramidite solid phase synthesis method. In these cases, the active group Rₓ₀ linked by the phosphoramidite solid phase synthesis method is linked to the 5' position of the ribose in the 5'-terminal nucleotide of the aptamer via a phosphodiester bond.

In some embodiments, the coupling reaction condition is one of the conditions for thiol-disulfide exchange reaction, and one of the active groups Rₓ₁ and Rₓ₂ is a group comprising a thiol group, and the other comprises a leaving group linked by a disulfide bond. To avoid side reactions, in some embodiments, Rₓ₁ in the above phosphoramidite monomer with an active group Rₓ₁ is present in a form of protected Rₓ₁', and the preparation method further comprises the step of isolating the compound comprising an active group Rₓ₁ and a delivery group by contacting the prepared compound comprising the protected active group Rₓ₁' and a delivery group with a deprotection reagent under deprotection reaction condition. In some embodiments, the Rₓ₁' comprises a disulfide bond leaving group, the deprotection reaction condition is the condition for thiol-disulfide exchange reaction, and the deprotection reagent is a disulfide bond activator. In some embodiments, the disulfide bond activator is dithiodipyridine. Those skilled in the art could obtain the above phosphoramidite monomer with an active group Rₓ₁ or Rₓ₁' by various methods. In some embodiments, the phosphoramidite monomer with an active group Rₓ₁ or Rₓ₁' is commercially purchased. For example, the phosphoramidite monomer as shown by Formula (105) is commercially available. 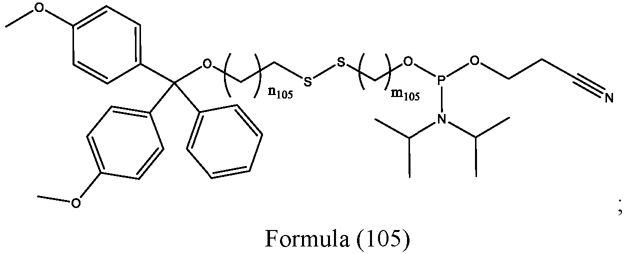 wherein n₁₀₅ and m₁₀₅ independently of one another are an integer of 1-10.

Those skilled in the art could obtain the compound comprising an active group Rₓ₂ and a diagnostic agent group by various methods. In some embodiments, the coupling reaction condition is the condition for thiol-disulfide exchange reaction, the active group Rₓ₂ comprises a thiol group, and the compound comprising an active group Rₓ₂ and a diagnostic agent group may be obtained by those skilled in the art via various known methods. For example, it may be prepared by using a phosphoramidite monomer comprising a thiol group according to the phosphoramidite solid phase synthesis method, or may be commercially purchased. In some embodiments, the coupling reaction condition is the condition for phosphoramidite solid phase synthesis reaction, the active group Rₓ₂ is a phosphoramidite group, and the compound comprising an active group Rₓ₂ and a diagnostic agent group may be, for example, a compound comprising a phosphoramidite group and a diagnostic agent group which can be readily commercially customized or obtained by the phosphoramidite solid phase synthesis method. In some embodiments, the coupling reaction condition is Michael addition reaction condition, the active group Rₓ₂ is a *N*-succinimidyl group, and the compound comprising an active group Rₓ₂ and a diagnostic agent group may be, for example, a compound comprising a N-succinimidyl group and a diagnostic agent group which can be readily commercially customized or obtained by the phosphoramidite solid phase synthesis method. Therein, the N-succinimidyl group may be linked to the diagnostic agent by using a readily commercially available phosphoramidite monomer containing the N-succinimidyl group according to the phosphoramidite solid phase synthesis method. In some embodiments, the active group Rₓ₁ and active group Rₓ₂ are respectively a nucleotide sequence I and a nucleotide sequence II, each of which comprises 5-25 modified or unmodified nucleotides, and the nucleotide sequence I and the nucleotide sequence II are at least partly reverse complementary. The delivery group is linked to the nucleotide sequence I, the diagnostic agent group is linked to the nucleotide sequence II, and the nucleotide sequence I and the nucleotide sequence II do not elicit immune response or toxic reaction in a subject. The coupling reaction condition is the reaction condition for annealing to form a nucleic acid double strand. In some embodiments, the nucleotide sequence I and the nucleotide sequence II both consist of 17 nucleotides and are completely reverse complementary. In some embodiments, the nucleotide sequence I and the nucleotide sequence II respectively have the sequences as shown by SEQ ID NO: 40 and SEQ ID NO: 41. In some embodiments, the nucleotide sequence I and the nucleotide sequence II respectively have the sequences as shown by SEQ ID NO: 42 and SEQ ID NO: 43.

The delivery group is formed by removing one or more hydrogen atoms or functional groups from an aptamer. In some embodiments, the group at the 5'position of the ribose in the 5'-terminal nucleotide of the consecutive nucleotide sequence and the group at the 3'position of the ribose in the 3'-terminal nucleotide of the consecutive nucleotide sequence are both hydroxyl groups, and the delivery group is formed by removing one hydrogen atom from the 5'-hydroxyl group of the 5'-terminal nucleotide of the above aptamer. In some embodiments, the delivery group is formed by removing one hydrogen atom from the 3'-hydroxyl group of the 3'-terminal nucleotide of the above aptamer. In some embodiments, the delivery group is formed by removing the 5'-hydroxyl group from the 5'-terminal nucleotide of the aptamer of the present disclosure. In some embodiments, a delivery group is formed by removing the 3'-hydroxyl group from the 3'-terminal nucleotide of the aptamer of the present disclosure. In some embodiments, the delivery group is formed by removing the 2'-hydroxyl group of the ribose from a nucleotide contained in the aptamer of the present disclosure. The aptamer may be obtained by a conventional method for preparing an aptamer in the art (e.g., solid phase synthesis and liquid phase synthesis methods of nucleic acids). Therein, the nucleic acid solid phase synthesis has commercial customized services. A modified nucleotide can be introduced into the aptamer of the present disclosure by using a nucleoside monomer with a corresponding modification. The methods for preparing a nucleoside monomer with a corresponding modification and the methods for introducing a modified nucleotide into the aptamers are also well known to those skilled in the art. All nucleoside monomers with modifications can be commercially available or prepared by known methods.

In some embodiments, the conjugate may also be used in the present disclosure in the form of a pharmaceutically acceptable salt or a precursor compound thereof. In the context of the present disclosure, a "pharmaceutically acceptable salt" refers to a corresponding salt formed by a drug to increase the stability, solubility, and/or bioavailability of the drug without pharmaceutically producing additional side effects on the human body, such as potassium salt, sodium salt, or carboxylate. A "precursor compound" refers to a compound that is not identical with the conjugate in terms of structure and function, but could react and form the conjugate provided by the present disclosure after entering into the body or in the body fluid environment, thereby exerting its effect and achieving the purpose of the present disclosure. Under certain circumstances, these precursor compounds can increase the stability, extend the slow-release time, increase the bioavailability, etc. of the drug. In some embodiments, the precursor compound comprises the precursor groups capable of reacting in the human body to form all diagnostic agent groups A₀ in a conjugate. In some embodiments, the precursor compound includes a compound formed by substituting all active hydroxyl groups in a conjugate with acetoxy groups. In some embodiments, the precursor compound comprises a drug precursor group, which is a residue formed by a precursor compound of the diagnostic agent group in a conjugate. In some embodiments, the drug precursor group may be, for example, a group formed by substituting active hydrogen in a hydroxyl or amino functional group in the diagnostic agent group with an acyl, alkyl, or phosphoryl group. Those skilled in the art could understand that use of these pharmaceutically acceptable salts and precursor compounds are also within the scope of the present disclosure.

### Pharmaceutical composition

In one aspect, the present disclosure further provides a pharmaceutical composition, comprising the conjugate provided by the present disclosure and a pharmaceutically acceptable carrier.

The pharmaceutically acceptable carrier may be a carrier conventionally used in the art, for example, but not limited to, one or more of water, normal saline, magnetic nanoparticles (such as Fe₃O₄-based or Fe₂O₃-based nanoparticles), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In some embodiments, the pharmaceutically acceptable carrier comprises a physiologically acceptable compound, which exerts the function of, for example, stabilizing the pharmaceutical composition or increasing or decreasing the absorption of the aptamer, conjugate and/or the pharmaceutical composition. The physiologically acceptable compound is selected from one or more of the following compounds: carbohydrates, such as glucose, sucrose, and/or glucan; antioxidants, such as ascorbic acid and/or glutathione; chelating agents; low molecular weight proteins; compositions that reduce the clearance or hydrolysis of any co-administered substance; excipients; stabilizers and buffers. A detergent may also be used for stabilizing the composition or increasing or decreasing the absorption of the pharmaceutical composition. The physiologically acceptable compound may further include one or more of wetting agents, emulsifiers, dispersants, or preservatives especially used for preventing microbial growth or action. The physiologically acceptable compound is known to those skilled in the art, which is not described here in detail. Those skilled in the art could easily understand that the choices of the pharmaceutically acceptable carrier and physiologically acceptable compound depend on, for example, administration routes and specific physiochemical characteristics of any co-administered substance.

In some embodiments, the pharmaceutically acceptable carrier is sterile and usually does not comprise an undesirable substance. The pharmaceutical composition of the present disclosure may further comprise pharmaceutically acceptable auxiliary substances according to requirements to approach physiological conditions, such as pH regulators and buffers and toxicity regulators, for example sodium acetate, sodium chloride, potassium chloride, calcium chloride, and sodium lactate. The concentration of the conjugate provided by the present disclosure in the pharmaceutical composition may vary within a wide range, and is selected mainly according to fluid volume, viscosity, body weight, etc. and according to the specific administration manner.

In some embodiments, in the pharmaceutical composition, there are no special requirements for the contents of the conjugate and the pharmaceutically acceptable carrier. In some embodiments, the weight ratio of the conjugate to the pharmaceutically acceptable carrier may be 1:(1-500), and in some embodiments, the above weight ratio is 1:(1-50).

In some embodiments, the pharmaceutical composition may also comprise other pharmaceutically acceptable adjuvants, which may be one or more of various formulations and compounds conventionally used in the art. For example, said other pharmaceutically acceptable adjuvants may comprise at least one of a pH buffer, a protection agent, and an osmotic pressure regulator.

The pH buffer may be a tris(hydroxymethyl)aminomethane hydrochloride buffer solution with a pH of 7.5-8.5, and/or a phosphate buffer solution with a pH of 5.5-8.5, such as a phosphate buffer solution with a pH of 5.5-8.5.

The protection agent may be at least one of inositol, sorbitol, sucrose, trehalose, mannose, maltose, lactose, and glucose. The content of the protection agent may be from 0.01 wt% to 30 wt% based on the total weight of the pharmaceutical composition.

The osmotic pressure regulator may be sodium chloride and/or potassium chloride. The content of the osmotic pressure regulator allows the osmotic pressure of the pharmaceutical composition to be 200-700 mOsm/kg. Depending on the desired osmotic pressure, those skilled in the art can readily determine the content of the osmotic pressure regulator. In some embodiments, the dose of the formulation prepared from the pharmaceutical composition will be adjusted according to different administration manners during administration.

In some embodiments, the pharmaceutical composition may be a liquid formulation, for example, an injection solution; or a lyophilized powder for injection, which will be mixed with a liquid adjuvant to form a liquid formulation upon administration. The liquid formulation may be used for, but not limited to, administration by subcutaneous, intramuscular, or intravenous injection, and the pharmaceutical composition may also be delivered by, but not limited to, puncture injection, oropharyngeal inhalation, nasal administration, or other routes. In some embodiments, the pharmaceutical composition is used for administration by subcutaneous, intramuscular, or intravenous injection.

In some embodiments, the pharmaceutical composition may be in the form of a liposome formulation. In some embodiments, the pharmaceutically acceptable carrier used in the liposome formulation comprises an amine-containing transfection compound (hereinafter also referred to as an organic amine), a helper lipid, and/or a PEGylated lipid. Therein, the organic amine, the helper lipid, and the PEGylated lipid may be respectively selected from one or more of the amine-containing transfection compounds or the pharmaceutically acceptable salts or derivatives thereof, the helper lipids, and the PEGylated lipids as described in the Chinese patent application CN103380113A, which is incorporated herein by reference in its entirety.

In some embodiments, the organic amine may be a compound as shown by Formula (201) or a pharmaceutically acceptable salt thereof as described in the Chinese patent application CN103380113A: 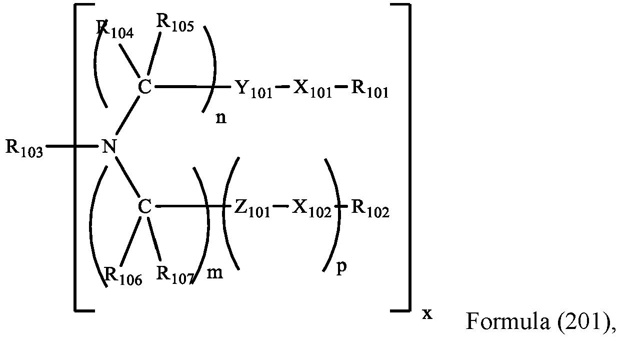 wherein:
X₁₀₁ and X₁₀₂ independently of one another are O, S, N-A or C-A, wherein A is hydrogen or a C₁-C₂₀ hydrocarbon chain;
Y₁₀₁ and Z₁₀₁ independently of one another are C=O, C=S, S=O, CH-OH or SO₂; R₁₀₁, R₁₀₂, R₁₀₃, R₁₀₄, R₁₀₅, R₁₀₆, and R₁₀₇ independently of one another are hydrogen; a cyclic or an acyclic, substituted or unsubstituted, branched or straight-line aliphatic group; a cyclic or an acyclic, substituted or unsubstituted, branched or straight-line heteroaliphatic group; a substituted or unsubstituted, branched or straight-line acyl group; a substituted or unsubstituted, branched or straight-line aryl group; or a substituted or unsubstituted, branched or straight-line heteroaryl group;
x is an integer of 1-10;
n is an integer of 1-3, m is an integer of 0-20, and p is 0 or 1, wherein if m = p = 0, then R₁₀₂ is hydrogen; and
if at least one of n and m is 2, then R₁₀₃ and the nitrogen in Formula (201) form a structure as shown by Formula (202) or (203): 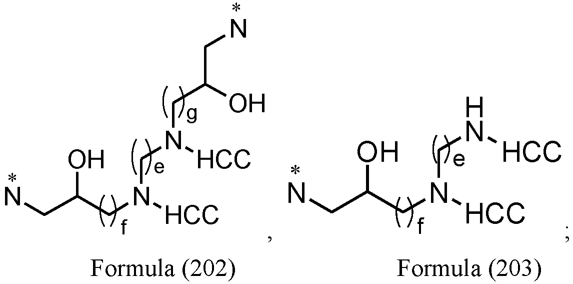
wherein g, e, and f independently of one another are an integer of 1-6, "HCC" represents a hydrocarbon chain, and each *N represents a nitrogen atom in Formula (201).

In some embodiments, R₁₀₃ is a polyamine. In other embodiments, R₁₀₃ is a ketal. In some embodiments, R₁₀₁ and R₁₀₂ in Formula (201) independently of one another are any substituted or unsubstituted, branched or straight-line alkyl or alkenyl, wherein the alkyl or alkenyl has 3 to about 20 carbon atoms (such as 8 to about 18 carbon atoms) and 0-4 double bonds (such as 0-2 double bonds).

In some embodiments, if n and m independently of one another are 1 or 3, R₁₀₃ may be any of the following Formulae (204)-(213): 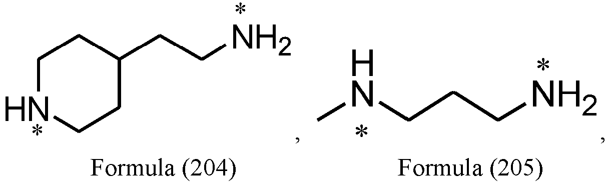 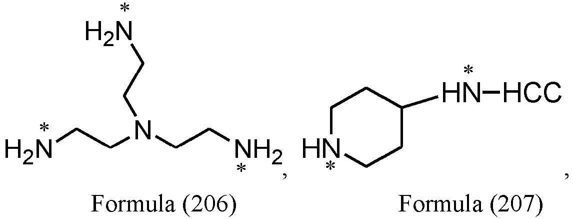 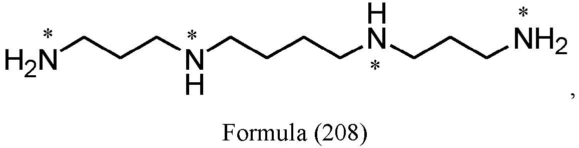 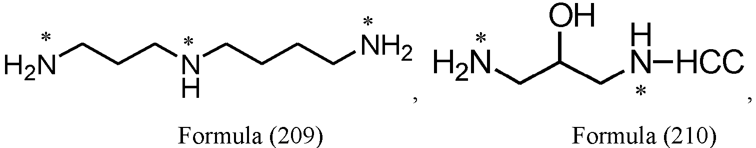 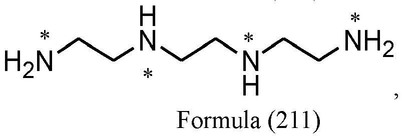 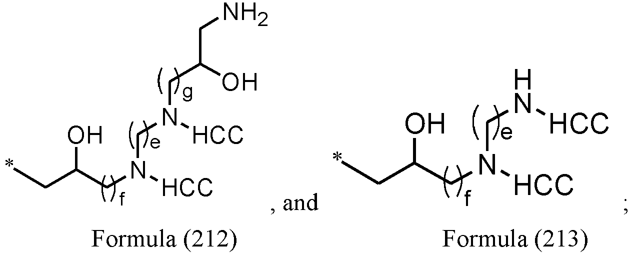 wherein, in Formulae (204)-(213), g, e, and f independently of one another are an integer of 1-6, each "HCC" represents a hydrocarbon chain, and each * represents a potential attachment point of R₁₀₃ to the nitrogen atom in Formula (201), wherein each H at any * position may be replaced to achieve the attachment to the nitrogen atom in Formula (201).

Those skilled in the art could obtain the compound as shown by Formula (201) by any appropriate method. In some embodiments, the compound as shown by Formula (201) may be prepared according to the description of the Chinese patent application CN103380113A.

In some embodiments, the organic amine is an organic amine as shown by Formula (214) and/or an organic amine as shown by Formula (215):

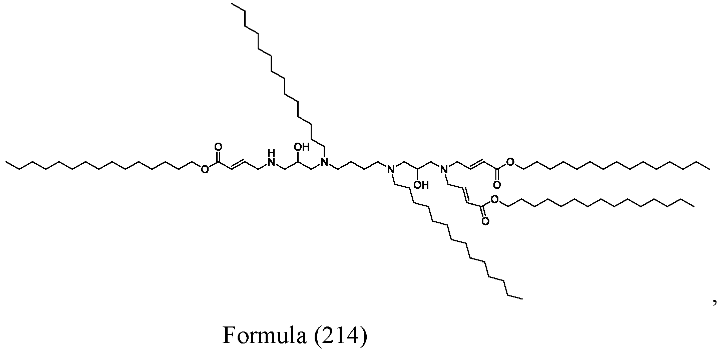

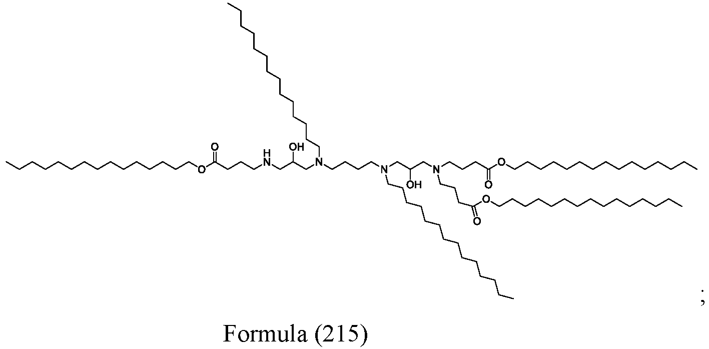

The helper lipid is cholesterol, a cholesterol analog, and/or a cholesterol derivative.

The PEGylated lipid is 1,2-dipalmitoyl-sn-glycero-3-phosphoethanolamine-N-[methoxy(polyethylene glycol)]-2000.

In some embodiments, the molar ratio among the organic amine, the helper lipid, and the PEGylated lipid in the pharmaceutical composition is (19.7-80):(19.7-80):(0.3-50), for example, the molar ratio may be (50-70):(20-40):(3-20).

In some embodiments, the pharmaceutical composition particles formed by the conjugate provided by the present disclosure and the above amine-containing transfection reagents have an average diameter of about 30 nm to about 200 nm, typically about 40 nm to about 135 nm, and more typically, the average diameter of the liposome particles is about 50 nm to about 120 nm, about 50 nm to about 100 nm, about 60 nm to about 90 nm, or about 70 nm to about 90 nm; for example, the average diameter of the liposome particles is about 30, 40, 50, 60, 70, 75, 80, 85, 90, 100, 110, 120, 130, 140, 150, or 160 nm.

In some embodiments, in the pharmaceutical composition formed by the conjugate provided by the present disclosure and the above amine-containing transfection reagents, the weight ratio (weight/weight ratio) of the conjugate to total lipids, e.g., the organic amines, the helper lipids, and/or the PEGylated lipids, ranges from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12, or from about 1:6 to about 1:10. For example, the weight ratio of the conjugate provided by the present disclosure to total lipids is about 1:5, 1:6, 1:7, 1:8, 1:9, 1:10, 1:11, 1:12, 1:13, 1:14, 1:15, 1:16, 1:17, or 1:18.

In some embodiments, the pharmaceutical composition may be marketed with each component being separate, and used in the form of a liquid formulation. In some embodiments, the pharmaceutical composition formed by the conjugate provided by the present disclosure and the above pharmaceutically acceptable carrier may be prepared by various known processes, except for replacing the existing conjugate with the conjugate provided by the present disclosure. In some embodiments, the pharmaceutical composition may be prepared according to the following process:
The organic amines, helper lipids, and PEGylated lipids are suspended in alcohol at a molar ratio above and mixed homogeneously to yield a lipid solution; the alcohol is used in an amount such that the resultant lipid solution is present at a total mass concentration of 2 to 25 mg/mL (e.g., 8 to 18 mg/mL). The alcohol is a pharmaceutically acceptable alcohol, such as an alcohol that is in liquid form at about room temperature, for example, one or more of ethanol, propylene glycol, benzyl alcohol, glycerol, polyethylene glycol 200, polyethylene glycol 300, and polyethylene glycol 400, such as ethanol.

The conjugate provided by the present disclosure is dissolved in a buffered salt solution to produce an aqueous solution of the conjugate. The buffered salt solution has a concentration of 0.05 to 0.5 M, such as 0.1 to 0.2 M. The pH of the buffered salt solution is adjusted to 4.0 to 5.5, such as 5.0 to 5.2. The buffered salt solution is used in an amount such that the conjugate is present at a concentration of no more than 0.6 mg/mL, such as 0.2 to 0.4 mg/mL. The buffered salt may be one or more selected from the group consisting of soluble acetates and soluble citrates, such as sodium acetate and/or potassium acetate.

The lipid solution and the aqueous solution of the conjugate are mixed. The product obtained by mixing is incubated at a temperature of 40 to 60°C for at least 2 minutes (e.g., 5 to 30 minutes) to produce an incubated liposome formulation. The volume ratio of the lipid solution to the aqueous solution of the conjugate is 1:(2-5), such as 1:4.

The incubated liposome formulation is concentrated or diluted, subject to impurity removal, and then sterilized to afford the pharmaceutical composition of the present disclosure, which has the following physicochemical parameters: a pH of 6.5 to 8, an encapsulation efficiency of no less than 80%, a particle size of 40 to 200 nm, a polydispersity index of no more than 0.30, and an osmotic pressure of 250 to 400 mOsm/kg. For example, the physicochemical parameters may be as follows: a pH of 7.2 to 7.6, an encapsulation efficiency of no less than 90%, a particle size of 60 to 100 nm, a polydispersity index of no more than 0.20, and an osmotic pressure of 300 to 400 mOsm/kg.

Therein, the concentration or dilution step may be performed before, after, or simultaneously with removal of the impurities. The method for removing impurities may be any of various existing methods, for example, ultrafiltration using a tangential flow system and a hollow fiber column under 100 kDa, with a phosphate buffer (PBS) at pH 7.4 as ultrafiltration exchange solution. The method for sterilization may be any of various existing methods, such as filtration sterilization on a 0.22 µm filter.

### Use of the conjugate provided by the present disclosure

In another aspect, the present disclosure further provides the use of the conjugate and/or pharmaceutical composition of the present disclosure in the manufacture of a medicament for diagnosing tumors and tumor-related diseases or symptoms.

In another aspect, the present disclosure further provides a method for diagnosing tumors and tumor-related diseases or symptoms, comprising administering an effective amount of the conjugate and/or the pharmaceutical composition of the present disclosure to a subject in need thereof.

By administering the conjugate and/or the pharmaceutical composition of the present disclosure, the method of the present disclosure can effectively diagnose tumors and tumor-related diseases or symptoms; and with the highly specific targeting effect of the conjugate of the present disclosure, the distribution of the diagnostic agent and/or therapeutic agent in other undesired organs/tissues of the body can be decreased, thereby reducing potential side effects. In some embodiments, the medicament may be used for diagnosing tumor cells/tissues in a sample to be tested in an *in vitro* experiment. **In** some embodiments, the medicament may be used for diagnosing tumor cells/tissues in a subject. **In** some embodiments, the medicament may be used for treating tumors or tumor-related diseases and symptoms in a subject.

As used herein, the term "administration/administer" refers to placing the aptamer and/or conjugate and/or the pharmaceutical composition into a subject by a method or route where the aptamer and/or conjugate and/or the pharmaceutical composition is at least partly located at a desired site to achieve a desired effect. The administration routes suitable for the method of the present disclosure include topical administration and systemic administration. **In** general, topical administration results in the delivery of more of the aptamer and/or conjugate and/or the pharmaceutical composition to a particular site as compared with the whole body of the subject; whereas systemic administration results in the delivery of the aptamer and/or conjugate and/or the pharmaceutical composition to substantially the whole body of the subject.

Furthermore, the inventors of the present disclosure have unexpectedly found that the aptamer and/or conjugate and/or the pharmaceutical composition of the present disclosure can efficiently pass through the blood brain barrier and target tumors in the brain upon systemic administration, thereby further improving the delivery efficiency of the diagnostic agent groups, saving costs, and reducing undesired side effects.

The administration to a subject can be achieved by any suitable route known in the art, including but not limited to, oral or parenteral routes, such as intravenous administration, intramuscular administration, subcutaneous administration, transdermal administration, intratracheal administration (aerosol), pulmonary administration, nasal administration, rectal administration, and topical administration (including buccal administration and sublingual administration). The administration frequency may be once or more times daily, weekly, biweekly, triweekly, monthly, or yearly.

The dose of the aptamer and/or conjugate and/or the pharmaceutical composition of the present disclosure can be a conventional dose in the art, which may be determined according to various parameters, especially age, weight, and gender of a subject. Toxicity and efficacy may be determined in cell cultures or experimental animals by standard pharmaceutical procedures, for example, by determining LD50 (the lethal dose that causes 50% population death) and ED50 (the dose that can cause 50% of the maximum response intensity in a quantitative response, or that causes 50% of the experimental subjects to have a positive response in a qualitative response). The dose range for human use may be derived based on the data obtained from cell culture analysis and animal studies.

When the aptamer and/or the conjugate and/or the pharmaceutical composition of the present disclosure is administered, for example, to male or female C57BL/6J or C3H/HeNCrlVr mice with an age of 6 to 12 weeks and a body weight of 18 to 25 g (based on the amount of the aptamer and/or the conjugate and/or the conjugate in the pharmaceutical composition): for the conjugate formed by a conjugate and a pharmaceutically acceptable conjugation molecule, the dosage of the aptamer and/or conjugate may be 0.001 to 100 mg/kg body weight, in some embodiments 0.01 to 50 mg/kg body weight, in some further embodiments 0.05 to 20 mg/kg body weight, in some even further embodiments is 0.1 to 15 mg/kg body weight, and in some still further embodiments 0.1 to 10 mg/kg body weight. When the aptamer and/or the conjugate and/or the pharmaceutical composition of the present disclosure is administered, the above dosages may be preferred.

### Kit

The present disclosure provides a kit comprising the conjugate and/or the pharmaceutical composition provided by the present disclosure.

In some embodiments, the kit of the present disclosure may provide the aptamer and/or the conjugate and/or the pharmaceutical composition in a container. In some embodiments, the kit of the present disclosure may comprise a container for providing pharmaceutically acceptable excipients. In some embodiments, the kit may further comprise additional ingredients, such as stabilizers or preservatives. In some embodiments, the kit of the present disclosure may comprise at least one additional therapeutic agent in a container other than the container for providing the aptamer and/or the conjugate and/or the pharmaceutical composition of the present disclosure. In some embodiments, the kit may comprise an instruction for mixing the aptamer and/or the conjugate and/or the pharmaceutical composition with pharmaceutically acceptable carriers and/or adjuvants or other ingredients (if any).

In the kit of the present disclosure, the aptamer and/or the conjugate and the pharmaceutically acceptable carriers and/or adjuvants, as well as the pharmaceutical composition and/or the pharmaceutically acceptable adjuvants may be provided in any form, e.g., in a liquid form, a dry form, or a lyophilized form. In some embodiments, the aptamer and/or the conjugate and the pharmaceutically acceptable carriers and/or adjuvants as well as the pharmaceutical composition and the optional pharmaceutically acceptable adjuvants are substantially pure and/or sterile. In some embodiments, the kit of the present disclosure may provide sterile water.

Hereinafter, the present disclosure will be further illustrated with reference to the Examples, but is not limited thereto in any respect.

### Example

Unless otherwise specified, the reagents and culture media used in the following Examples are all commercially available, and the procedures used, such as nucleic acid electrophoresis and real-time PCR, are all performed according to the methods described in Molecular Cloning (Cold Spring Harbor LBboratory Press (1989)).

### Preparation Examples 1-9 and 12 Synthesis of the conjugates provided by the present disclosure

The conjugates numbered AP1-AP8 and AP12 in Table 1 were respectively synthesized by the solid phase synthesis methods, comprising sequentially linking all nucleoside monomers in the 3' to 5' direction according to the corresponding nucleotide sequences of AP1-AP8 and AP12 in Table 1 respectively, and then linking the Cy5 phosphoramidite monomer according to the manner of linking nucleoside phosphoramidite monomers in the solid phase synthesis method (pursued from Suzhou GenePharma Inc., Lot No. CY5P21H1B). Subsequently, the nucleotide sequence was added into the mixture solution of methylamine aqueous solution and aqueous ammonia in equal volumes (the amount of the solution relative to the conjugate was 0.5 ml/µmol), the mixture reacted at 25°C for 2h, the solid was removed by filtration, and the supernatant was concentrated in vacuum to dryness.

Purification of the prepared conjugates was achieved by using a preparative ion chromatography purification column (Source 15Q) with a gradient elution of NaCl aqueous solution. Specifically, eluant A was 20 mM sodium phosphate (pH 8.1), solvent was water/acetonitrile in 9:1 (v/v); eluant B was 1.5 M sodium chloride, 20 mM sodium phosphate (pH 8.1), solvent was water/acetonitrile in 9:1 (v/v); elution gradient: the ratio of eluant A: eluant B = 100:0-50:50. The eluate was collected, combined and desalted by using a reverse phase chromatography purification column. The specific condition comprises: using a Sephadex column for desalination (filler: Sephadex G25) and eluting with deionized water. The resultant eluate was concentrated to remove the solvent and lyophilized to respectively obtain conjugates AP1-AP8, in which the 5' terminal was linked to the fluorescent group Cy5. Therein, the fluorescent group Cy5 was linked to the 5' position of the ribose of the 5' terminal nucleotide in the consecutive nucleotide sequence by a phosphodiester bond.

Conjugate AP9 was prepared according to the same method, in which the 5' position of the ribose of the 5' terminal nucleotide was linked to the fluorescent group Cy5 by a phosphate ester linking group, with the only difference of sequentially linking nucleoside monomers according to the corresponding nucleic acid sequences of AP9 in Table 1, and using Cy3 phosphoramidite monomer (purchased from Shanghai Zhaowei Technology Development Co., Ltd., Cat No. OP-038) to replace Cy5 phosphoramidite monomer for preparation.

After completion of the synthesis of the aforementioned conjugates AP1-AP9 and AP12, the resultant conjugates were diluted to a concentration of 0.2 mg/mL by using ultra-pure water (homemade by Milli-Q ultra-pure water instrument, with resistivity of 18.2 MΩ* cm (25°C)). The molecular weight was determined by a LC-MS instrument (LC-MS, liquid chromatography-mass spectrometry, purchased from Waters Crop., model: LCT Premier). As a result, the measured value of the molecular weight was in conformity with the calculated value, indicating that the target conjugates had been obtained.

### Comparative preparation Examples 10, 11, 13 and 14 Synthesis of the comparative conjugates

The conjugates numbered comparative AP10, comparative AP11, comparative AP13 and comparative AP14 in Table 1 were respectively synthesized according to the method of the preparation Examples 1-8 and 12, with the only difference of sequentially linking nucleoside monomers respectively according to the corresponding sequences of comparative AP10, comparative AP11, comparative AP13 and comparative AP14 in Table 1. Therefore, the comparative conjugates comparative AP10, comparative AP11, comparative AP13 and comparative AP14 were respectively obtained, in which the 5' position of the ribose of the 5' terminal nucleotide was linked to the fluorescent group Cy5 by a phosphate ester linking group. Therein, comparative AP10 and comparative AP11 were respectively negative control conjugates with different sequences or modification schemes, and their aptamer sequences have certain randomness and almost have no sequence homology with the aptamers provided by the present disclosure. Comparative AP13 and comparative AP14 were respectively comparative conjugates whose sequences have only a few nucleotide differences from the sequences of conjugates AP2 and AP4. Specifically, as compared with AP2, comparative AP13 adds two nucleotides G and A at the 5' terminal, deletes one nucleotide C at the 3' terminal, and deletes one nucleotide U at the position corresponding to the 17th-18the nucleotide from the 5' terminal of AP2 within the strand; as compared with AP4, comparative AP14 adds two nucleotides G and A at the 5' terminal, deletes one nucleotide U at the 3' terminal, and deletes one nucleotide U at the position corresponding to the 17th-18th nucleotide from the 5' terminal of AP2 within the strand.

**Table 1 Nucleotide sequences of the conjugates**

| Preparation Example | Conjugate | Nucleotide sequence 5'-3' | SEQ ID NO: |
|---|---|---|---|
| Preparation Example 1 | AP1 | CY5-CfUfGGAGUfUfCfAGACfGUfGUfUfGCfUf CfUfUfCfCfGAUfCfUfCf | 15 |
| Preparation Example 2 | AP2 | CY5-CmUmGmGmAmGmUmUmCmAmGmAmC mGmUmGmUmUmGmCmUmCmUmUmCmCmG mAmUmCmUmCm | 21 |
| Preparation Example 3 | AP3 | CY5-CmsUmsGmGmAmGmUmUmCmAmGmAm CmGmUmGmUmUmGmCmUmCmUmUmCmCm GmAmUmCmsUmsCm | 34 |
| Preparation Example 4 | AP4 | CY5-CmUmGmGmAmGmUmUmCmAmGmAmC | 22 |
| | | mGmUmGmUmUmGmCmUmCmUmUm | |
| Preparation Example 5 | AP5 | CY5-CmUmGmGmAmGmUmUmCmAmGmAmC mGmUmUmGmUmGmCmUmCmUmUmCmCmG mAmUmCmUmCm | 23 |
| Preparation Example 6 | AP6 | CY5-CmUmGmGmAmGmUmUmCmAmGmAmC mGmUmUmGmUmGmCmUmCmUmUm | 24 |
| Preparation Example 7 | AP7 | CY5-CmUmGmGmAmGmUmUmCmAmGmAmC mCmGmUmGmUmGmCmUmCmUmUmCmCmG mAmUmCmUmCm | 25 |
| Preparation Example 8 | AP8 | CY5-CmUmGmGmAmGmUmUmCmAmGmAmC mCmGmUmGmUmGmCmUmCmUmUm | 26 |
| Preparation Example 9 | AP9 | CY3-CmUmGmGmAmGmUmUmCmAmGmAmC mGmUmGmUmUmGmCmUmCmUmUmCmCmG mAmUmCmUmCm | 21 |
| Comparative preparation Example 10 | Comparative AP10 | CY5-CfCfAGAGUfCfGCfUfGAGAUfUfCfGUfGG CfGCfUfAUfAUf | 46 |
| Comparative preparation Example 11 | Comparative AP11 | CY5-UfCfUfAUfGGCfUfGCfCfGAUfCfUfGGUfC fUfCfCfAUfGUfACfGUf | 47 |
| Preparation Example 12 | AP12 | CY5-UmCmUmAmUmGmGmCmUmGmCmCmG mAmUmCmUmGmGmUmCmUmCmCmAmUmG mUmAmCmGmUm | 33 |
| Comparative preparation Example 13 | Comparative AP13 | CY5-GmAmCmUmGmGmAmGmUmUmCmAmG mAmCmGmUmGmUmGmCmUmCmUmUmCmC mGmAmUmCmUm | 48 |
| Comparative preparation Example 14 | Comparative AP14 | CY5-GmAmCmUmGmGmAmGmUmUmCmAmG mAmCmGmUmGmUmGmCmUmCmUm | 50 |

In Table 1, C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by phosphorothioate linkage; CY3 and CY5 respectively represent the attachment positions of fluorescent dye group Cy3 (Cyanine 3) and Cy5 (Cyanine 5) on the nucleotide sequence.

### Preparation Example 21-26 Synthesis of conjugates AP21-AP26 provided by the present disclosure

The conjugates numbered AP21-AP26 in Table 2 were synthesized according to the method of preparation Examples 1-8 and 12, and the synthesized conjugates were confirmed by determining molecular weights thereof, with the only difference of sequentially linking nucleoside monomers according to the corresponding sequences of AP21-AP26 in Table 2. Therefore, conjugates AP21-AP26 with fluorescent group Cy5 linked at the 5' position of the ribose of the 5' terminal nucleotide by a phosphate ester linking group were obtained respectively. Therein, as compared with AP4 above, AP21 deletes one nucleotide at the 3' terminal; as compared with AP4 above, AP22 has the structure in which the motifs S₁ and S₄ each delete one nucleotide corresponding to Formula (1); AP23 has the structure of AP22 in which the motif S₁ deletes one more nucleotide corresponding to Formula (1); AP24 is a conjugate obtained by changing the first two nucleotides at the 5' terminal of AP4; as compared with AP4 above, AP25 and AP26 are the conjugates obtained by changing N_{b} motif corresponding to Formula (1).

After completion of the synthesis of the aforementioned conjugates AP11-AP26, the resultant conjugates were diluted to a concentration of 0.2 mg/mL by using ultra-pure water (homemade by Milli-Q ultra-pure water instrument, with resistivity of 18.2 MΩ* cm (25°C)). The molecular weight was determined by a LC-MS instrument (LC-MS, liquid chromatography-mass spectrometry, purchased from Waters Crop., model: LCT Premier). As a result, the measured value of the molecular weight was in conformity with the calculated value, indicating that the target conjugates had been obtained.

The MS calculated values and the MS measured values of conjugates AP11-AP26 are shown in Table 2-a below:

**Table 2-a The results of molecular weight determination of the conjugates**

| Number | Calculated MS value | Measured MS value |
|---|---|---|
| AP21 | 8173.61 | 8171.39 |
| AP22 | 7814.38 | 7812.23 |
| AP23 | 7455.14 | 7452.96 |
| AP24 | 8516.85 | 8514.56 |
| AP25 | 8414.74 | Molecular ion peak: 2101.9(4-valence); 1681.4(5-valence) |
| AP26 | 8471.75 | 8469.53 |

### Comparative preparation Examples 27-35 Synthesis of control conjugates comparative AP27 - comparative AP35

The conjugates numbered comparative AP27- comparative AP35 in Table 2 were synthesized according to the method of preparation Examples 1-8 and 12, and confirmed by determining molecular weight thereof, with the only difference of sequentially linking nucleoside monomers according to the corresponding sequences of comparative AP27- comparative AP35 in Table 2. Therefore, comparative AP27-comparative AP35 with fluorescent group Cy5 linked at the 5' position of the ribose of the 5' terminal nucleotide by a phosphate ester linking group were obtained respectively. Therein, comparative conjugates comparative AP27 and comparative AP28 were control conjugates in which the base composition of the sequences was the same as that of AP4, but the arrangement order of nucleotides was different; conjugates comparative AP29 and comparative AP30 were control conjugates in which the base composition of the sequences was the same as that of AP2, but the arrangement order of nucleotides was different; as compared with comparative AP4, comparative AP31 deletes two nucleotides at the 5' terminal, and deletes one nucleotide at the 3' terminal; as compared with comparative AP4, AP32 deletes N_{b} motif corresponding to Formula (1); as compared with AP4, AP33 deletes Nₐ and N_{c} motifs corresponding to Formula (1); as compared with comparative AP35, comparative AP34 was a control conjugate which respectively increases multiple nucleotides and changes partial sequence in S2 and S3 motifs.

In each of the conjugates above, the diagnostic agent group Cy3 or Cy5 was linked to the 5' position of the ribose of the 5' terminal nucleotide of the nucleotide sequence.

**Table 2 Nucleotide sequences of conjugates**

| Preparation Example | Conjugate | Nucleotide sequence 5'-3' | SEQ ID NO: |
|---|---|---|---|
| Preparation Example 21 | AP21 | CY5-CmUmGmGmAmGmUmUmCmAmGmAmCmG mUmGmUmUmGmCmUmCmUm | 27 |
| Preparation Example 22 | AP22 | CY5-CmUmGmAmGmUmUmCmAmGmAmCmGmU mGmUmUmGmCmUmCmUm | 28 |
| Preparation Example 23 | AP23 | CY5-CmUmGmCmUmUmCmAmGmAmCmGmUmG mUmUmAmGmCmUmUm | 29 |
| Preparation Example 24 | AP24 | CY5-AmCmGmGmAmGmUmUmCmAmGmAmCmG mUmGmUmUmGmCmUmCmUmUm | 30 |
| Preparation Example 25 | AP25 | CY5-CmUmGmGmAmGmUmUmCmAmCmUmAmC mUmGmUmUmGmCmUmCmUmUm | 31 |
| Preparation Example 26 | AP26 | CY5-CmUmGmGmAmGmUmUmCmAmGmUmUmG mUmGmUmUmGmCmUmCmUmUm | 32 |
| Comparative preparation Example 27 | Comparative AP27 | CY5-GmGmAmCmAmUmGmGmAmUmUmCmUmU mGmUmCmUmGmUmGmUmCmCm | 44 |
| Comparative preparation Example 28 | Comparative AP28 | CY5-GmAmUmCmUmGmUmGmUmCmUmUmAmG mAmCmGmUmGmUmUmGmCmCm | 45 |
| Comparative preparation Example 29 | Comparative AP29 | CY5-GmAmUmCmGmUmGmAmCmGmAmCmUmC mUmUmGmUmCmUmGmCmUmGmUmCmCmUmG mAmUmCm | 51 |
| Comparative preparation Example 30 | Comparative AP30 | CY5-CmGmUmCmUmCmCmAmGmCmAmUmGmU mGmUmAmGmGmUmCmUmCmUmGmUmCmUmC mUmGmAm | 52 |
| Comparative preparation Example 31 | Comparative AP31 | CY5-GmGmAmGmUmUmCmAmGmAmCmGmUmG mUmUmGmCmUmCmUm | 53 |
| Comparative preparation Example 32 | Comparative AP32 | CY5-CmUmGmGmAmGmUmUmCmGmUmUmGmC mUmCmUmUmCmCmGmAmUmCmUmCm | 54 |
| Comparative preparation Example 33 | Comparative AP33 | CY5-CmUmGmGmAmCmAmGmAmCmGmUmGmU mCmUmUmCmCmGmAmUmCmUmCm | 55 |
| Comparative preparation Example 34 | Comparative AP34 | CY5-CmUmGmGmAmGmUmUmCmAmGmAmUmC mAmGmAmCmGmUmGmUmGmUmGmUmUmGmC mUmCmUm | 56 |
| Comparative preparation Example 35 | Comparative AP35 | CY5-CmUmGmGmAmGmUmUmCmAmGmAmCmG mUmGmUmAmGmCmUmCmGmUmGmUmGmAmU mCmUmCm | 57 |

In Table 2, C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; CY5 represent the attachment position of fluorescent dye group Cy5 (Cyanine 5) group on the nucleotide sequence.

After completion of the synthesis of the aforementioned comparative conjugates comparative AP27- comparative AP35, the resultant conjugates were diluted to a concentration of 0.2 mg/mL by using ultra-pure water (homemade by Milli-Q ultra-pure water instrument, with resistivity of 18.2 MΩ* cm (25°C)). The molecular weight was determined by a LC-MS instrument (LC-MS, liquid chromatography-mass spectrometry, purchased from Waters Crop., model: LCT Premier). As a result, the measured value of the molecular weight was in conformity with the calculated value, indicating that the target conjugates hd been obtained.

The MS calculated values and the MS measured values of comparative conjugates comparative AP27- comparative AP35 are shown in Table 2-b below:

**Table 2-b: The results of molecular weight determination of the conjugates**

| Number | Calculated MS value | Measured MS value |
|---|---|---|
| comparative AP27 | 8493.81 | 8491.53 |
| comparative AP28 | 8493.81 | 8491.58 |
| comparative AP29 | 11113.50 | 11111.00 |
| comparative AP30 | 11113.50 | 11111.02 |
| comparative AP31 | 7534.23 | 7532.11 |
| comparative AP32 | 9069.14 | 9067.04 |
| comparative AP33 | 8795.08 | 8793.02 |
| comparative AP34 | 11217.59 | 11215.28 |
| comparative AP35 | 11216.60 | 11214.42 |

### Experimental Example 1 Evaluation and comparison of the ability of the conjugates provided by the present disclosure and control conjugates to enter into U118-MG glioma cells in vitro

In this experimental Example, the ability of conjugate AP1 and control conjugate comparative AP10 to enter into U118-MG glioma cells *in vitro* of was evaluated and compared.

U118MG neuroglioma cells (purchased from the Shanghai Cell Bank of Chinese Academy of Sciences) were cultured in DMEM medium (Thermo Fisher) supplemented with 10% fetal bovine serum (FBS, Thermo Fisher) at 37°C in an incubator containing 5% CO₂/95% air.

U118MG cells were seeded into a 96-well plate at 5 × 10⁵ cells/well. Conjugate AP1 prepared above and yeast tRNA were respectively formulated into conjugates solution 1A, 1B, 1C, 1D, 1E, 1F, 1G, 1H, and 1I with DMEM medium, and these conjugate solutions 1A-1I comprised yeast tRNA at a final concentration of 100 µg/mL and respectively comprised conjugate AP1 at final concentrations of 5 nM, 10 nM, 20 nM, 40 nM, 60 nM, 80 nM, 100 nM, 150 nM, 200 nM, and 500 nM.

Control conjugate comparative AP10 prepared above and yeast tRNA were respectively formulated into conjugates solution 1A', 1B', 1C', 1D', 1E', 1F', 1G', 1H', and 1I' with DMEM medium, and these conjugate solutions 1A'-1I' comprised yeast tRNA at a final concentration of 100 µg/mL and respectively comprised control conjugate comparative AP10 at final concentrations of 5 nM, 10 nM, 20 nM, 40 nM, 60 nM, 80 nM, 100 nM, 150 nM, 200 nM, and 500 nM.

The aforementioned culture wells containing U118MG cells were respectively added with the conjugate solutions 1A-1I above in an amount of 200 µL/well and designated as test group 1A- test group 1I. The aforementioned culture wells containing U118MG cells were respectively added with the conjugate solution 1A'-1I' above in an amount of 200 µL/well and designated as control group 1A'- control group 1I'. Another culture well containing U118MG cells was added with 200 µL DMEM medium containing yeast tRNA at a final concentration of 100 µg/mL and designated as blank control group 1J. The 96-well plate containing the above test groups and the blank control group were incubated at 37°C for 1h in the dark; after incubation, the supernatant of the reaction mixture of each test group and the blank control group was aspirated, and DPBS buffer (purchased from Thermo Fisher) was added to wash the plate 3 times (100 µL each time). After the washing solution was aspirated, each culture well was added with 100 µL DMEM medium to obtain a cell suspension. The supernatant was removed by centrifugation and then 500 µL DPBS buffer was added to re-suspend the cells. Flow cytometry analysis was performed with flow cytometer (BD Biosciences): 10,000 cells in each test group or the blank control group were analyzed, the fluorescence intensity distribution was determined and the mean fluorescence intensity (MFI, a.u.) was calculated. Furthermore, the mean fluorescence intensity (MFI, a.u.) corresponding to each final concentration of conjugates is listed in Table 3 below for comparison, and the relative fluorescence difference R is calculated according to the following equation: R = (MFI_{AP1}- MFI_{CAP10}) / MFI_{CAP10}.

Therein, MFI_{AP1} represents mean fluorescence intensity of conjugate AP1 in U118-MG glioma cells, and MFI_{CAP10} represents mean fluorescence intensity of comparative AP10 in U118-MG glioma cells. The R value reflects the relative ability of conjugate AP1 to enter into U118-MG glioma cells: when R>2, it indicates that the corresponding conjugate has a very stronger ability to enter into cells (*see* Table 2 of Engineering of Targeted Nanoparticles for Cancer Therapy Using Internalizing Aptamers Isolated by Cell-Uptake Selection. ACS Nano. 2012 January 24; 6(1), which are incorporated herein by reference incorporated herein).

**Table 3 The mean fluorescence intensity and R value of conjugates**

| Concentration of aptamers (nM) | MFI_{CAP10} (a.u.) | MFI_{AP1} (a.u.) | R |
|---|---|---|---|
| 5 nM | 25.4 | 263.4 | 9.37 |
| 10 nM | 67.4 | 503.4 | 6.47 |
| 20 nM | 176.4 | 1089.4 | 5.18 |
| 40 nM | 443.4 | 1440.4 | 2.25 |
| 60 nM | 666.4 | 2355.4 | 2.53 |
| 80 nM | 787.4 | 2921.4 | 2.71 |
| 100 nM | 1167.4 | 3360.4 | 1.88 |
| 150 nM | 1251.4 | 3969.4 | 2.17 |
| 200 nM | 1868.4 | 7895.4 | 3.23 |
| 500 nM | 4029.4 | 15770.4 | 2.91 |

As can be seen from Table 3, at different concentrations, conjugate AP1 provided by the present disclosure showed significantly higher mean fluorescence intensity in U118MG glioma cells as compared with comparative AP10 with a random sequence, indicating that the conjugate provided by the present disclosure has excellent ability to enter into U118MG glioma cells.

### Experimental Example 2 The entry of conjugates into different cells observed by a fluorescence imaging system

In this experiment, the entry of conjugate AP1 provided by the present disclosure and comparative conjugate comparative AP10 into different cells was observed by a PerkinElmer High Content Imaging system (Operatta).

U118MG glioma cells, SVGp12 normal astrocytes, T98G human brain glioma cells, U251 human glioma cells, A549 human non-small cell lung cancer cells, MCF-7 human breast cancer cells, and 293T human kidney epithelial cells were all purchased from the Shanghai Cell Bank of Chinese Academy of Sciences. Before the experiment, for each cell line, the cells in the logarithmic growth phase were selected and seeded into a 96-well plate at 5000 cells/well (each cell line was seeded into 2 culture wells), and cultured at 37°C in an incubator containing 5% CO₂/95% air for 24h. The supernatant was aspirated and DPBS buffer (purchased from Thermo Fisher) was added to wash the plate 2 times (100 µL each time).

Conjugate AP1 prepared above and yeast tRNA were respectively formulated into aptamer solution 2A with DMEM medium, and the conjugate solution 2A comprised yeast tRNA at a final concentration of 100 µg/mL and conjugate AP1 at a final concentration of 100 nM.

Control conjugate comparative AP10 prepared above and yeast tRNA were respectively formulated into control conjugate solution 2B with DMEM medium, and control conjugate solution 2B comprised yeast tRNA at a final concentration of 100 µg/mL and comparative AP10 at a final concentration of 100 nM.

For each cell line, another culture well was added with 200 µL conjugate solution 2A and incubated at 37°C for 30 min in the dark, then the supernatant was aspirated and DPBS buffer was added to wash the well 2 times (100 µL each time) to obtain test group 2X.

For each cell line, another culture well was added with 200 µL control conjugate solution 2B and incubated at 37°C for 30 min in the dark, then the supernatant was aspirated and DPBS buffer was added to wash the well 2 times (100 µL each time) to obtain control group 2Y.

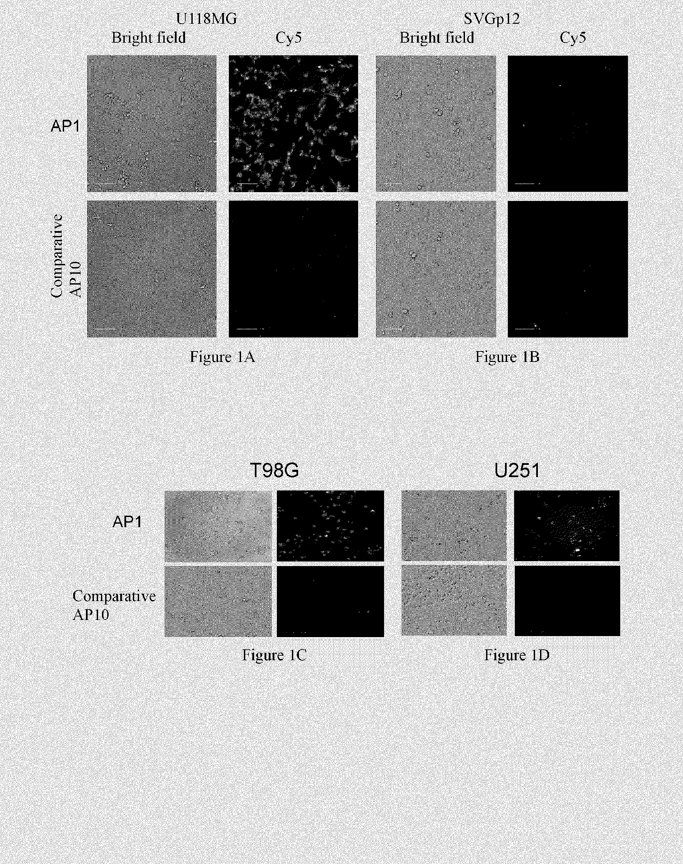

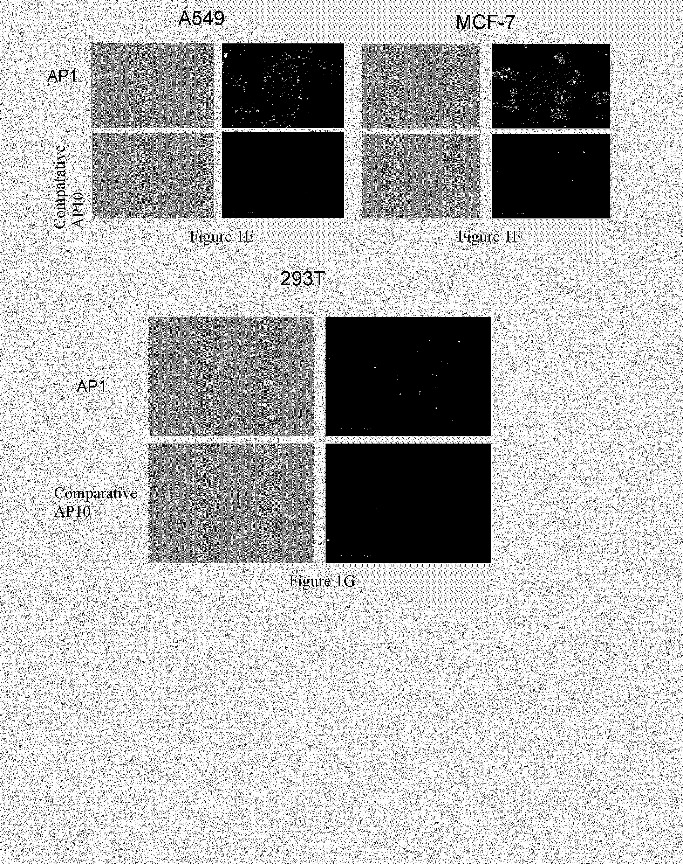

Test group 2X and control group 2Y of the above cell lines were respectively added with 100 µL DMEM medium at 37°C, and imaged in a high content imaging system. Normalization was performed according to the mean fluorescence intensity of control group 2Y, that is, subtracting the mean fluorescence intensity of control group 2Y from the fluorescence intensity of test group 2X before performing imaging. The results are shown in Figure 1A- Figure 1G.

Figures 1A and 1B are respectively the high content imaging images showing the entry of AP1 and comparative AP10 into U118MG glioma cells and SVGp12 normal astrocytes. As can be seen from Figures 1A and 1B, only the U118MG cells treated with AP1 showed strong Cy5 fluorescence signal, while the SVGp12 cells did not show any fluorescence signal in both the test group and the control group, indicating that the conjugate provided by the present disclosure has a very strong ability to enter into U118MG glioma cells as compared with comparative AP10, and basically did not enter into SVGp12 normal astrocytes. The results above also indicate that the conjugate provided by the invention can selectively target and deliver the diagnostic agent groups to U118MG glioma cells.

Furthermore, Figures 1C-1G are respectively the high content imaging images showing the entry of AP1 and comparative AP10 into U251 human glioma cells, A549 human non-small cell lung cancer cells, MCF-7 human breast cancer cells, and 293T human renal epithelial cells. As can be seen from Figures 1C-1G, the U251 human glioma cells, A549 human non-small cell lung cancer cells and MCF-7 human breast cancer cells treated with AP showed a strong Cy5 fluorescence signal, while neither the tumor cells treated with comparative AP10 nor the 293T human renal epithelial cells showed any fluorescence signal in both the test group and the control group, indicating that the conjugate provided by the present disclosure has a very strong ability to enter into various tumor cells as compared with comparative AP10, and basically did not enter into normal cells. The conjugate provided by the invention can selectively target and deliver the diagnostic agent groups to various tumor cells.

### Experimental Example 3 The entry of conjugates into tumor spheres observed by a fluorescence imaging system

In this experiment, the entry of conjugate AP1 provided by the present disclosure and comparative conjugate comparative AP10 into U118MG glioma tumor spheres and A549 human non-small cell lung cancer tumor spheres was observed by a PerkinElmer High Content Imaging system (Operatta).

80 µL fresh, sterilized and unsolidified 2% agarose was added into the culture wells of a 96-well plate. After complete solidification, for each cell line, the cells in the logarithmic growth phase were selected and seeded into a 96-well plate at 2000 cells/well (each cell line was seeded into 2 culture wells, 200 µL cell suspension/well), cultured in DMEM medium supplemented with 10% FBS and 1% penicillin-streptomycin, and cultured at 37°C in an incubator containing 5% CO₂/95% air. Half of the supernatant was aspirated every 48h and fresh DMEM medium was added along the wall of the culture wells. After 7 days of culture, the cells were observed under the microsope to confirm that complete cell spheres were obtained, indicating successful tumor sphere cultivation.

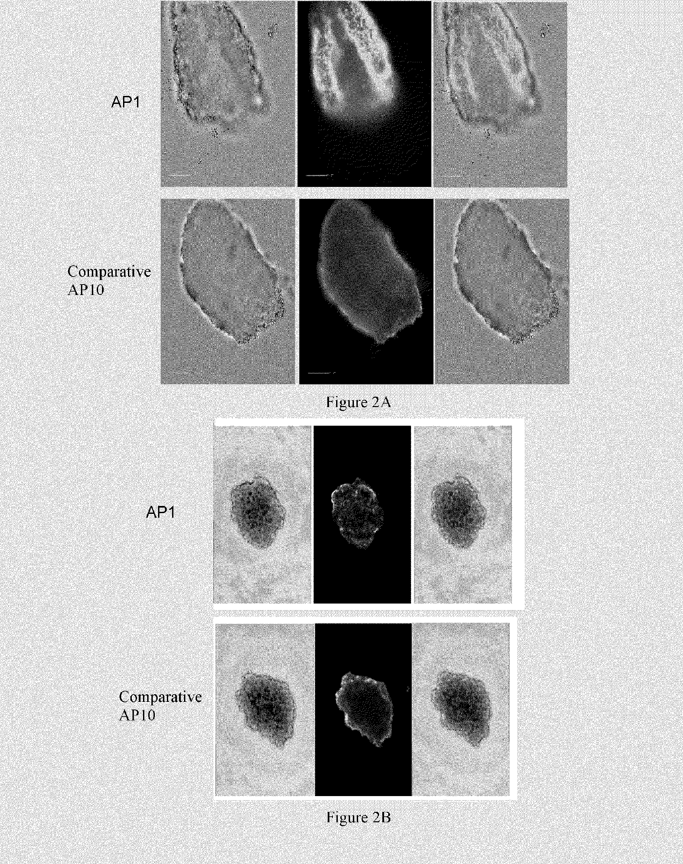

Each culture well was added with AP1 or comparative AP10 according to the method of experimental Example 2, and imaged in a high content imaging system, with the only difference of directly performing imaging after culture without washing and performing imaging according to the measured fluorescence intensity of Cy5. The results are shown in Figures 2A and 2B.

Figures 2A and 2B are respectively the high content imaging images showing the entry of AP1 and comparative AP10 into U118MG glioma tumor spheres and A549 human non-small cell lung cancer tumor spheres. As can be seen from Figures 2A and 2B, the interiors of AP1-treated U118MG glioma tumor spheres and A549 tumor spheres showed strong Cy5 fluorescence signal, while only the edge of the comparative AP10-treated tumor spheres showed significantly weak fluorescence signal, indicating that the conjugate provided by the present disclosure has a strong ability to enter into the interior of U118MG and A549 tumor spheres as compared with comparative AP10. The results above indicate that the conjugate provided by the invention can effectively deliver the diagnostic agent groups to the interior of tumors and have high drugability of diagnostic agent drug.

### Experimental Example 4 In vivo distribution of conjugates in U118MG cell subcutaneous tumor model mice

U118MG human glioma cells (purchased from Guangzhou Jennio Biotech Co., Ltd) were cultured in DMEM complete medium (MACGENE, Cat No. CM15019) supplemented with 10% fetal bovine serum (FBS, RMBIO) at 37°C in an incubator containing 5% CO₂/95% air.

U118MG human glioma cells in the logarithmic growth phase were selected and digested (0.25% pancreatic enzyme). The cells were collected, centrifuged to remove the supernatant, and then resuspended in DMEM medium supplemented with 10% FBS to be formulated into a cell culture solution with a concentration of 1×10⁸ cells/mL.

Experimental animals were 10 NOD-SCID mice (male, 12 weeks old, purchased from SPF (Beijing) Biotechnology Co., LTD.). The cell culture solution above was inoculated at the subcutaneous site of the right forelimb of a NOD-SCID mouse with an inoculation volume of 100 µL per mouse, i.e., each mouse was inoculated with 1×10⁷ cells. The mice were further fed for 20 days after injection.

AP1, AP2, and comparative AP11 were dissolved into solutions with a concentration of 0.3 mg/mL (based on aptamer) with 1×DMEM medium (purchased from M&C gene technology (BEIJING) LTD., Lot No. K1902200).

The mice inoculated with U118MG above were randomly divided into 3 groups (4 mice per group). For the 4 mice per group, the administration volume was calculated according to 10 µL/g body weight, and AP1, AP2 and comparative AP11 were respectively administered to different groups of mice by tail vein injection and designated as test group 4A1, test group 4A2 and control group 4A3. Thus, the administration dosage of each mouse was 3 mg/kg (based on aptamer). Another mouse inoculated with U118MG above was administered with 1×DMEM medium by tail vein injection (the administration volume was 10 µL/g body weight), and designated as blank control group 4Y.

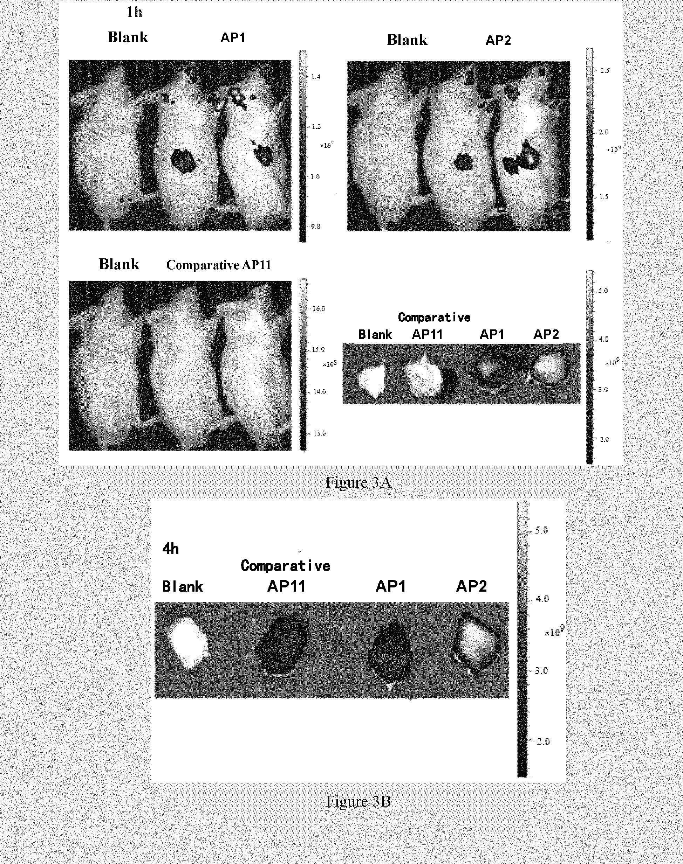

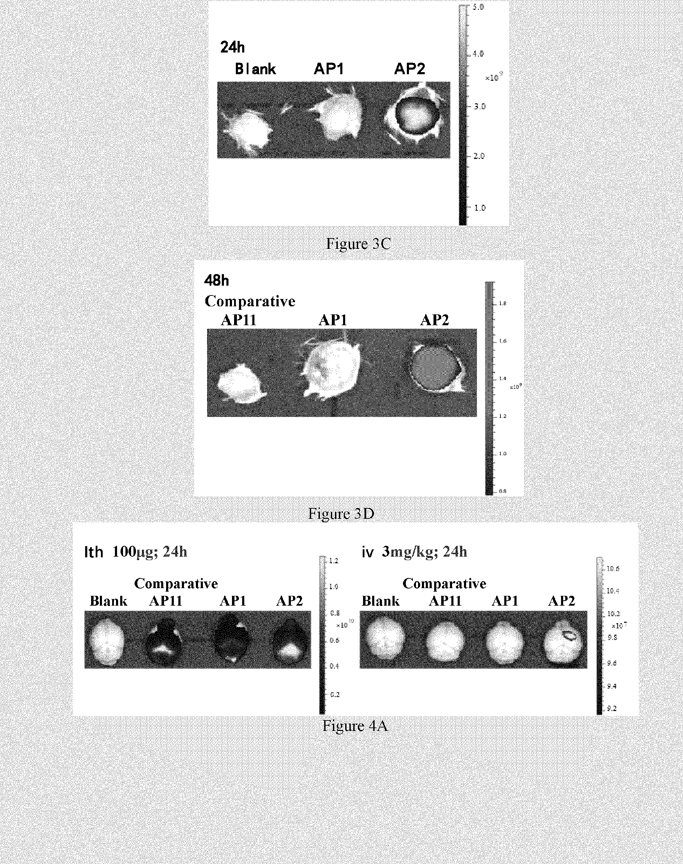

At 1h after administration, the mice of each group were placed in a small animal *in vivo* optical imaging system IVIS Lumina Series III (PerkinElmer). The mice were anesthetized with isoflurane gas, and the anesthetized mice were placed on their back in the small animal *in vivo* optical imaging system for *in vivo* imaging. At 1h, 4h, 24h and 48h after administration, one mouse of each group was respectively euthanized, and tumor tissues were taken for fluorescence imaging (no blank control group in the 24h and 48h results). The results are respectively shown in Figures 3A, 3B, 3C and 3D.

Figures 3A-3D are respectively images showing the *in vivo* imaging and tumor tissue imaging at 1h, 4h, 24h and 48h after administration, wherein blank represents blank control group 4Y. As can be seen from Figures 3A-3D, blank control group 4Y and control group 4A3 administered with comparative AP11 of a random sequence showed substantially no or weak fluorescence signal in mice. In contrast, conjugates AP1 and AP2 provided by the present disclosure showed strong fluorescence signal at the sites of tumor inoculation in mice, indicating that the aptamer provided by the present disclosure can specifically target and deliver the fluorescent group to U118MG glioma. Furthermore, conjugate AP2 still showed strong fluorescence signal at 24h to 48h after administration, indicating that the conjugates provided by the present disclosure could stably target glioma tissue over a long period of time.

### Experimental Example 5 In vivo distribution of conjugates in U118MG cell tumor in situ model mice

U118MG human glioma cells in the logarithmic growth phase cultured as descruved in experimental Example 4 were selected and digested with 0.25% pancreatic enzyme. The cells were collected, the supernatant was aspirated, and then the cells were resuspended in DMEM medium supplemented with 10% FBS to be formulated into a cell culture solution with a cell density of 4×10⁷ cells/mL.

Experimental animals were 16 NOD-SCID mice (male, 12 weeks old, purchased from SPF (Beijing) Biotechnology Co., LTD.). The cell culture solution above was inoculated into a NOD-SCID mouse: the cell culture solution was injected into the right striatum of a mouse by the manner of lateral ventricle injection in the mouse, and the position was AP (anteroposterior): 1 mm, ML (medial lateral): 1.5 mm, DV (dorsal ventral): 3.5 mm, the injection volume was 10 µL, i.e., each mouse was inoculated with 4×10⁵ cells. The mice were further fed for 14 days after injection.

AP1, AP2 and comparative AP11 were respectively dissolved into conjugate solutions with a concentration of 2 mg/mL (based on aptamer) with 1×DMEM medium (purchased from M&C gene technology (BEIJING) LTD., Lot No. K1902200). 10 mice above were taken and respectively injected with 50 µL AP1 and AP2 solution by the administration manner of intrathecal injection, with an administration dosage of 100 µg per animal (based on the amount of aptamer). 3 mice per group were administered and respectively designated as test groups 5A and 5B; each of 2 mice was respectively injected with 50 µL comparative AP11 solution with an administration dosage of 100 µg per animal (based on the amount of aptamer) and designated as control group 5C; each of 2 mice was respectively injected with 50 µL DMEM medium and designated as blank control group 5Y.

AP1, AP2 and comparative AP11 were respectively dissolved into conjugate solutions with a concentration of 0.3 mg/mL (based on aptamer) with 1×DMEM medium. The other 6 mice were taken and respectively injected with AP1, AP2 and comparative AP11 solutions by tail vein injection. The administration dosage for all animals was calculated according to body weight, the administration volume was all 10 µL/g , and the administration dosage was 3 mg/kg per animal (based on the amount of aptamer). 2 mice of each group were administered and respectively designated as test groups 5D, 5E and control group 5F.

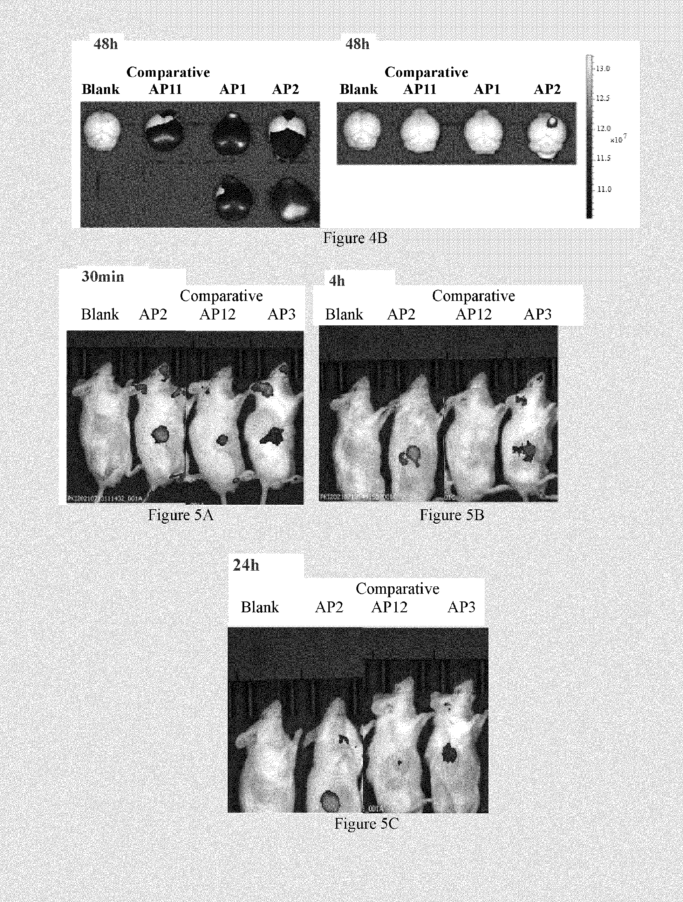

At 24h after administration, one mouse in each group was respectively euthanized and brain tissue was taken, and the remaining mice were euthanized at 48h after administration and brain tissues were taken. The brain tissues of mice were taken for fluorescence imaging in IVIS Lumina Series III. The results are respectively shown in Figures 4A and 4B.

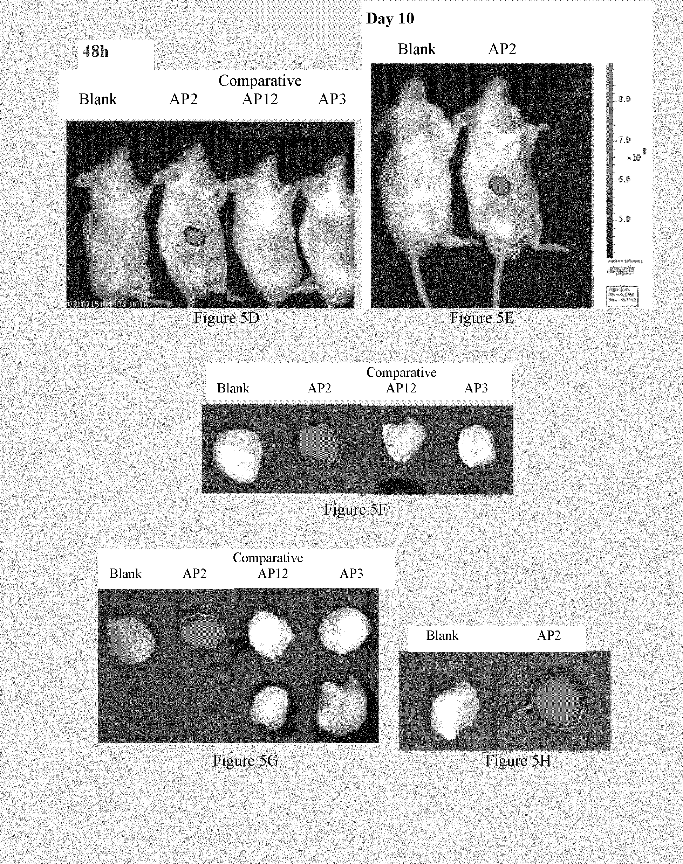

Figures 5A and 5B are respectively images showing the fluorescence imaging of the brain tissues of the U118MG tumor in situ model mice established after administration of blank control group 5Y, test groups 5A, 5B, 5D and 5E, and control groups 5C and 5F at 24h and 48h after administration. As can be seen from the results in Figures 4A and 4B, the blank control group and the control group of comparative AP11 shows no obvious fluorescence signal at the tumor site, indicating that they do not have significant targeting effect on in situ brain glioma. In contrast, test groups 5A and 5B administered with the conjugates provided by the present disclosure show strong fluorescence signals at the site of tumor inoculation, indicating that the conjugates could effectively target tumor tissues and that the aptamer provided by the present disclosure can effectively deliver different diagnostic agent groups (e.g., fluorescent groups) to tumor tissues. Furthermore, test group 5E shows obvious fluorescence signals at both 24 and 48h, indicating that conjugate AP2 can still reach and target brain glioma when administered via tail vein, and the conjugates provided by the present disclosure can also penetrate the Blood-Brain Barrier (BBB) and enter into brain glioma.

### Experimental Example 6 In vivo distribution of conjugates with different modifications in U118MG subcutaneous tumor model mice

U118MG subcutaneous tumor model mice were prepared according to the method described in experimental Example 4.

AP2, AP3 and comparative AP12 were respectively dissolved into conjugate soutions with a concentration of 0.3 mg/mL (based on aptamer) with 1 ×DMEM medium. 9 mice were divided into 3 groups (3 mice per group). AP2, AP3 and comparative AP12 solutions were respectively administered to each mouse of each group by tail vein injection. The administration dosage for all animals was calculated according to body weight, the administration volume was all 10 µL/g, and the administration dosage was 3 mg/kg per animal (based on the amount of aptamer). The three groups of mice were respectively designated as test group 6A, test group 6B and test group 6C. The other 2 mice were administered with DMEM medium with an administration volume of 10 µL/g and designated as blank control group 6Y. One mouse of each group was placed in a small animal *in vivo* optical imaging system IVIS Lumina Series III (PerkinElmer) at 30 min, 1h, 24h and 48h after administration, respectively. The mice were anesthetized by isoflurane gas, and the anesthetized mice were placed on their back in the small animal *in vivo* optical imaging system for *in vivo* imaging. The results are respectively shown in Figures 5A-5D. On Day 10 after administration, one mouse of blank control group 6Y and one mouse of test group 6A were taken for *in vivo* imaging, and the results are shown in Figure 5E; at 24h and 48h after administration, one mouse of each group was respectively euthanized and tumor tissues were taken for fluorescence imaging, and the results are respectively shown in Figures 5F and 5G. On Day 10 after administration, one mouse of blank control group 6Y and one mouse of test group 6A was respectively euthanized and tumor tissues were taken for fluorescence imaging, and the results are shown in Figure 5H. In Figures 5A-5H, Blank represents blank control group 6Y.

Figures 5A-5E are respectively images showing the *in vivo* fluorescence imaging of mice at different time points after administration of the conjugates of different sequences provided by the present disclosure and comparative conjugates; Figures 5F-5H are respectively images showing the tumor tissue imaging at different time points. The results of Figures 5A-5H indicate that fluorescence signals are visible in subcutaneous tumors of all test groups and control group at 30 min after administration. At 4h to 24h after administration, mice in test groups 6A, 6B and 6C administered with AP2, AP3 and AP12 provided by the present disclosure still show high fluorescence intensity; at 48h to Day 10 after administration, mice in test group 6A administered with AP2 still show strong fluorescent signal. These results indicate that the conjugates provided by the present disclosure can stably target tumor tissues over a longer period of time as compared with control conjugates, and the conjugate provided by the present disclosure can stably target and deliver diagnostic agent groups to tumors over a long period of time.

### Experimental Example 7 In vivo distribution of conjugates in A549 subcutaneous tumor model mice

A549 human non-small cell lung cancer cell subcutaneous tumor model mice were prepared according to the method described in experimental Example 4, with the only difference that the cell culture solution was prepared using A549 human non-small cell lung cancer cells instead of U118MG glioma cells.

11 NOD-SCID mice ( male, 6-8 weeks old, purchased from SPF (Beijing) Biotechnology Co., LTD.) were selected and divided randomly: 2 mice in the blank control group and 3 mice in each of the other groups. The culture medium containing A549 tumor cells above was inoculated at the subcutaneous site of the right forelimb of a mouse, and each mouse was inoculated 100 µL A549 tumor cells; thus, through calculation, the number of inoculated cells was 1×10⁷ cells per mouse.

AP2, AP3 and comparative AP12 were respectively dissolved into conjugate solutions with a concentration of 0.3 mg/mL (based on aptamer) with 1×DMEM medium (purchased from M&C gene technology (BEIJING) LTD., Lot No. K1902200). On Day 21 after inoculation of A549 tumor cells, each mouse was respectively adminstered with 1×DMEM and AP2, AP3, and AP12 conjugate solutions formulated above by tail vein injection, and the mice injected with 1×DMEM, AP2, AP3, and AP12 conjugate solutions were respectively designated as blank control group, AP2 group, AP3 group, and AP12 group. The administration dosage for all animals was calculated according to body weight, and the administration volume was 10 µL/g per mouse. That is, except the blank control group, the administration dosage of each mouse in the other groups was 3 mg/kg (based on the amount of aptamer); the blank control group was administered with only 10 µL/g DMEM medium.

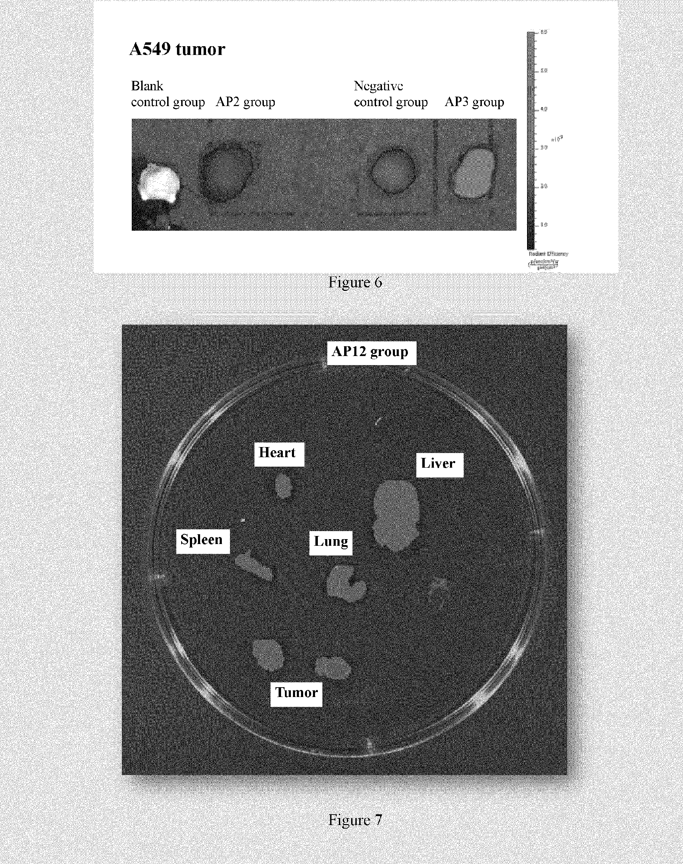

At 1h and 24h after administration, one mouse of each group was respectively euthanized, and tumor tissues were taken for fluorescence imaging in IVIS Lumina Series III. Two organs of each mouse were arranged horizontally in sequence and placed under the same field of view for taking pictures. The results are shown in Figure 6.

Figure 6 shows the fluorescence imaging of the organs of one mouse in each group at 1h after administration. As can be seen from Figure 6, except the blank control group, strong fluorescence signal intensity was detected in A549 tumors of AP2, AP3 and AP12 groups, and the fluorescence signal intensity of AP3 group was stronger than those of other groups.

As can be seen from the above results, the conjugates with different modifications of the present disclosure can specifically target A549 tumors.

### Experimental Example 8 Ability of the conjugates provided by the present disclosure to enter into PAN02 tumor cells

PAN02 tumor cell model was used in this experimental Example to verify the ability of the conjugates provided by the present disclosure to enter into PAN02 tumor cells.

PAN02 tumor cells (purchased from the Shanghai Cell Bank of Chinese Academy of Sciences) in the logarithmic growth phase were selected, digested and then resuspended in DMEM medium supplemented with 10% FBS until the cell density of PAN02 tumor cells reached 1×10⁸ cells/mL.

A 6 weeks old C57/BL mouse (male) was selected. The medium containing PAN02 tumor cells above was inoculated at the subcutaneous site of the right forelimb of the mouse. The volume of PAN02 tumor cells inoculated in the mouse was 100 µL; thus, through calculation, the number of the cells inoculated in the mouse was 1×10⁷ cells.

AP2 was dissolved into a conjugate solution with a concentration of 0.3 mg/mL (based on aptamer) with 1 ×DMEM medium (purchased from M&C gene technology (BEIJING) LTD., Lot No. K1902200). On Day 21 after inoculation of PAN02 tumor cells, the mouse was administered with AP2 conjugate solution by tail vein injection. The administration dosage was calculated according to mouse body weight, the administration volume was 10 µL/g, and the administration dosage was 3 mg/kg (based on the amount of aptamer).

At 135 min after administration, the mouse was euthanized. The heart, liver, spleen, lung and tumor tissues of the euthanized mouse were taken for fluorescence imaging in IVIS Lumina Series III, and placed under the same field of view for taking pictures. The results are shown in Figure 7.

Figure 7 is an image showing the fluorescence imaging of various organ tissues in PAN02 subcutaneous tumor model mice established after administration of the conjugates provided by the present disclosure. As can be seen from Figure 7, the fluorescence signal intensity in PAN02 tumor cells is significantly stronger than that in other organs, indicating that the conjugates provided by the present disclosure can specifically target PAN02 pancreatic cancer tumor cells.

### Experimental Example 9 Ability of conjugates conjugated with different fluorescent groups to enter into U118MG glioma cells

U118MG glioma cells model was used in this experimental Example to verify the ability of the conjugates respectively comprising Cy5-fluorescent group and Cy3-fluorescent group provided by the present disclosure to enter into U118MG glioma.

U118MG glioma cells (purchased from Guangzhou Jennio Biotech Co., Ltd) in the logarithmic growth phase were selected, digested and then resuspended in DMEM medium supplemented with 10% FBS until the cell density of U118MG glioma cells reached 1×10⁸ cells/mL.

11 NOD-SCID mice (male, 6-8 weeks old, purchased from SPF (Beijing) Biotechnology Co., LTD.) was selected and divided randomly: 3 mice in the first group, 2 mice in the second group, 3 mice in the third group, and 3 mice in the fourth group. The culture medium containing U118MG glioma cells above was inoculated at the subcutaneous site of the right forelimb of a mouse, the volume of inoculated cells in each mouse was 100 µL; thus, through calculation, the number of inoculated cells was 1×10⁷ cells per mouse.

AP2, AP9 and comparative AP12 were respectively dissolved into conjugate solutions with a concentration of 0.3 mg/mL conjugate solutions (based on aptamer) with 1×DMEM medium (purchased from M&C gene technology (BEIJING) LTD., Lot No. K1902200). On Day 21 after inoculation of the U118MG glioma above, each mouse was respectively administered with 1×DMEM and AP2, AP9 and AP12 conjugate solutions formulated above by tail vein injection. Therein, 3 mice in the first group were injected with AP2 conjugate solution, 2 mice in the second group were injected with AP9 conjugate solution, 3 mice in the third group were injected with AP12 conjugate solution, and 3 mice in the fourth group were injected with 1×DMEM as the blank control group. The administration dosage for all animals was calculated according to body weight, and the administration volume was 10 µL/g per mouse. That is, the administration dosage of each mouse in the first to third groups was 3 mg/kg (based on the amount of aptamer).

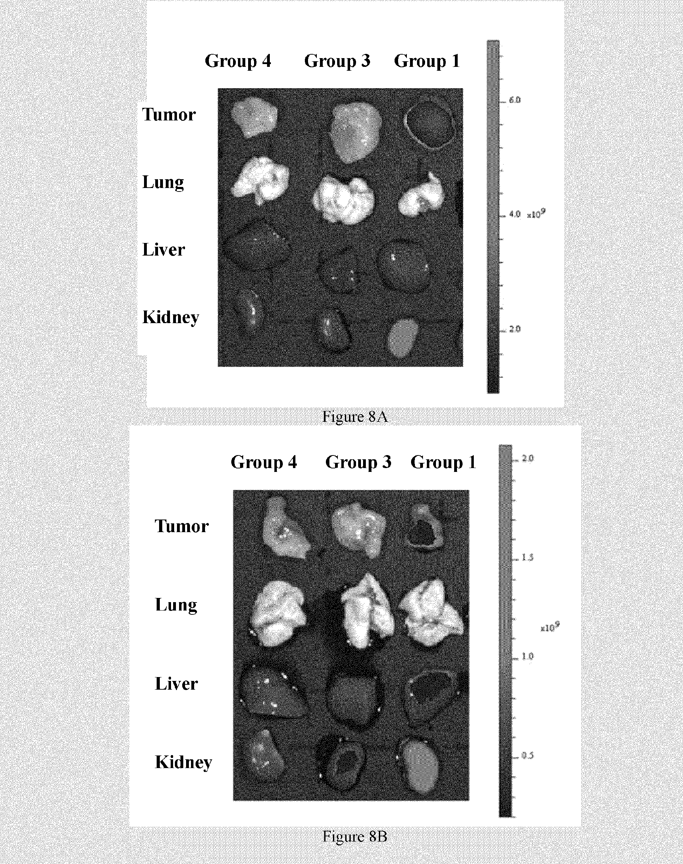

The day of administration is designated as D1. At 48h, 96h, and 11 days (D12) after administration, one mouse of each group was respectively euthanized. The tumor, lung, liver, and kidney tissues of each euthanized mouse were taken for fluorescence imaging in IVIS Lumina Series III. Four organs of each mouse were arranged vertically in sequence and placed under the same field of view for taking pictures. The results are shown in Figures 8A-8E. Therein, group 1 represents the test group administered with AP2, group 2 represents the test group administered with AP9, group 3 represents the test group administered with AP12, and group 4 represents the blank control group.

Figure 8A shows the fluorescence imaging of the organs of 1 mouse in the first group, 1 mouse in the third group, and 1 mouse in the fourth group at 48h after administration. As can be seen from Figure 8A, no fluorescence signal was detected in the third and fourth groups. At the same time, significant fluorescence signals were detected in tumor tissues of the mice injected with AP2, and no fluorescence signal was detected in tissues other than the metabolic organ kidney, indicating that AP2 can effectively target tumor tissues and has no significant targeted delivery in other tissues as compared with the comparative conjugates.

Figure 8B shows the fluorescence imaging of the organs of 1 mouse in the first group, 1 mouse in the third group, and 1 mouse in the fourth group at 96h after administration. As can be seen from Figure 8B, AP2 still shows significant fluorescence signal in tumor tissues after 96h.

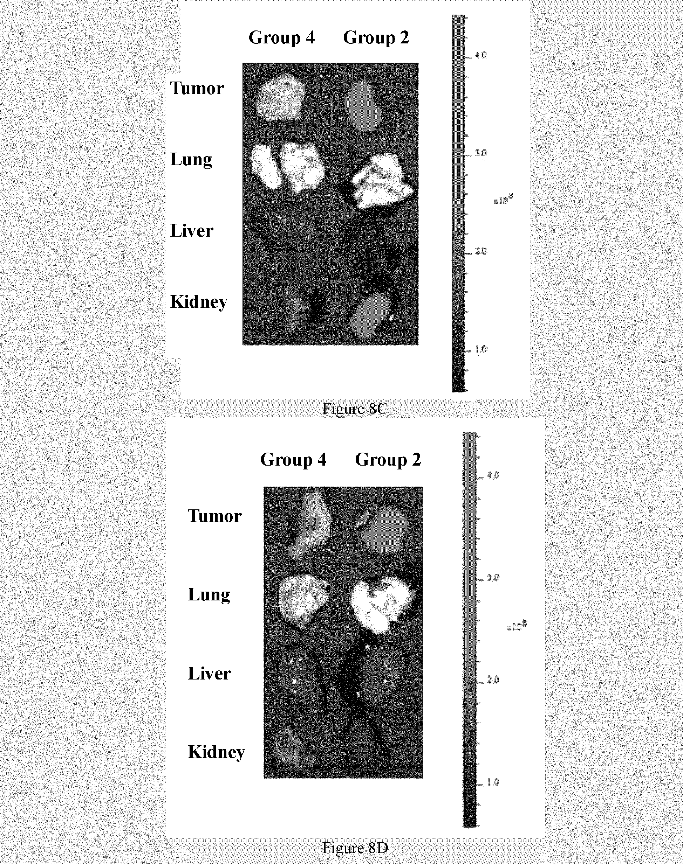

Figure 8C shows the fluorescence imaging of the organs of 1 mouse in the second group and 1 mouse in the fourth group at 48 hours after administration. As can be seen from Figure 8C, significant fluorescence signals can be detected in tumor tissues of the mice injected with Cy3-labeled AP9 as compared with the control group, indicating that the conjugate provided by the present disclosure can efficiently target and deliver different diagnostic agent groups (e.g., fluorescent groups) to tumor tissues, and the conjugates comprising diagnostic agent groups provided by the present disclosure can stably target tumor tissues.

Figure 8D shows the fluorescence imaging of the organs of 1 mouse in the second group and 1 mouse in the fourth group at 96h after administration. As can be seen from Figure 8D, significant fluorescence signals can be detected in tumor tissues of the mice injected with Cy3-labeled AP9, and no fluorescence signal was detected in tissues other than the metabolic organ kidney. It can be seen that the conjugate provided by the present disclosure can efficiently target and deliver different diagnostic agent groups (e.g., different fluorescent groups) to U118MG glioma and can maintain stability over a longer period of time.

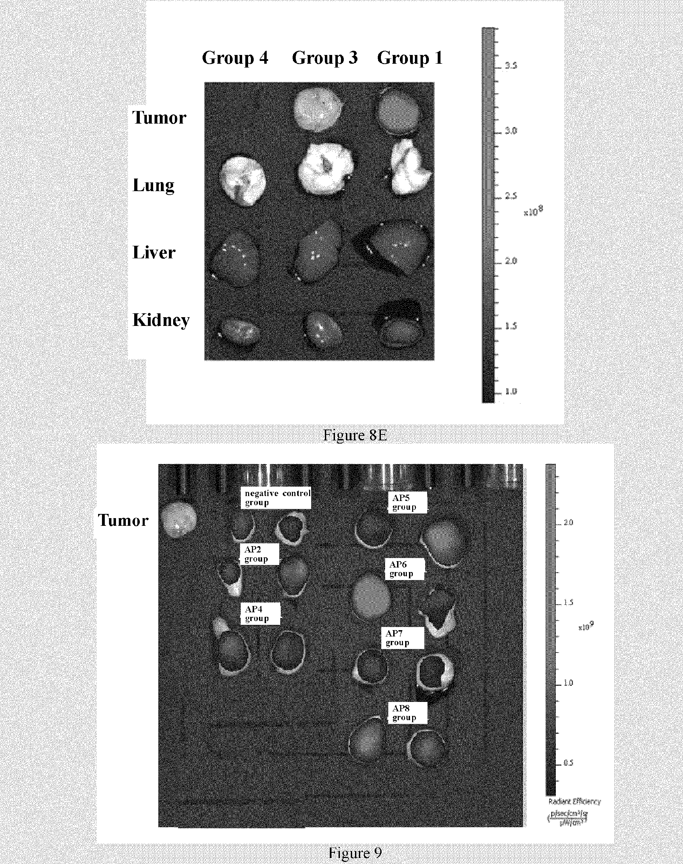

Figure 8E shows the fluorescence imaging of the organs of 1 mouse in the first group, 1 mouse in the third group, and 1 mouse in the fourth group on Day 11 after administration. As can be seen from Figure 8E, significant fluorescence signal of AP2 can still be detected in tumor tissues, and no fluorescence signal was detected in tissues other than the metabolic organ kidney on Day 11 after administration. It can be seen that the conjugates provided by the present disclosure can still stably target U118MG glioma tissue with high specificity over a longer period of time.

From above results, it can be seen that the conjugate provided by the present disclosure can not only specifically target glioma tissue, but also deliver different diagnostic agent groups to glioma. Thus, the conjugate provided by the present disclosure shows excellent delivery capability; meanwhile, the conjugates comprising diagnostic agent groups provided by the present disclosure can efficiently and stably target glioma, so that the presence of glioma can be rapidly and effectively diagnosed, and the diagnostic effect can be kept stable over a long period of time.

### Experimental Example 10 Targeting effect of conjugates with different sequences and lengths on U118MG glioma

U118MG glioma cells model was used in this experimental Example to investigate the targeting effect of conjugates with different sequences and lengths on U118MG glioma.

U118MG glioma cells (purchased from Guangzhou Jennio Biotech Co., Ltd) in the logarithmic growth phase were selected, digested and then resuspended in DMEM medium supplemented with 10% FBS until the cell density of U118MG glioma cells reached 1×10⁸ cells/mL.

14 NOD-SCID mice (male, 6-8 weeks old, purchased from SPF (Beijing) Biotechnology Co., LTD.) was selected and divided randomly: 2 mice in each group. The culture medium containing U118MG glioma cells above was inoculated at the subcutaneous site of the right forelimb of a mouse, the volume of inoculated U118MG glioma in each mouse was 100 µL; thus, through calculation, the number of inoculated cells was 1×10⁷ cells per mouse.

AP2, AP4, AP5, AP6, AP7, AP8, and AP12 were respectively dissolved conjugate solutions with a concentration of 0.3 mg/mL (based on aptamer) with 1×DMEM medium (purchased from M&C gene technology (BEIJING) LTD., Lot No. K1902200). On Day 21 after inoculation of the U118MG glioma above, each mouse was respectively administered with AP2, AP4, AP5, AP6, AP7, AP8, and AP12 conjugate solutions formulated above by tail vein injection. Therein, the groups of mice injected with AP2, AP4, AP5, AP6, AP7, AP8 and AP12 were respectively designated AP2 group, AP4 group, AP5 group, AP6 group, AP7 group, AP8 group and AP12 group. The administration dosage for all animals was calculated according to body weight, and the administration volume was 10 µL/g per mouse. That is, the administration dosage was 3 mg/kg per mouse (based on the amount of aptamer).

The day of administration is designated as D1. At 1h, 24h (D2), 48h (D3) and 72h (D4) after administration, the mice of each group were placed in a small animal *in vivo* optical imaging system IVIS Lumina Series III. The mice were anesthetized by isoflurane gas, and the anesthetized mice were placed on their back in the small animal *in vivo* optical imaging system for *in vivo* imaging. The fluorescence signal of Cy5 was dynamically detected and the distribution of different conjugates labeled by Cy5 marker was tracked *in vivo.* Analysis of the observation results showed that fluorescence signals were visible in subcutaneous tumors of all mice at 1h after administration, and there was no significant difference in fluorescence signal intensity among all groups of mice. At 24 and 48h after administration, fluorescence signals could still be detected in each group of mice, but the fluorescence signals in subcutaneous tumors in mice injected with AP6 and AP8 were stronger than those in mice injected with AP2, AP4, AP5, AP7, and AP12.

On D9, one mouse of each group was respectively euthanized. The tumor, lung, liver, and kidney tissues of each euthanized mouse were taken for fluorescence imaging in IVIS Lumina Series III. Tumor tissues of each mouse were arranged vertically and placed under the same field of view for taking pictures. The results are shown in Figure 9.

Figure 9 is an image showing the fluorescence imaging of tumors in each group of mice on D9 after administration. As can be seen from Figure 9, Cy5 fluorescence signal can be detected in tumor tissues of mice in all groups, indicating that the conjugates with different sequences and lengths of the present disclosure can stably and effectively target tumor tissues over a long period of time.

### Experimental Example 11 In vivo targeting activity of the conjugates in mice

In this experimental Example, the *in vivo* targeting activity of conjugates AP2, AP21-26 and comparative AP27- comparative AP30 in mice were investigated.

U118MG cells were cultured and inoculated subcutaneously into 24 mice (all female) according to the method of experimental Example 4 to obtain the mice inoculated with U118MG subcutaneous tumors.

Conjugates AP2, AP21-26 and comparative AP27-comparative AP30 were respectively formulated into 0.3 mg/mL solutions with DMEM medium.

The administration began 14 days after inoculation of U118MG cells, and the day of administration was designated as D1. The administration manner of tail vein injection was adopted in this experiment: administration was performed once a day, three times in total.

24 mice inoculated with U118MG subcutaneous tumors were randomly divided into 12 groups (2 mice per group).

For 7 groups of mice, each mouse in each group was administered with AP2, AP21, AP22, AP23, AP24, AP25, or AP26 with a single administration volume of 10 µL/g mouse body weight (through calculation, the single administration dosage was 3 mg/kg), and these groups were designated as test groups 11A-11G, respectively.

For other 4 groups of mice, each mouse in each group was administered with comparative AP27, comparative AP28, comparative AP29, or comparative AP30 with a single dose volume of 10 µL/g mouse body weight (through calculation, the single administration dosage was 3 mg/kg), and these groups were designated as control groups 11H-11K, respectively.

For 2 mice of the last group, each mouse was administered with DMEM medium with an administration volume of 10 µL/g mouse body weight, and this group was designated as blank control group 12Y

At 1h, 24h and 48h after first administration, a small animal *in vivo* optical imaging system IVIS Lumina Series III was used for *in vivo* imaging of the mice. On D5, mice of each group were euthanized, and the tumor tissues and kidneys were taken for fluorescence imaging.

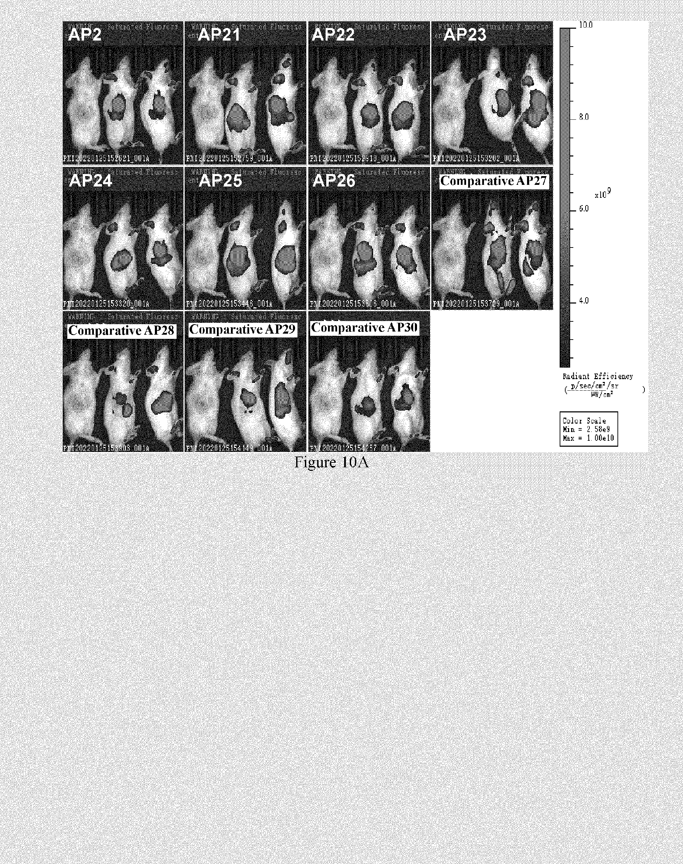

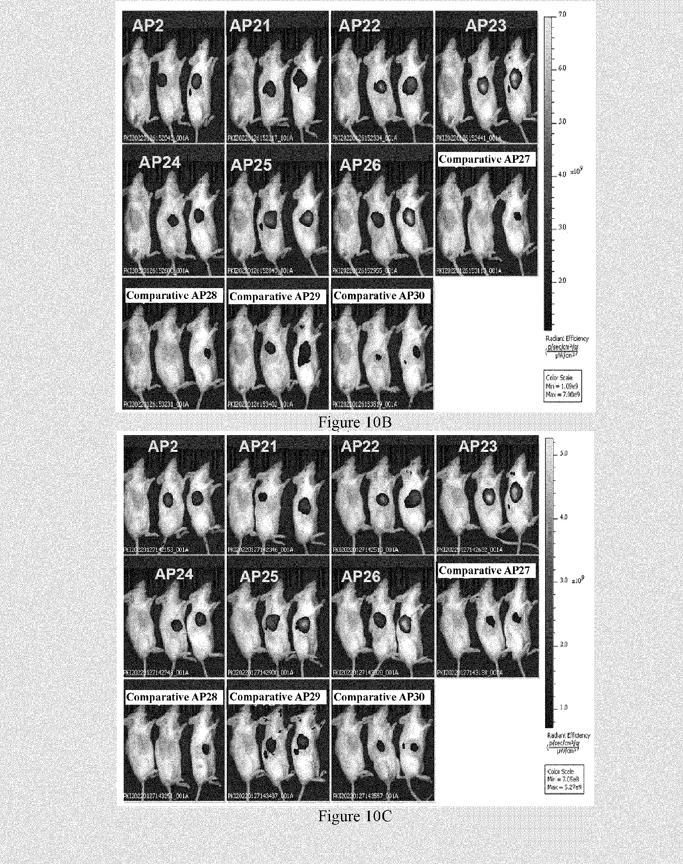

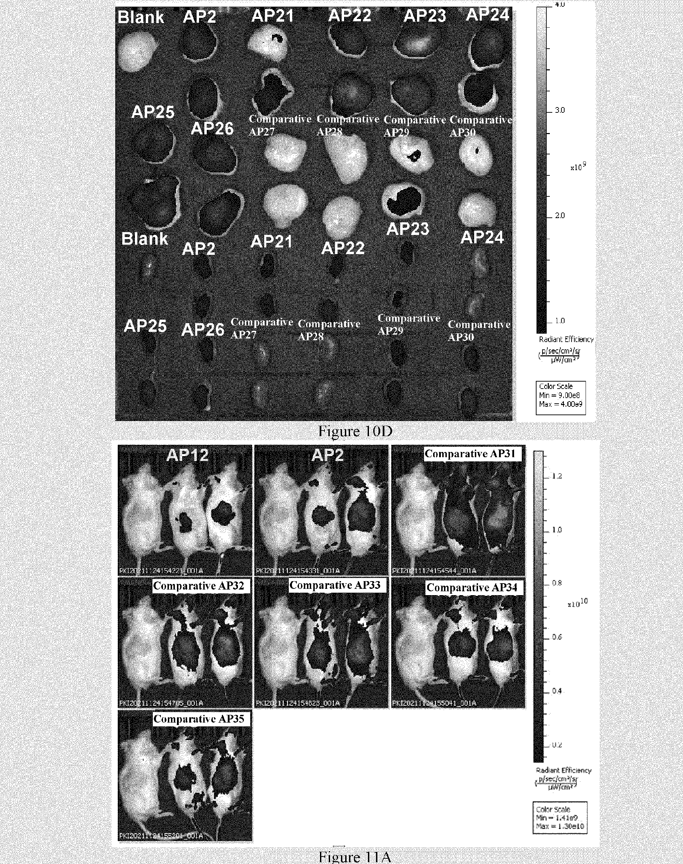

Figures 10A-10C are respectively images showing the fluorescence imaging results in mice at 1h, 24h and 48h after administrating different conjugates, in which the leftmost mouse of the three mice in each panel is a mouse in blank control group 11Y. As can be seen from Figure 10A, the blank control group does not show any fluorescence signal; in contrast, the mice of all test groups and control groups show fluorescence signals at subcutaneous tumor sites at 1h after administration; as can be seen from Figures 10B and 10C, only the mice of test groups 11A-11G show strong fluorescence signals at subcutaneous tumor sites at 24h and 48h after administration, while the mice of control groups 11H-11K substantially do not show fluorescence signals or show only weak fluorescence signals. Furthermore, Figure 10D is an image showing the fluorescence signal imaging of tumor tissues and kidneys in each group of mice after the mice were euthanized at D5, wherein blank represents blank control group 11Y. As can be seen from Figure 10D, the mice in blank control group 11Y and control group 11H-11K substantially do not show fluorescence signals or show only weak fluorescence signals at tumor tissues; in contrast, the mice in test group 11A-11G which administered with the conjugates provided by the present disclosure show strong fluorescence signals at tumor tissues, while showing only weak fluorescence signals at the metabolic organ kidney, indicating that the conjugate of the present disclosure can stably and efficiently target tumor tissues as compared with the control conjugates. Various conjugates provided by the present disclosure can stably and efficiently target tumor tissues, thereby facilitating the successful diagnosis and monitoring of the presence of tumors.

### Experimental Example 12 In vivo targeting activity of conjugates in mice

In this experimental Example, the *in vivo* targeting activity of conjugates AP2, AP12 and comparative AP31- comparative AP35 in mice were investigated.

U118MG cells were cultured and inoculated subcutaneously into 16 mice (all male) according to the method of experimental Example 4 to obtain the mice inoculated with U118MG subcutaneous tumors.

Conjugates AP2, AP12 and comparative AP31-comparative AP35 were respectively formulated into 0.3 mg/mL solutions with DMEM medium.

The administration began 21 days after inoculation of U118MG cells, and the day of administration was designated as D1. The administration manner of tail vein injection was adopted in this experiment: administration was performed once a day, three times in total.

16 mice inoculated with U118MG subcutaneous tumors were randomly divided into 8 groups (2 mice per group).

For 2 groups of mice, each mouse in each group was administered with AP2 or AP12 with a single administration volume of 10 µL/g mouse body weight ( through calculation, the single administration dosage was 3 mg/kg), and these groups were designated as test groups 12A-12B, respectively.

For other 5 groups of mice, each mouse in each group was administered with comparative AP31, comparative AP32, comparative AP33, comparative AP34, or comparative AP35 with a single dose volume of 10 µL/g mouse body weight (through calculation, the single administration dosage was 3 mg/kg), and these groups were designated as control groups 12C-12G, respectively.

For 2 mice of the last group, each mouse was administered with DMEM medium with an administration volume of 10 µL/g mouse body weight, and this group was designated as blank control group 12Y.

At 1h, 24h and 48h after first administration, a small animal *in vivo* optical imaging system IVIS Lumina Series III was used for *in vivo* imaging of the mice. On D6, mice of each group were euthanized, the tumor tissues and kidneys were taken for fluorescence imaging.

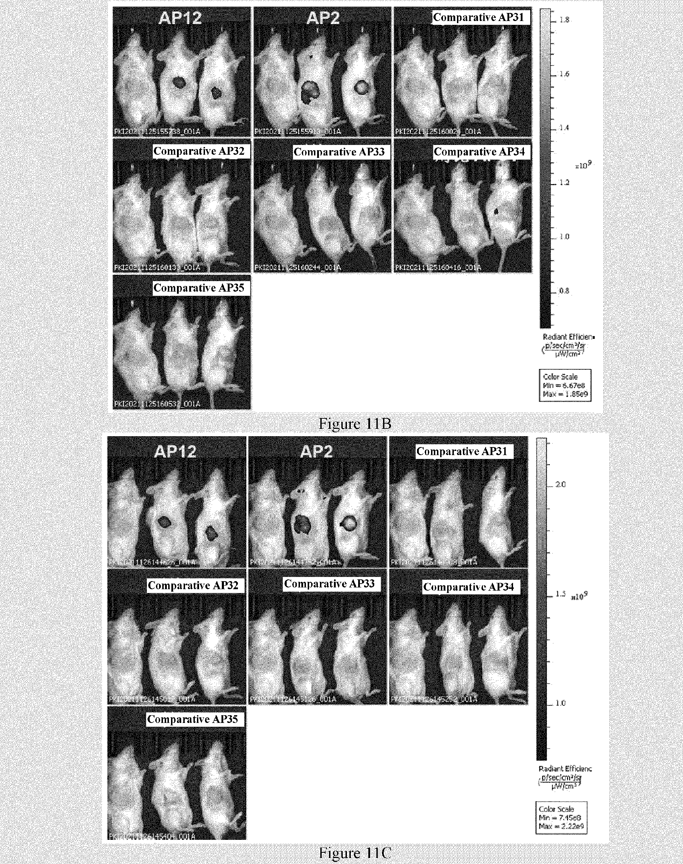

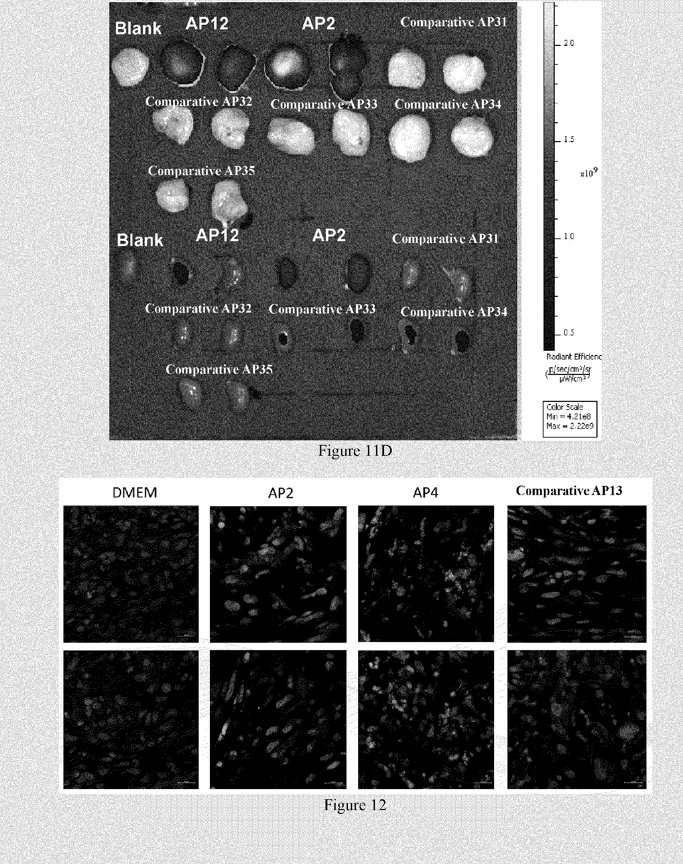

Figures 11A-11D are respectively images showing the fluorescence imaging results in mice at 1h, 24h and 48h after administrating different conjugates, in which the leftmost mouse of the three mice in each panel is a mouse in blank control group 12Y. As can be seen from Figure 11A, the blank control group does not show any fluorescence signal; in contrast, the mice of all test groups and control groups show fluorescence signals at subcutaneous tumor sites at 1h after administration; as can be seen from Figures 11B and 11C, only the mice of test groups 12A and 12B show strong fluorescence signals at subcutaneous tumor sites at 24h and 48h after administration, while the mice of blank control group 12Y and control groups 12C-12G do not show fluorescence signal. Furthermore, Figure 11D is an image showing the fluorescence signal imaging of tumor tissues and kidneys in each group of mice after the mice were euthanized at D6, wherein blank represents blank control group 12Y. As can be seen from Figure 11D, the mice in blank control group 12Y and control group 12C-12G do not show fluorescence signal at tumor tissues; in contrast, the mice in test group 12A or 12B which administered with the conjugates provided by the present disclosure show strong fluorescence signals at tumor tissues, while showing only weak fluorescence signals at the metabolic organ kidney, indicating that the conjugates provided by the present disclosure can stably and efficiently target tumor tissues as compared with the control conjugates, and various conjugates without the structure shown in formula (1) do not show targeting effect on tumor tissues.

### Experimental Example 13 Distribution of conjugates AP2, AP4 and AP13 in U118MG human glioma cells

U118MG human glioma cells (purchased from Guangzhou Jennio Biotech Co., Ltd) were cultured according to the method of experimental Example 4. U118MG human glioma cells in the logarithmic growth phase were selected and digested (0.25% pancreatic enzyme). The cells were collected, centrifuged to remove the supernatant, and then resuspended in serum-free DMEM medium to be formulated into a cell culture solution with a concentration of 1×10⁸ cells/mL.

Experimental animals were 8 NOD-SCID mice (male, 12 weeks old, purchased from SPF (Beijing) Biotechnology Co., LTD.). The cell culture solution above was inoculated at the subcutaneous site of the right back of a NOD-SCID mouse with an inoculation volume of 100 µL per mouse, i.e., each mouse was inoculated with 1×10⁷ cells. The mice were further fed for 21 days after injection. 8 mice were randomly divided into 4 groups (2 mice per group).

Conjugates AP2, AP4 and AP13 were dissolved into conjugate solutions with a concentration of 0.3 mg/mL (based on aptamer) with serum-free DMEM medium.

Administration began 21 days after inoculation of U118MG cells, and the day of administration was designated as D1. The administration manner of tail vein injection was adopted in this experiment (single administration).

For 3 groups of mice, each mouse in different groups was administered with conjugates AP2, AP4 or AP13 with a single administration volume of 10 µL/g mouse body weight (through calculation, the single administration dosage was 3 mg/kg), and these groups were designated as test groups 1-3, respectively.

For the last group, each mouse was administered with DMEM with an administration volume of 10 µL/g mouse body weight, and this group was designated as blank control group.

At 24h after first administration, the mice were euthanized. The tumor tissues were collected and fixed in 4% paraformaldehyde solution, dehydrated with 15% sucrose for 24h and then dehydrated with 30% sucrose for 24h, infiltrated with OCT embedding agent, and frozen in liquid nitrogen. Then, the frozen tissues were sliced using a freezing microtome (Type POLAR-D-JC, purchased from Sakura Finetek Japan Co., Ltd.) with a thickness of 10 µm, to obtain the tumor tissue sections.

The tissue sections were stood still at room temperature for 5 min, fixed with 4% paraformaldehyde for 15 min, and washed with PBS for 3 times (5 mL each time). Then, the sections were stained with DAPI for 3 minutes in the dark, washed with above steps of washing for 3 times, and sealed, to obtain DAPI stained sections.

Imaging analysis of the resultant DAPI stained sections above was performed by using a laser confocal imager (Type LSM 900 Basic Operation, purchased from Carl Zeiss (Shanghai) Managing Co., LTD.), with the select parameters: Cy5 laser intensity of 20% (em 650nm, ex 670nm), DAPI laser intensity of 1.5% (em 360nm, ex 460nm), taking pictures in Best signal mode. The results are shown in Figure 12.

Figure 12 is an image showing the laser confocal imaging results of U118MG glioma cells 24h after administration of different conjugates to the mice subcutaneously inoculated with U118MG glioma.

The experimental results show that the conjugates provided by the present disclosure can efficiently and specifically deliver the diagnostic agent groups into the interior of tumor cells of U118MG glioma as compared with blank control group DMEM and comparative conjugate AP13, showing excellent targeting effect and potential diagnostic capability.

Some embodiments of the present disclosure are described in detail above, but the present disclosure is not limited to the specific details of the above embodiments. Various simple variations of the technical solutions of the present disclosure can be made within the scope of the technical concept of the present disclosure, and these simple variations are within the scope of the present disclosure.

It is to be noted that each of the specific technical features described in the above embodiments can be combined in any suitable manner as long as no contradiction is caused. In order to avoid unnecessary repetition, various possible combinations are no longer described in the present disclosure.

In addition, various different embodiments of the present disclosure may also be arbitrarily combined as long as it does not deviate from the idea of the present disclosure, which should also be regarded as the disclosure of the present disclosure.

## Claims

1. A conjugate comprising one or more delivery groups and one or more diagnostic agent groups, wherein each of the delivery groups is independently linked to the diagnostic agent group via a covalent bond or via a linking group;
wherein the delivery group is formed by removing one or more hydrogen atoms or one or more functional radicals from an aptamer, the aptamer comprises a segment of consecutive nucleotide sequence, wherein the group connecting two adjacent nucleotides is independently a phosphate ester group or a phosphate ester group with modification group(s), each nucleotide is selected from one of modified or unmodified A, U, C or G, and the consecutive nucleotide sequence has a sequence shown in Formula (1):
5'- T₁-S₁-Nₐ-S₂-N_{d}-S₃-N_{c}-S₄-T₂-3' Formula (1)
wherein T₁ is a motif consisting of 1-3 nucleotides, T₂ is a motif consisting of 0-15 nucleotides, and T₂ does not comprise a motif that is completely reverse complementary to T₁;
S₁ and S₄ are each motifs consisting of 3-7 nucleotides, S₁ and S₄ are of the same length and are completely reverse complementary;
Nₐ and N_{c} are each motifs consisting of 1-4 nucleotides, each nucleotide in Nₐ is not complementary to each nucleotide in N_{c}, and the total number of U in Nₐ and N_{c} accounts for more than 50% of the total number of all nucleotides in Nₐ and N_{c};
S₂ and S₃ are each motifs consisting of 1-4 nucleotides, S₂ and S₃ are of the same length and are completely reverse complementary;
N_{b} is a motif consisting of 3-6 nucleotides, and the nucleotides at both ends of N_{b} do not form AU or GC complementarity.

2. The conjugate according to claim 1, wherein the length of the consecutive nucleotide sequence is 18-50 nucleotides, or 20-40 nucleotides, or 21-36 nucleotides, or 24-32 nucleotides.

3. The conjugate according to claim 1 or 2, wherein T₁ consists of 2 nucleotides; or, T₁ consists of 2 nucleotides and comprises at least one C; or, in the 5'-3' direction, T₁ is CU, UC or AC.

4. The conjugate according to any one of claims 1 to 3, wherein T₂ consists of 0-10 nucleotides; or, in the 5'-3' direction, T₂ consists of 1-9 nucleotides starting with U.

5. The conjugate according to any one of claims 1 to 4, wherein S₁ and S₄ each consist of 3-5 nucleotides and are of the same length; or, in the reverse complement formed by S₁ and S₄, GC complementarity accounts for at least 40% of the total number of complementarity; or, in the 5'-3' direction, S₁ is GCU and S₄ is AGC, or S₁ is GAGU and S₄ is GCUC, or S₁ is GGAGU and S₄ is GCUCU, or S₁ is UAUGG and S₄ is CCAUG.

6. The conjugate according to any one of claims 1 to 5, wherein the sum of the number of nucleotides in Nₐ and N_{c} is an integer of 2 to 4; or, the sum of the number of nucleotides in Nₐ and N_{c} is 3 or 4, and the sum of the number of U in Nₐ and N_{c} is 2 or 3; or, in the 5'-3' direction, Nₐ or N_{c} is independently U, UU, UC or CU.

7. The conjugate according to any one of claims 1 to 6, wherein S₂ and S₃ each consist of 2-3 nucleotides and are of the same length; or, the reverse complement formed by S₂ and S₃ comprises at least one GC complementarity; or, in the 5'-3' direction, S₂ is CA and S₃ is UG, or S₂ is AC and S₃ is GU, or S₂ is GCC and S₃ is GGU.

8. The conjugate according to any one of claims 1 to 7, wherein N_{b} consists of 4 or 5 nucleotides; or, in the 5'-3' direction, N_{b} is GACG, GACGU, GACCG, UACU, GUUG or GAUCU.

9. The conjugate according to any one of claims 1 to 8, wherein the consecutive nucleotide sequence has the sequence shown in SEQ ID NO: 1, SEQ ID NO: 2 or SEQ ID NO: 3.

10. The conjugate according to any one of claims 1 to 8, wherein the consecutive nucleotide sequence has the nucleotide sequence shown in SEQ ID NO: 4:
5'-N₆GGAGUUCAN₁N₂N₃N₄UGN₅GCUCN₇-3' (SEQ ID NO: 4),
wherein, N₁, N₂, N₃ independently of one another are one of A, U, C and G; N₄ is U, C or G or a motif consisting of two of U, C or G; N₅ is U, CU or UU; N₆ is CU, UC or AC; N₇ is U, UU or UUN₈; N₈ is a motif consisting of 1-15 nucleotides.

11. The conjugate according to claim 10, wherein the motif N₁N₂N₃N₄ consisting of N₁, N₂, N₃ and N₄ is one of GACG, GACGU, GACCG, UACU, GUUG or GAUCU.

12. The conjugate according to claim 10 or 11, wherein the consecutive nucleotide sequence has the nucleotide sequence shown in any one of SEQ ID NOs: 5-11.

13. The conjugate according to claim 10 or 11, wherein N₈ is a motif consisting of 1-8 nucleotides; or, in the 5'-3' direction, the nucleotide sequence of N₈ is CCGAUCUC; or, the consecutive nucleotide sequence has a sequence shown in any one of SEQ ID NOs: 12-14.

14. The conjugate according to any one of claims 1 to 13, wherein each cytosine nucleotide in the consecutive nucleotide sequence is a fluoro modified cytosine nucleotide, and/or each uracil nucleotide in the consecutive nucleotide sequence is a fluoro modified uracil nucleotide; or, each nucleotide in the consecutive nucleotide sequence is a 2'-methoxy modified nucleotide; or, one or more uracil nucleotides in the motifs N_{b} and S₃ in the consecutive nucleotide sequence have a modified base.

15. The conjugate according to claim 14, wherein the consecutive nucleotide sequence has the nucleotide sequence shown in one of SEQ ID NOs: 15-33.

16. The conjugate according to claim 1, wherein at least one group connecting two adjacent nucleotides in the consecutive nucleotide sequence is a phosphorothioate group, or each group connecting two adjacent nucleotides is a phosphorothioate group.

17. The conjugate according to claim 16, wherein the consecutive nucleotide sequence has the nucleotide sequence shown in one of SEQ ID NOs: 34-39.

18. The conjugate according to any one of claims 1 to 17, wherein each of the diagnostic agent groups is independently selected from a contrast agent group or a fluorescence labelling tracer group.

19. The conjugate according to any one of claims 1 to 18, wherein the conjugate has a structure as shown by Formula (2): 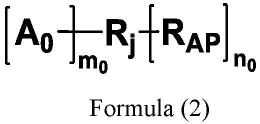
wherein, each R_{AP} group is independently a group having a structure as shown by Formula (3): 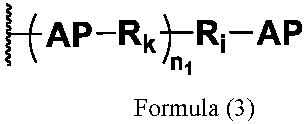
wherein, each AP group is identical or different and independently represents one of the delivery groups; Rⱼ, each Rₖ or each Rᵢ are identical or different and independently represent a covalent bond or a linking group respectively, and Rᵢ and Rₖ are not both a covalent bond at the same time; each n₁ independently of one another represents an integer of 0 to 4;
each A₀ group is the same or different and independently represents one diagnostic agent group; m₀ is an integer of 1 to 6; n₀ is an integer of 1 to 6, 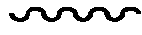 represents the site where a group is covalently linked.

20. The conjugate according to claim 19, wherein m₀ is an integer of 1 to 4, and/or n₀ is an integer of 1 to 3, and/or each n₁ is independently an integer of 0 to 1; alternatively, m₀ is 1, and/or n₀ is 1, and/or at least one or each n₁ is 0.

21. The conjugate according to claim 19 or 20, wherein each of the Rₖ and/or each of the Rᵢ is independently a covalent bond or a straight-chain alkylene group having a length of 1 to 70 carbon atoms, or one or more carbon atoms in the straight-chain alkylene group are replaced by one or more groups selected from the group consisting of: C(O), NH, O, S, CH=N, S(O)₂, OP(O)₂, OP(O)(S), C₅-C₈ glycosidylene group, C₂-C₁₀ alkenylene group, C₂-C₁₀ alkynylene group, C₆-C₁₀ arylene group, C₃-C₁₈ heterocyclylene group, and C₅-C₁₀ heteroarylene group; and wherein the straight-chain alkylene group may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, - SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), - N(C₁-C₁₀ alkyl)(C₁-C₁₀ alkylphenyl), -NH(C₁-C₁₀ alkylphenyl), cyano, nitro, -CO₂H, - C(O)O(C₁-C₁₀ alkyl), -CON(C₁-C₁₀ alkyl)(C₁-C₁₀ alkyl), -CONH(C₁-C₁₀ alkyl), - CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O) (phenyl), -C(O)C₁-C₁₀ alkyl, -C(O)C₁-C₁₀ alkylphenyl, - C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl), and -NHSO₂(C₁-C₁₀ haloalkyl).

22. The conjugate according to claim 21, wherein each n₁ is independently 0 or 1, and each Rⱼ is independently a covalent bond, or any one following linking group or a connection combination of more than one following linking groups: C₁-C₂₀ alkylene, phosphate ester bond, phosphorothioate bond, amide bond, ester bond, ether bond, thioether bond, disulfide bond, 1,2,3-triazolylene group, a polyethylene glycol subunit, pyrrolidinylene group, 2-oxopyrrolidinylene group, phenylene, cyclohexylene, 2-succinimidylene group, 2-thiosuccinimidylene group, an amino acid subunit, a nucleotide subunit.

23. The conjugate according to claim 22, wherein each Rᵢ is independently any one following group or a connection combination of two following groups: a covalent bond, a disulfide bond, propylene phosphate ester group, 2-thiosuccinimidylene group, an amino acid subunit, or a GAU trinucleotide subunit.

24. The conjugate according to any one of claims 19 to 23, wherein Rⱼ is a covalent bond and m₀ is 1.

25. The conjugate according to any one of claims 19 to 23, wherein Rⱼ is a linking group, the linking group Rⱼ comprises a main chain moiety, a side chain moiety and a conjugation linking moiety, wherein the main chain moiety is respectively linked to the conjugation linking moiety and the side chain moiety, each of the side chain moieties is respectively linked to the main chain moiety and the R_{AP} group, each of the conjugation linking moieties is respectively linked to the main chain moiety and the diagnostic agent group A₀, wherein,
the main chain moiety is a straight-chain alkylene group having a length of 1 to 70 carbon atoms, or one or more carbon atoms in the straight-chain alkylene group are replaced by one or more groups selected from the group consisting of: C(O), NH, O, S, CH=N, S(O)₂, OP(O)₂, C₅-C₈ glycosidylene group, C₂-C₁₀ alkenylene group, C₂-C₁₀ alkynylene group, C₆-C₁₀ arylene group, C₃-C₁₈ heterocyclylene group and C₅-C₁₀ heteroarylene group; and wherein the straight-chain alkylene group may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl ), -NH(C₁-C₁₀ alkyl), -N(C₁-C₁₀ alkyl)(C₁-C₁₀ alkylphenyl), - NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), - NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, - SO₂NH(C₁-C₁₀ alkyl), -SO₂NH(phenyl), -NHSO₂(C₁-C₁₀ alkyl), -NHSO₂(phenyl) and -NHSO₂(C₁-C₁₀ haloalkyl);
each of the side chain moieties is independently a covalent bond, or a straight-chain alkylene group having a length of 1 to 70 carbon atoms, or one or more carbon atoms in the straight-chain alkylene group are replaced by one or more groups selected from the group consisting of: C(O), NH, O, S, CH=N, S(O)₂, OP(O)₂, C₅-C₈ glycosidylene group, C₂-C₁₀ alkenylene group, C₂-C₁₀ alkynylene group, C₆-C₁₀ arylene group, C₃-C₁₈ heterocyclylene group and C₅-C₁₀ heteroarylene group; and the straight-chain alkylene group may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, - SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl ), -NH(C₁-C₁₀ alkyl), - N(C₁-C₁₀ alkyl)(C₁-C₁₀ alkylphenyl), -NH(C₁-C₁₀ alkylphenyl), cyano, nitro, -CO₂H, - C(O)O(C₁-C₁₀ alkyl), -CON(C₁-C₁₀ alkyl)(C₁-C₁₀ alkyl), -CONH(C₁-C₁₀ alkyl), - CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, - C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl) and -NHSO₂(C₁-C₁₀ haloalkyl);
each of the conjugation linking moieties is independently a covalent bond, or any one following linking structure, or a connection combination of more than one following linking structures: C₁-C₁₀ straight-chain alkylene, phosphate ester bond, phosphorothioate bond, amide bond, ester bond, ether bond, disulfide bond, 1,2,3-triazolylene group, a polyethylene glycol subunit, pyrrolidinylene group, 2-oxopyrrolidinylene group, phenylene, cyclohexylene, 2-succinimidylene group, 2-thiosuccinimidylene group, an amino acid subunit, a nucleotide subunit.

26. The conjugate according to claim 25, wherein each of the conjugation linking moieties in the linking group Rⱼ is respectively linked to the main chain moiety and one of the diagnostic agent groups A₀; the number of the side chain moieties is n₀, and each of the side chain moieties is respectively linked to the main chain moiety and one of the R_{AP} groups.

27. The conjugate according to claim 25 or 26, wherein all of the side chain moieties are linked to the same atom in the main chain moiety; or, each of the side chain moieties is linked to different atoms in the main chain moiety.

28. The conjugate according to claim 27, wherein m₀ is 1, and the linking group Rⱼ comprises a structure as shown in Formula (301): 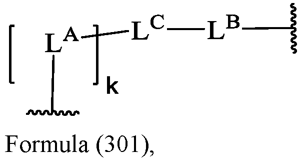
wherein, k is an integer of 1 to 3; L^{C} is the main chain moiety, L^{A} is the side chain moiety, L^{B} is the conjugation linking moiety, and 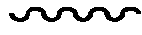 represents the site where a group is covalently linked;
the main chain moiety L^{C} is a covalent bond or a 2- to 4-valence, straight-line or branched C₁-C₂₅ saturated hydrocarbyl, or one or more carbon atoms in the saturated hydrocarbyl are replaced by one or more groups selected from the group consisting of: C(O), NH, O, S, CH=N, S(O)₂, OP(O)₂, C₅-C₈ glycosidylene group, C₂-C₅ alkenylene group, C₂-C₅ alkynylene group, C₆-C₁₀ arylene group, C₃-C₈ heterocyclylene group and C₅-C₁₀ heteroarylene group; wherein the saturated hydrocarbyl may have any one or more substituents selected from the group consisting of: C₁-C₅ alkyl, C₆-C₁₀ aryl, C₅-C₁₀ heteroaryl, -OC₁-C₅ alkyl, -OC₁-C₅ alkylphenyl, -C₁-C₅ alkyl-OH, -SC₁-C₅ alkyl, nitro, -C(O)O(C₁-C₅ alkyl), -CON(C₁-C₅ alkyl)(C₁-C₅ alkyl), -CONH(C₁-C₅ alkyl), -CONH₂, -NHC(O)(C₁-C₅ alkyl), - NHC(O)(phenyl), -N(C₁-C₅ alkyl)C(O)(C₁-C₅ alkyl), -N(C₁-C₅ alkyl)C(O)(phenyl), - C(O)C₁-C₅ alkyl, -C(O)C₁-C₅ alkylphenyl, -OC(O)C₁-C₅ alkyl, -SO₂(C₁-C₅ alkyl), - SO₂(phenyl), -SO₂NH₂, -SO₂NH(C₁-C₅ alkyl), -SO₂NH(phenyl), -NHSO₂(C₁-C₅ alkyl) and -NHSO₂(phenyl);
each of the side chain moieties is independently a covalent bond, or a straight-chain alkylene group having a length of 1 to 70 carbon atoms, or one or more carbon atoms in the straight-chain alkylene group are replaced by one or more groups selected from the group consisting of: C(O), NH, O, S, CH=N, S(O)₂, OP(O)₂, C₅-C₈ glycosidylene group, C₂-C₁₀ alkenylene group, C₂-C₁₀ alkynylene group, C₆-C₁₀ arylene group, C₃-C₁₈ heterocyclylene group and C₅-C₁₀ heteroarylene group; and the straight-chain alkylene group may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, - SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl ), -NH(C₁-C₁₀ alkyl), - N(C₁-C₁₀ alkyl)(C₁-C₁₀ alkylphenyl), -NH(C₁-C₁₀ alkylphenyl), cyano, nitro, -CO₂H, - C(O)O(C₁-C₁₀ alkyl), -CON(C₁-C₁₀ alkyl)(C₁-C₁₀ alkyl), -CONH(C₁-C₁₀ alkyl), - CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, - C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl) and -NHSO₂(C₁-C₁₀ haloalkyl);
each of the conjugation linking moieties is independently a covalent bond, or any one following structure, or a connection combination of more than one following linking structures: C₁-C₁₀ straight-chain alkylene, phosphate ester bond, phosphorothioate bond, amide bond, ester bond, ether bond, disulfide bond, 1,2,3-triazolylene group, a polyethylene glycol subunit, pyrrolidinylene group, 2-oxopyrrolidinylene group, phenylene, cyclohexylene, 2-succinimidylene group, 2-thiosuccinimidylene group, an amino acid subunit, a nucleotide subunit.

29. The conjugate according to claim 28, wherein the conjugate has a structure as shown in Formula (305):

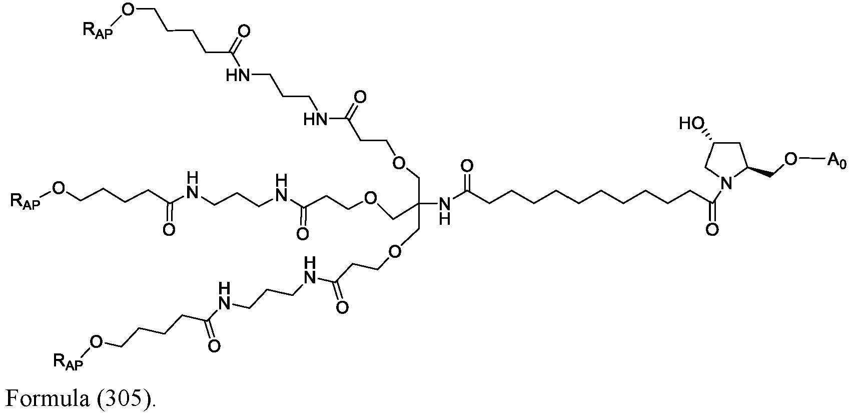

30. The conjugate according to claim 25, wherein the linking group Rⱼ has a structure shown in Formula (306): 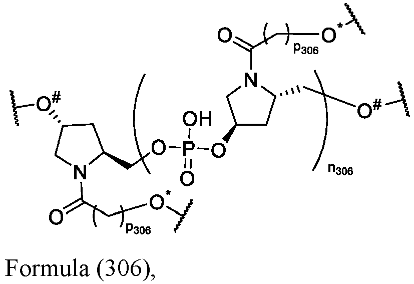 wherein, n₃₀₆ is an integer of 0-3, each p₃₀₆ is independently an integer of 1 to 6, 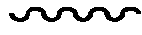 represents the site where a group is covalently linked; the structure formed by alternating pyrrolidinylene group and phosphodiester groups constitutes the main chain moiety, and each of the side chain moieties is constituted by a chain of atoms between the carbonyl group linked to the nitrogen atom on the pyrrolidinyl group and the oxygen atom marked by *, and the side chain moieties form a phosphate ester bond, an ether bond or an ester bond linkage with the R_{AP} group via the oxygen atom marked by *; at least one of the oxygen atoms marked by # is the conjugation linking moiety and forms an ether bond, an ester bond or a phosphate ester bond linkage with the diagnostic agent group A₀, and the remaining oxygen atom marked by # is linked to a hydrogen atom to form a hydroxyl group, or is linked to a C₁-C₃ alkyl groups to form a C₁-C₃ alkoxy groups.

31. The conjugate according to claim 30, wherein the conjugate has a structure as shown in Formula (307a), (307b) or (307c):

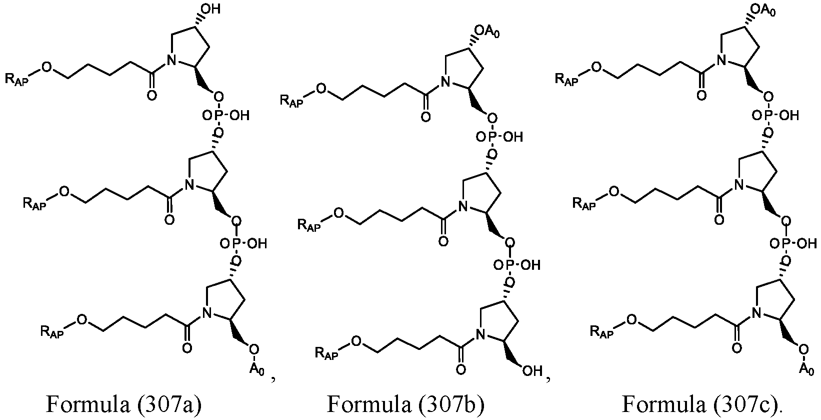

32. The conjugate according to claim 25, wherein the conjugate has a structure as shown in Formula (308): 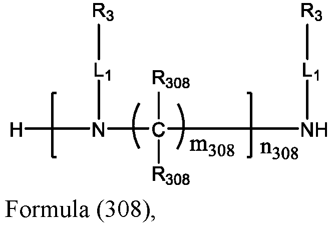
wherein, n₃₀₈ is an integer selected from 1 to 10;
each m₃₀₈ is independently an integer selected from 2 to 10;
each R₃₀₈ is independently H, C₁-C₁₀ alkyl, C₁-C₁₀ haloalkyl or C₁-C₁₀ alkoxy;
each R₃ is independently the diagnostic agent group A₀, or is the R_{AP} group, and at least one R₃ is the diagnostic agent group A₀, and at least one R₃ is the R_{AP} group;
each L₁ linked to the diagnostic agent group A₀ represents the conjugation linking moiety, and each L₁ linked to the R_{AP} represents the side chain moiety.

33. The conjugate according to claim 32, wherein each L₁ is independently selected from the group consisting of groups L4-L23 and any connection combination thereof.

34. The conjugate according to claim 33, wherein each L₁ is independently selected from the group consisting of connection combinations of at least two of the groups (L4)- (L9), (L13), (L14), and (L18); or, each L₁ is independently a connection combination of at least two of the groups (L4), (L5), (L7, ) (L9), (L13), (L14), and (L18).

35. The conjugate according to any one of claims 32 to 34, wherein the length of each L₁ is independently 3-25 atoms; or, the length of each L₁ is independently 4-15 atoms.

36. The conjugate according to any one of claims 32 to 35, wherein n₃₀₈ is an integer of 2 to 6, 2 to 4 R₃ are the R_{AP} groups, and the remaining R₃ are the diagnostic agent groups.

37. The conjugate according to any one of claims 32 to 36, wherein each m₃₀₈ is independently of one another an integer of 2 to 5, and/or each m₃₀₈ is equal.

38. The conjugate according to any one of claims 32-37, wherein n₃₀₈ is an integer selected 2 to 4; each m₃₀₈ is independently an integer selected from 2 to 4; and each R₃₀₈ is H.

39. The conjugate according to claim 38, wherein one R₃ is the diagnostic agent group A₀ and the remaining R₃ are the R_{AP} groups.

40. The conjugate according to claim 38 or 39, wherein each L₁ comprises both an attachment site linked to the N atom of the nitrogen-containing backbone and an attachment site linked to the diagnostic agent group A₀ or the R_{AP} group, and the site linked to the N atom of the nitrogen-containing backbone forms an amide bond to the N atom; or, one or more L₁ are selected from B5, B6, B5' or B6': 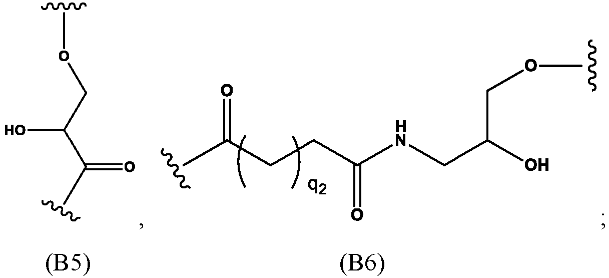 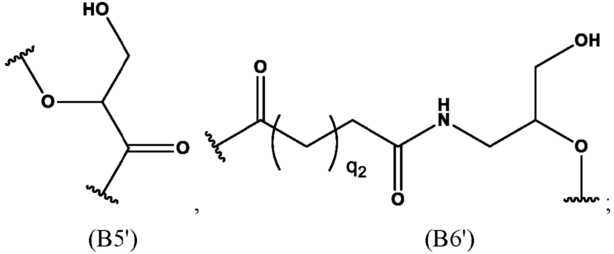 wherein, 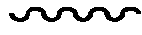 represents the site where a group is covalently linked, q₂ is an integer of 1 to 10; or, q₂ is an integer of 1 to 5.

41. The conjugate according to any one of claims 32-40, wherein the conjugate has a structure as shown in Formula (403), (404), (405), (406), (407), (408), (409), (410), (411), (412), (413), (414), (415), (416), (417), (418), (419), (420), (421), (422), (423), (424), (425), (426) or (427):

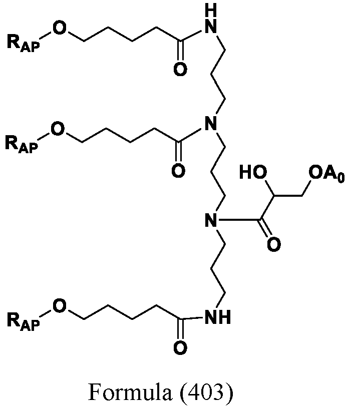

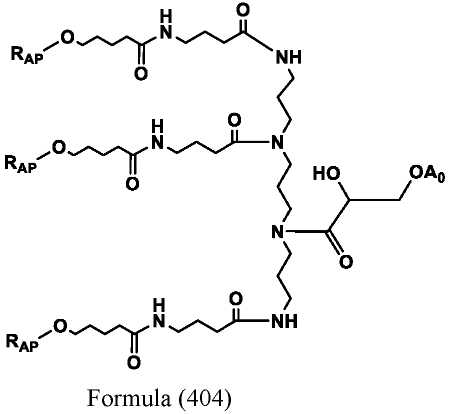

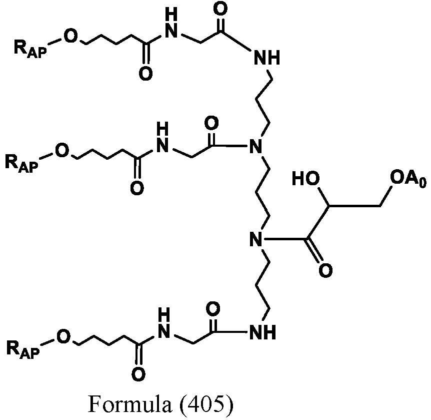

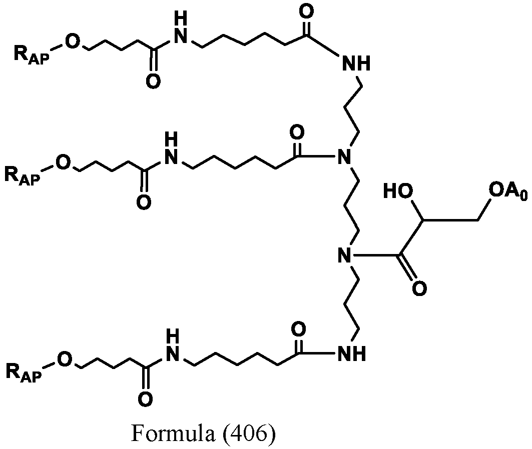

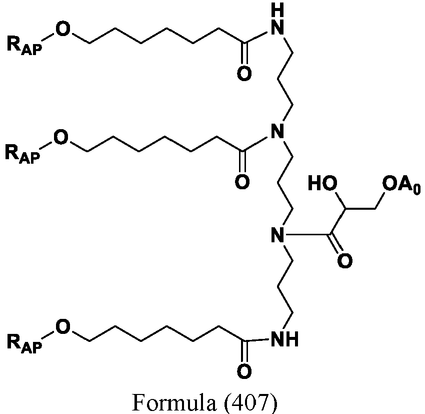

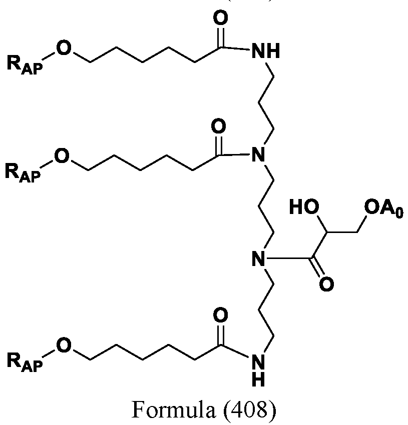

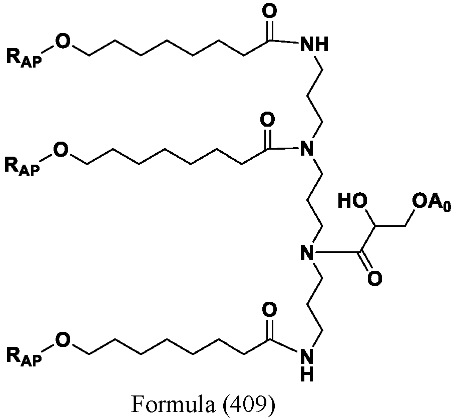

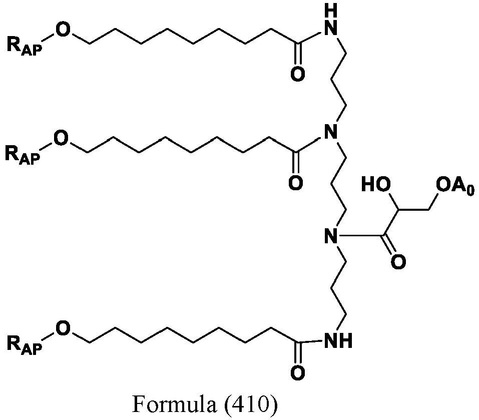

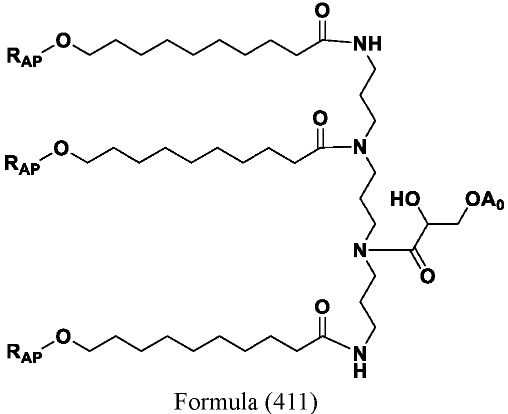

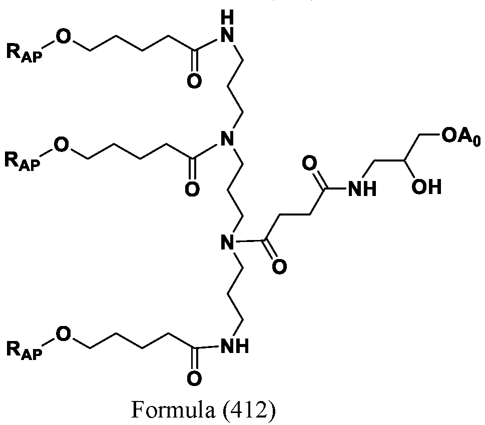

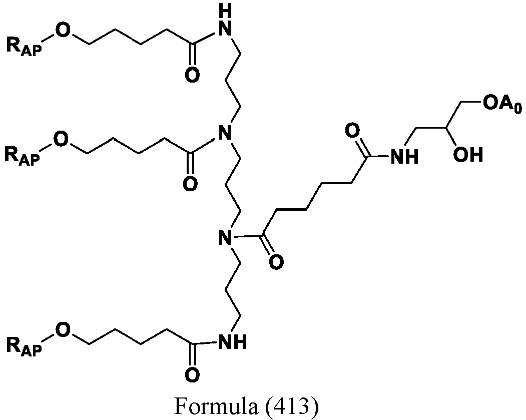

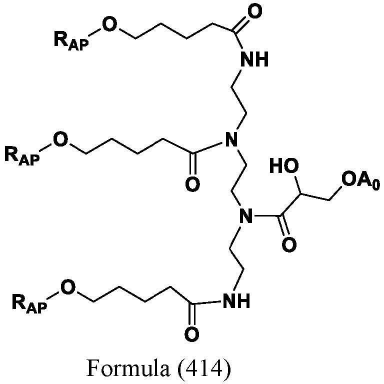

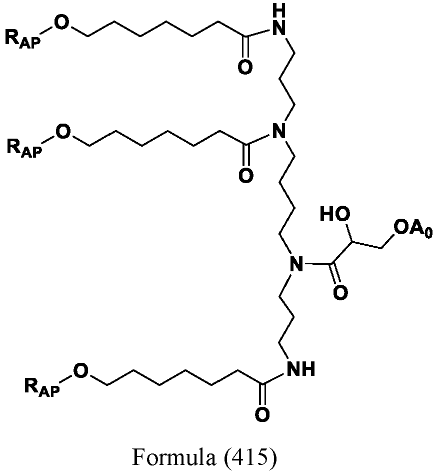

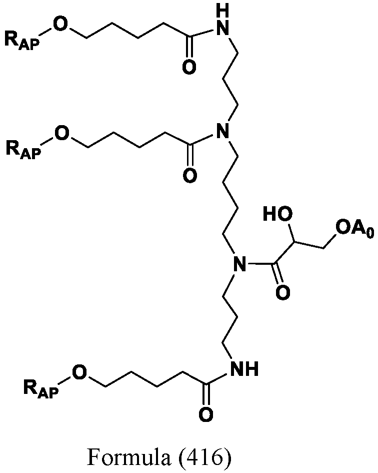

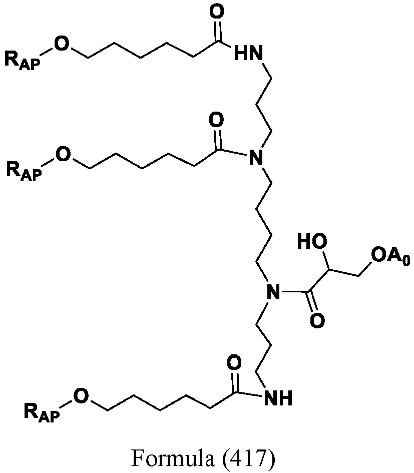

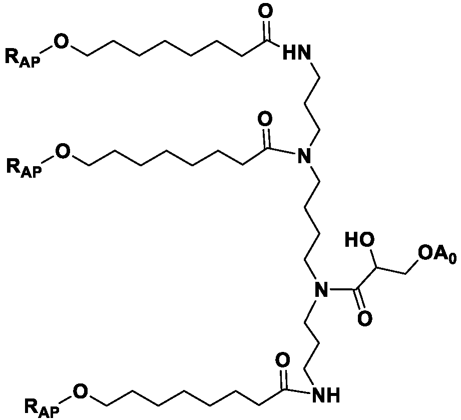

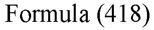

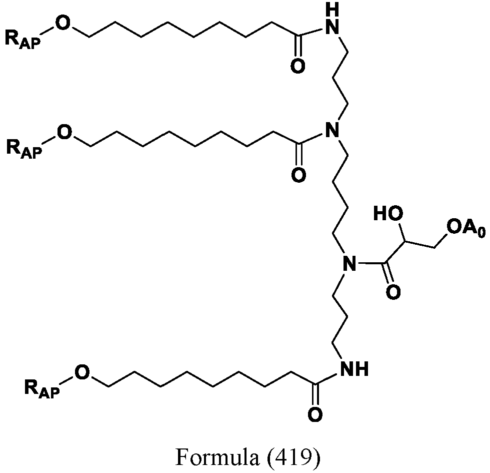

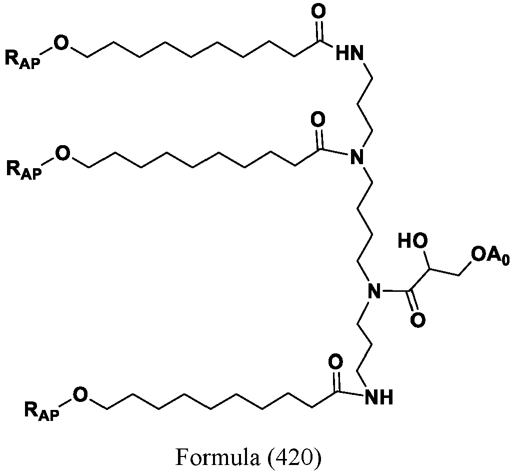

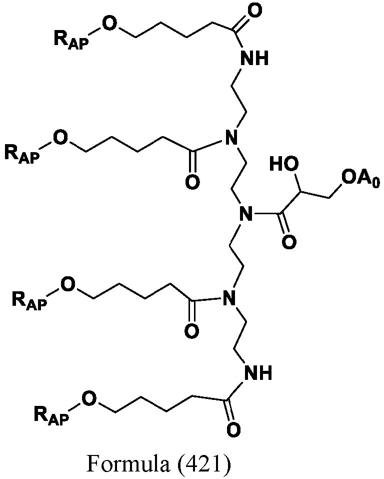

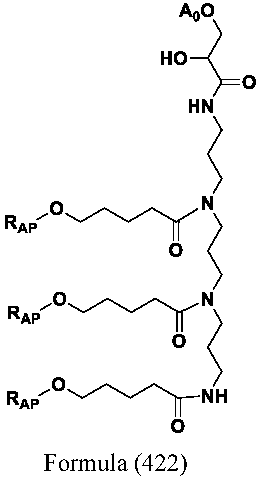

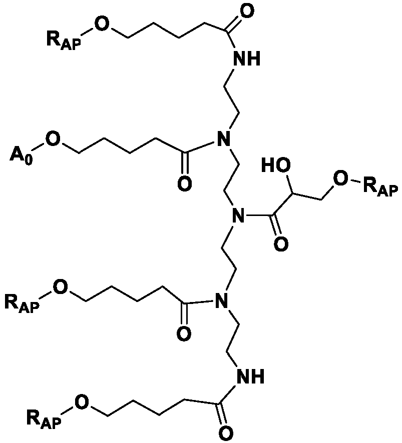

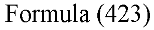

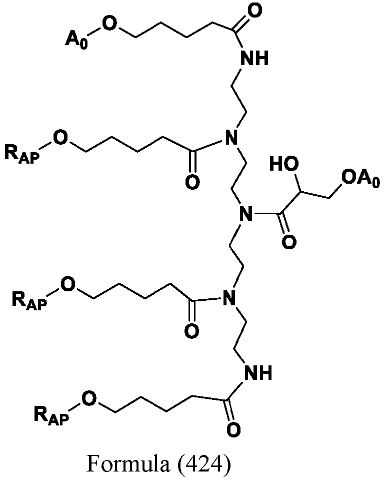

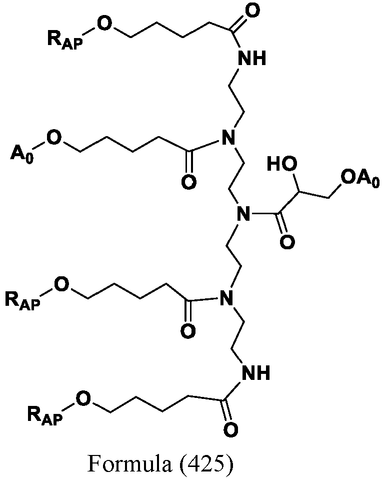

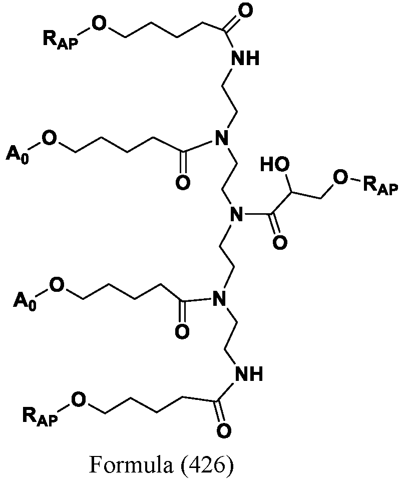

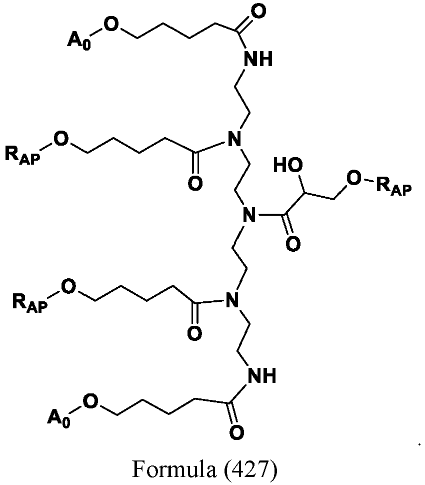

42. The conjugate according to any one of claims 19 to 23, wherein Rⱼ comprises a nucleotide sequence I and a nucleotide sequence II, the nucleotide sequence I and the nucleotide sequence II each comprise 5 to 25 modified or unmodified nucleotides, the nucleotide sequence I and the nucleotide sequence II are at least partially reverse complementary, the delivery group is linked to the nucleotide sequence I, the diagnostic agent group is linked to the nucleotide sequence II, and the nucleotide sequence I and the nucleotide sequence II do not elicit an immune response or a toxic reaction in a subject.

43. The conjugate according to claim 42, wherein the 3' terminal of the delivery group is linked to the 5' position of the ribose of the 5' terminal nucleotide of the nucleotide sequence I via a phosphate ester bond, and the diagnostic agent group is linked to the 5' position of the ribose of the 5' terminal nucleotide of the nucleotide sequence II; or, the diagnostic agent group comprises a segment of nucleotide sequence, and the 3' terminal of the nucleotide sequence is linked to the 5' position of the ribose of the 5' terminal nucleotide of the nucleotide sequence II via a phosphate ester bond.

44. The conjugate according to claim 43, wherein the nucleotide sequence I and the nucleotide sequence II are substantially reverse complementary or completely reverse complementary; or, the nucleotide sequence I and the nucleotide sequence II are equal in length and are both 10-20 modified or unmodified nucleotides; or, the nucleotide sequence I and the nucleotide sequence II both consist of 17 nucleotides and are completely reverse complementary; or, the nucleotide sequence I and the nucleotide sequence II have the sequences shown in SEQ ID NO: 40 and SEQ ID NO: 41, respectively:
5'- GUACAUUCUAGAUAGCC -3' (SEQ ID NO: 40)
5'- GGCUAUCUAGAAUGUAC -3' (SEQ ID NO: 41),
or, the nucleotide sequence I and the nucleotide sequence II have the sequences shown in SEQ ID NO: 42 and SEQ ID NO: 43, respectively:
5'- GmUfAmCfAmUfUfCfUfAmGmAmUfAmGmCfCf -3' (SEQ ID NO: 42)
5'- GmGmCfUfAmUfCfUfAmGmAmAmUfGmUfAmCf -3' (SEQ ID NO: 43).

45. The conjugate according to any one of claims 19-44, wherein Rⱼ is cleavable.

46. The conjugate according to any one of claims 1 to 45, wherein the conjugate further comprises at least one delivery auxiliary group, and the delivery auxiliary group is selected from one or more of C₁₀-C₃₀ hydrocarbyl, cholesteryl group, and phospholipid group.

47. A pharmaceutical composition comprising the conjugate according to any one of claims 1 to 46 and a pharmaceutically acceptable carrier.

48. Use of the conjugate according to any one of claims 1 to 46 and/or the pharmaceutical composition according to claim 47 in the manufacture of a medicament for diagnosing tumors and tumor-related diseases or symptoms.

49. A method for diagnosing tumors and tumor-related diseases, comprising administering an effective amount of the conjugate according to any one of claims 1 to 46 and/or the pharmaceutical composition according to claim 47 to a subject in need thereof.

50. A kit comprising the conjugate according to any one of claims 1 to 46 and/or the pharmaceutical composition according to claim 47.
